(19) Europäisches Patentamt
European Patent Office
Office européen des brevets

(11) EP 2 271 613 B1

(12) # EUROPÄISCHE PATENTSCHRIFT

(45) Veröffentlichungstag und Bekanntmachung des Hinweises auf die Patenterteilung:
**18.06.2014 Patentblatt 2014/25**

(21) Anmeldenummer: **09725940.2**

(22) Anmeldetag: **25.03.2009**

(51) Int Cl.:
***C07C 215/20*** (2006.01) ***C07D 209/04*** (2006.01)
***C07D 209/44*** (2006.01) ***A61K 31/133*** (2006.01)
***A61P 25/00*** (2006.01)

(86) Internationale Anmeldenummer:
**PCT/EP2009/002184**

(87) Internationale Veröffentlichungsnummer:
**WO 2009/118171 (01.10.2009 Gazette 2009/40)**

(54) **HYDROXYMETHYLCYCLOHEXYLAMINE**
HYDROXYMETHYLCYCLOHEXYLAMINES
HYDROXYMÉTHYLE-CYCLOHEXYLAMINE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **27.03.2008 EP 08005809**

(43) Veröffentlichungstag der Anmeldung:
**12.01.2011 Patentblatt 2011/02**

(73) Patentinhaber: **Grünenthal GmbH
52078 Aachen (DE)**

(72) Erfinder:
- **FROMANN, Sven
52066 Aachen (DE)**
- **ZEMOLKA, Saskia
52066 Aachen (DE)**
- **LINZ, Klaus
53359 Rhelnbach (DE)**
- **ENGLBERGER, Werner
52223 Stolberg (DE)**
- **THEIL, Fritz
12247 Berlin (DE)**

(74) Vertreter: **Bülle, Jan et al
Kutzenberger Wolff & Partner
Theodor-Heuss-Ring 23
50668 Köln (DE)**

(56) Entgegenhaltungen:
**WO-A-03/008370 WO-A-2004/043900**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft Hydroxymethylcyclohexylamine, welche eine Affinität an den μ-Opioid-Rezeptor und den ORL1-Rezeptor aufweisen, Verfahren zu deren Herstellung, Arzneimittel enthaltend diese Verbindungen und die Verwendung dieser Verbindungen zur Herstellung von Arzneimitteln.

Cyclohexan-Derivate, welche eine Affinität an den μ-Opioid-Rezeptor und den ORL1-Rezeptor aufweisen, sind im Stand der Technik bekannt. In diesem Zusammenhang kann beispielsweise vollumfänglich verwiesen werden auf WO2002/090317, WO2002/90330, WO2003/008370, WO2003/008371, WO2003/080557, WO2004/043899, WO2004/043900, WO2004/043902, WO2004/043909, WO2004/043949, WO2004/043967, WO2005/063769, WO2005/066183, WO2005/110970, WO2005/110971, WO2005/110973, WO2005/110974, WO2005/110975, WO2005/110976, WO2005/110977, WO2006/018184, WO2006/108565, WO2007/079927, WO2007/079928, WO2007/079930, WO2007/079931, WO2007/124903, WO2008/009415 und WO2008/009416.

Die bekannten Verbindungen sind jedoch nicht in jeglicher Hinsicht zufrieden stellend. So zeigen die bekannten Verbindungen mitunter eine nicht immer optimale Affinität an den ORL1-Rezeptor. Im Allgemeinen kann davon ausgegangen werden, dass mit steigender Affinität einer Verbindung an den ORL1-Rezeptor die erforderliche Dosis sinkt, um die gleiche pharmakologische Wirkung hervorzurufen. Je geringer die erforderliche Dosis jedoch ist, umso geringer ist auch die Wahrscheinlichkeit für das Auftreten von unerwünschten Nebenwirkungen.

Außerdem zeigen die bekannten Verbindungen in geeigneten Bindungsassays mitunter eine gewisse Affinität zum hERG-Ionenkanal, zum L-Typ Calcium-Ionenkanal (Phenylalkylamin-, Benzothiazepin-, Dihydropyridin-Bindungsstellen) bzw. zum Natrium-Kanal im BTX-Assay (Batrachotoxin), was jeweils als Anzeichen für kardiovaskuläre Nebenwirkungen gedeutet werden kann. Ferner zeigen zahlreiche der bekannten Verbindungen eine nur geringe Löslichkeit in wässrigen Medien, was sich u.a. negativ auf die Bioverfügbarkeit auswirken kann. Darüber hinaus ist die chemische Stabilität der bekannten Verbindungen oft nur unzureichend. So zeigen die Verbindungen mitunter keine ausreichende pH-, UV- bzw. Oxidationsstabilität, was sich u.a. negativ auf die Lagerstabilität und auch auf die orale Bioverfügbarkeit auswirken kann. Ferner weisen die bekannten Verbindungen teilweise ein ungünstiges PK/PD (Pharmakokinetik/Pharmakodynamik)-Profil auf, was sich z.B. in einer zu langen Wirkdauer zeigen kann.

Auch die metabolische Stabilität der bekannten Verbindungen scheint verbesserungsbedürftig. Eine verbesserte metabolische Stabilität kann auf eine erhöhte Bioverfügbarkeit hinweisen. Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten.

Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Es besteht ein Bedarf an weiteren Verbindungen, welche an den ORL1-Rezeptor binden. Die Verbindungen sollten möglichst wenigstens eine vergleichbare, vorzugsweise höhere Affinität an den ORL1-Rezeptor zeigen. Die zusätzliche Bindung an andere Rezeptoren (z.B. μ-Opioid-Rezeptor, δ-Opiiod-Rezeptor) bzw. die zusätzliche antagonistische Wirkung an anderen rezeptorn (z.B. B1R-Rezeptor) kann zusätzliche Vorteile mit sich bringen.

Darüber hinaus sollten die Verbindungen möglichst eine vergleichbare, vorzugsweise jedoch eine bessere Löslichkeit in wässrigen Medien aufweisen.

Der Erfindung liegt die Aufgabe zugrunde, Verbindungen zur Verfügung zu stellen, welche sich zu pharmazeutischen Zwecken eignen und Vorteile gegenüber den Verbindungen des Standes der Technik aufweisen.

Diese Aufgabe wird durch den Gegenstand der Patentansprüche gelöst. Es wurde überraschend gefunden, dass substituierte Cyclohexan-Derivate hergestellt werden können, welche eine Affinität an den μ-Opioid-Rezeptor und den ORL1-Rezeptor aufweisen.

Die Erfindung betrifft Verbindungen der allgemeinen Formel (1),

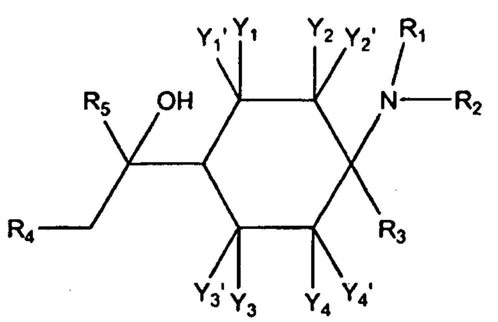

(1)

worin

$Y_1$, $Y_1$', $Y_2$, $Y_2$, $Y_3$, $Y_3$', $Y_4$ und $Y_4$' jeweils unabhängig voneinander ausgewählt sind aus der Gruppe bestehend aus -H, -F, -Cl, -Br, -I, -CN, $-NO_2$, -CHO, $-R_0$, $-C(=O)R_0$, -C(=O)-OH, $-C(=O)OR_0$, $-C(=O)NH_2$, $-C(=O)NHR_0$, $-C(=O)N(R_0)_2$, -OH, $-OR_0$, -OC(=O)H, $-OC(=O)R_0$, $-OC(=O)OR_0$, $-OC(=O)NHR_0$, $-OC(=O)N(R_0)_2$, -SH, $-SR_0$, $-S(=O)_{1-2}R_0$, $-S(=O)_{1-2}OH$, $-S(=O)_{1-2}OR_0$, $-S(=O)_{1-2}NH_2$, $-S(=O)_{1-2}NHR_0$ oder $-S(=O)_{1-2}N(R_0)_2$, $-NH_2$, $-NHR_0$, $-N(R_0)_2$, $-N^+(R_0)_3$, $-N^+(R_0)_2O^-$, $-NHC(=O)R_0$, $-NHC(=O)OR_0$, $-NHC(=O)NH_2$, $-NHC(=O)NHR_0$ und $-NHC(=O)N(R_0)_2$; oder $Y_1$ und $Y_1$', oder $Y_2$ und $Y_2$', oder $Y_3$ und $Y_3$', oder $Y_4$ und $Y_4$ gemeinsam für =O stehen;

$R_0$ jeweils unabhängig für $-C_{1-8}$-Aliphat, $-C_{3-12}$-Cycloaliphat, -Aryl, -Heteroaryl, $-C_{1-8}$-Aliphat-$C_{3-12}$-Cycloaliphat, $-C_{1-8}$-Aliphat-Aryl, $-C_{1-8}$-Aliphat-Heteroaryl, $-C_3$-8-Cycloaliphat-$C_{1-4}$-Aliphat, $-C_{3-8}$-Cycloaliphat-Aryl oder $-C_{3-8}$-Cycloaliphat-Heteroaryl steht;

$R_1$ und $R_2$ unabhängig voneinander für -H oder $-C_{1-8}$-Aliphat stehen; oder $R_1$ und $R_2$ zusammen einen Ring bilden und für $-(CH_2)_{2-4}$- stehen;

$R_3$ für $-R_0$ steht;

$R_4$ für -H, -F, -Cl, -Br, -I, -Aryl, -Heteroaryl, -C(=O)H, $-C(=O)R_0$, $-C(=O)OR_0$, -CN, $-C(=O)NH_2$, $-C(=O)NHR_0$, $-C(=O)N(R_0)_2$, -OH, $-OR_0$, -OC(=O)H, $-OC(=O)R_0$, $-OC(=O)OR_0$, $-OC(=O)NHR_0$, $-OC(=O)-N(R_0)_2$, $-NH_2$, $-NHR_0$, $-N(R_0)_2$, $-N^+(R_0)_3$, $-N^+(R_0)_2O^-$, $-NHC(=O)R_0$, $-NHC(=O)OR_0$, $-NHC(=O)NHR_0$, $-NHC(=O)-N(R_0)_2$, $-NO_2$, -SH, $-SR_0$, $-S(=O)_{1-2}R_0$, $-S(=O)_{1-2}OH$, $-S(=O)_{1-2}OR_0$, $-S(=O)_{1-2}NH_2$, $-S(=O)_{1-2}NHR_0$, $-S(=O)_{12}N(R_0)_2$, $-OS(=O)_{1-2}R_0$, $-OS(=O)_{1-2}OH$, $-OS(=O)_{1-2}OR_0$, $-OS(=O)_{1-2}NH_2$, $-OS(=O)_{1-2}NHR_0$ oder $-OS(=O)_{1-2}N(R_0)_2$ steht;

$R_5$ für -H, $-R_0$, -C(=O)H, $-C(=O)R_0$, $-C(=O)OR_0$, -CN, $-C(=O)NH_2$, $-C(=O)NHR_0$ oder $-C(=O)N(R_0)_2$ steht;

wobei

"Aliphat" jeweils ein verzweigter oder unverzweigter, gesättigter oder ein- oder mehrfach ungesättigter, unsubstituierter oder ein- oder mehrfach substituierter, aliphatischer Kohlenwasserstoffrest ist;

"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt;

wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, $-NO_2$, -CHO, =O, $-R_0$, $-C(=O)R_0$, -C(=O)OH, $-C(=O)OR_0$, $-C(=O)NH_2$, $-C(=O)NHR_0$, $-C(=O)N(R_0)_2$, -OH, $-OR_0$, -OC(=O)H, $-OC(=O)R_0$, $-OC(=O)OR_0$, $-OC(=O)NHR_0$, $-OC(=OFN(R_0)_2$, -SH, $-SR_0$, $-SO_3H$, $-S(=O)_{1-2}-R_0$, $-S(=O)_{1-2}NH_2$, $-NH_2$, $-NHR_0$, $-N(R_0)_2$, $-N^+(R_0)_3$, $-N^+(R_0)_2O^-$, $-NHC(=O)R_0$, $-NHC(=O)OR_0$, $-NHC(=O)NH_2$, $-NHC(=O)-NHR_0$, $-NH-C(=O)N(R_0)_2$, $-Si(R_0)_3$ und $-PO(OR_0)_2$ verstanden wird;

"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können, welche ihrerseits ein oder mehrere Heteroringatome aufweisen können, jeweils unabhängig voneinander ausgewählt aus N, 0 und S, und wobei jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;

"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;

wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, $-NO_2$, -CHO, =O, $-R_0$, $-C(=O)R_0$, -C(=O)OH, $-C(=O)OR_0$, $-C(=O)NH_2$, $-C(=O)NHR_0$, $-C(=O)-N(R_0)_2$, -OH, $-O(CH_2)_{1-2}O-$, $-OR_0$, -OC(=O)H, $-OC(=O)R_0$, $-OC(=O)OR_0$, $-OC(=O)NHR_0$, $-OC(=O)N(R_0)_2$, -SH, $-SR_0$, $-SO_3H$, $-S(=O)_{1-2}-R_0$, $-S(=O)_{12}NH_2$, $-NH_2$, $-NHR_0$, $-N(R_0)_2$, $-N^+(R_0)_3$, $-N^+(R_O)_2O^-$, $-NHC(=O)R_0$, $-NHC(=O)OR_0$, $-NH-C(=O)NH_2$, $-NHC(=O)NHR_0$, $-NHC(=O)N(R_0)_2$, $-Si(R_0)_3$ und $-PO(OR_0)_2$ verstanden wird; wobei ggf. vorhandene N-Ringatome jeweils oxidiert sein können;

in Form eines einzelnen Stereoisomers oder deren Mischung, der freien Verbindungen und/oder ihrer physiologisch verträglichen Salze.

Bei der Zusammenfassung verschiedener Reste, beispielsweise $R_1$ und $R_2$ sowie der Zusammenfassung von Resten an deren Substitueriten, wie z. B. $-OR_0$, $-OC(=O)R_0$, $-OC(=O)-NHR_0$, kann ein Substituent, z.B. $R_0$, für zwei oder mehrere Reste, beispielsweise $-OR_0$, $-OC(=O)R_0$, $-OC(=O)NHR_0$, innerhalb einer Substanz unterschiedliche Bedeutungen annehmen.

Die erfindungsgemäßen Verbindungen zeigen gute Bindung an den ORL1-Rezeptor und den μ-Opioid-Rezeptor.

In einer bevorzugten Ausführungsform weisen die erfindungsgemäßen Verbindungen ein Verhältnis ORL1/μ-Affinität von wenigstens 0,1 auf. Das ORL1/μ-Verhältnis ist definiert als $1/[K_{i(ORL1)}/K_{i(\mu)}]$. Besonders bevorzugt beträgt das ORL1/μ-Verhältnis wenigstens 0,2 oder wenigstens 0,5, bevorzugter wenigstens 1,0 oder wenigstens 2,0, noch bevorzugter wenigstens 3,0 oder wenigstens 4,0, am bevorzugtesten wenigstens 5,0 oder wenigstens 7,5 und insbesondere wenigstens 10 oder wenigstens 15. In einer bevorzugten Ausführungsform liegt das ORL1/μ-Verhältnis im Bereich von 0,1 bis 30, bevorzugter 0,1 bis 25.

In einer anderen bevorzugten Ausführungsform weisen die erfindungsgemäßen Verbindungen ein Verhältnis ORL1/μ-Affinität von mehr als 30, bevorzugter wenigstens 50, noch bevorzugter wenigstens 100, am bevorzugtesten wenigstens 200 und insbesondere wenigstens 300 auf.

Die erfindungsgemäßen Verbindungen weisen bevorzugt einen $K_i$-Wert am μ-Opioid-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des $K_i$-Werts am μ-Opioid-Rezeptor sind dem Fachmann bekannt. Bevorzugt erfolgt die Bestimmung wie im Zusammenhang mit den Beispielen beschrieben.

Die erfindungsgemäßen Verbindungen weisen bevorzugt einen $K_i$-Wert am ORL1-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des $K_i$-Werts am ORL1-Rezeptor sind dem Fachmann bekannt. Bevorzugt erfolgt die Bestimmung wie im Zusammenhang mit den Beispielen beschrieben.

Überraschenderweise hat sich gezeigt, dass Verbindungen mit Affinität zum ORL1- und μ-Opioid-Rezeptor, bei denen das durch $1/[K_{i(ORL1)}/K_{i(\mu)}]$ definierte Verhältnis von ORL1 zu μ im Bereich von 0,1 bis 30 liegt, vorzugsweise von 0,1 bis 25, ein pharmakologisches Profil aufweisen, das verglichen mit dem anderer Opioidrezeptorliganden deutliche Vorteile aufweist:

1. Die erfindungsgemäßen Verbindungen zeigen eine Wirksamkeit in Akutschmerzmodellen, die mitunter vergleichbar ist mit der gebräuchlicher Stufe-3 Opioide. Gleichzeitig zeichnen sie sich aber durch eine gegenüber klassischen μ-Opioiden deutlich bessere Verträglichkeit aus.

2. Im Gegensatz zu gebräuchlichen Stufe-3 Opioiden zeigen die erfindungsgemäßen Verbindungen eine deutlich höhere Wirksamkeit in Mono- und Polyneuropathieschmerzmodellen, was auf einen Synergismus von ORL1- und μ-Opioid Komponente zurückzuführen ist.

3. Im Gegensatz zu gebräuchlichen Stufe-3 Opioiden zeigen die erfindungsgemäßen Verbindungen in neuropathischen Tieren eine weitgehende, bevorzugt eine vollständige, Trennung von antiallodynischer bzw. antihyperalgetischer Wirkung und antinociceptivem Effekt.

4. Im Gegensatz zu gebräuchlichen Stufe-3 Opioiden zeigen die erfindungsgemäßen Verbindungen in Tiermodellen für chronischen Entzündungsschmerz (u.a. Carrageenan- oder CFA-induzierte Hyperalgesie, viszeraler Entzündungsschmerz) eine deutliche Wirkverstärkung gegenüber dem Akutschmerz.

5. Im Gegensatz zu gebräuchlichen Stufe-3 Opioiden sind μ-opioidtypische Nebenwirkungen (u.a. Atemdepression, opioidinduzierte Hyperalgesie, körperliche Abhängigkeit/Entzug, psychische Abhängigkeit/Sucht) bei den erfindungsgemäßen Verbindungen im therapeutisch wirksamen Dosisbereich deutlich reduziert, bzw. vorzugsweise

nicht zu beobachten.

Aufgrund der reduzierten μ-opioiden Nebenwirkungen einerseits und der erhöhten Wirksamkeit bei chronischem, bevorzugt neuropathischem Schmerz andererseits zeichnen sich die gemischten ORL1/μ-Agonisten somit durch deutlich vergrößerte Sicherheitsabstände gegenüber reinen μ-Opioiden aus. Daraus resultiert ein deutlich vergrößertes "therapeutisches Fenster" bei der Behandlung von Schmerzzuständen, bevorzugt chronischen Schmerzen, noch bevorzugter neuropathischen Schmerzen.

Bevorzugt sind $Y_1$, $Y_1$', $Y_2$, $Y_2$', $Y_3$, $Y_3$', $Y_4$ und $Y_4$' jeweils unabhängig voneinander ausgewählt aus der Gruppe bestehend aus -H, -F, -Cl, -Br, -I, -CN, -$NH_2$, -NH-$C_{1-6}$-Aliphat, -NH-$C_{38}$-Cycloaliphat, -NH-$C_{1-6}$-Aliphat-OH, -N($C_{1-6}$-Aliphat)$_2$, -N($C_{3-8}$-Ccyloaliphat)$_2$, -N($C_{1-6}$-Aliphat-OH)$_2$, -$NO_2$, -NH-$C_{1-6}$-Aliphat-$C_{3-8}$-Cycloaliphat, -NH-$C_{1-6}$-Aliphat-Aryl, -NH-$C_{1-6}$-Aliphat-Heteroaryl, -NH-Aryl, -NH-Heteroaryl, -SH, -S-$C_{1-6}$-Aliphat, -S-$C_{3-8}$-Cycloaliphat, -S-$C_{1-6}$-Aliphat-$C_{38}$-Cycloaliphat, -S-$C_{1-6}$-Aliphat-Aryl, -S-$C_{1-6}$-Aliphat-Heteroaryl, -S-Aryl, -S-Heteroaryl, -OH, -O-$C_{1-6}$-Aliphat, -O-$C_{3-8}$-Cycloaliphat, -O-$C_{1-6}$-Aliphat-OH, -O-$C_{1-6}$-Aliphat-$C_{3-8}$-Cycloaliphat, -O-$C_{1-6}$-Aliphat-Aryl, -O-$C_{1-6}$-Aliphat-Heretoaryl, -O-Aryl, -O-Heteroaryl, -O-C(=O)$C_{1-6}$-Aliphat, -O-C(=O)$C_{3-8}$-Cycloaliphat, -O-C(=O)$C_{1-6}$-Aliphat-OH, -O-C(=O)$C_{1-6}$-Aliphat-$C_{3-8}$-Cycloaliphat, -O-C(=O)$C_{1-6}$-Aliphat-Aryl, -O-C(=O)$C_{1-6}$-Aliphat-Heretoaryl, -O-C(=O)Aryl, -O-C(=O)Heteroaryl, -$C_{1-6}$-Aliphat, -$C_{3-8}$-Cycloaliphat, -$C_{1-6}$-Aliphat-$C_{3-8}$-Cycloaliphat, -$C_{1-6}$-Aliphat-Aryl, -$C_{16}$-Aliphat-Heteroaryl, -Aryl, -Heteroaryl, -C(=O)$C_{1-6}$-Aliphat, -C(=O)$C_{3-8}$-Cycloaliphat, -C(=O)$C_{1-6}$-Aliphat-$C_{3-8}$-Cycloaliphat, -C(=O)$C_{1-6}$-Aliphat-Aryl, -C(=O)$C_{1-6}$-Aliphat-Heteroaryl, -C(=O)Aryl, -C(=O)Heteroaryl, -$CO_2$H, -$CO_2$-$C_{1-6}$-Aliphat, -$CO_2$-$C_{3-8}$-Cycloaliphat, -$CO_2$-$C_{1-6}$-Aliphat-$C_{3-8}$-Cycloaliphat, -$CO_2$-$C_{1-6}$-Aliphat-Aryl, -$CO_2$-$C_{1-6}$-Aliphat-Heteroaryl, -$CO_2$-Aryl, -$CO_2$-Heteroaryl; oder $Y_1$ und $Y_1$', oder $Y_2$ und $Y_2$', oder $Y_3$ und $Y_3$', oder $Y_4$ und $Y_4$' stehen gemeinsam für =O. Bevorzugt sind $Y_1$, $Y_1$', $Y_2$, $Y_2$', $Y_3$, $Y_3$', $Y_4$ und $Y_4$' jeweils unabhängig voneinander ausgewählt aus der Gruppe bestehend aus -H, -F, -Cl, -Br, -I, -CN, -$NH_2$ und -OH.

In einer bevorzugten Ausführungsform ist einer der Reste $Y_1$, $Y_1$', $Y_2$, $Y_2$', $Y_3$, $Y_3$', $Y_4$ und $Y_4$' ungleich -H und die übrigen Reste stehen für -H.

Besonders bevorzugt stehen $Y_1$, $Y_1$', $Y_2$, $Y_2$', $Y_3$, $Y_3$', $Y_4$ und $Y_4$' jeweils für -H.

$R_0$ steht bevorzugt jeweils unabhängig für -$C_{1-8}$-Aliphat, -$C_{3-12}$-Cycloaliphat, -Aryl, -Heteroaryl, -$C_{1-8}$-Aliphat-$C_{3-12}$-Cycloaliphat, -$C_{1-8}$-Aliphat-Aryl oder -$C_{1-8}$-Aliphat-Heteroaryl. Dabei bedeuten -$C_{1-8}$-Aliphat-$C_{3-12}$-Cycloaliphat, -$C_{1-8}$-Aliphat-Aryl oder -$C_{1-8}$-Aliphat-Heteroaryl, dass die Reste -$C_{3-12}$-Cycloaliphat, -Aryl oder -Heteroaryl jeweils über eine zweibindige Brücke -$C_{1-8}$-Aliphat- gebunden sind. Bevorzugte Beispiele für -$C_{1-8}$-Aliphat-Aryl sind -$CH_2$-$C_6H_5$, -$CH_2CH_2$-$C_6H_5$, und -CH=CH-$C_6H_5$.

$R_1$ und $R_2$ stehen unabhängig voneinander für -H; -$C_{1-5}$-Aliphat; oder die Reste $R_1$ und $R_2$ bilden zusammen einen Ring und bedeuten -$(CH_2)_2$-, -$(CH_2)_3$- oder -$(CH_2)_4$-. Bevorzugt stehen $R_1$ und $R_2$ unabhängig voneinander jeweils für -H, -$CH_3$ oder -$CH_2CH_3$, oder $R_1$ und $R_2$ bilden gemeinsam einen Ring und stehen für -$CH_2CH_2$- oder -$CH_2CH_2CH_2$-. Besonders bevorzugt sind Verbindungen, worin $R_1$ und $R_2$ unabhängig voneinander für -$CH_3$ oder -H stehen, wobei $R_1$ und $R_2$ nicht gleichzeitig -H bedeuten. In einer bevorzugten Ausführungsform ist $R_1$ = $R_2$. In einer anderen bevorzugten Ausführungsform ist $R_1$ # $R_2$. Bevorzugt bilden $R_1$ und $R_2$ zusammen mit dem Stickstoffatom, an welches sie gebunden sind, eine der folgenden funktionellen Gruppen:

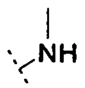 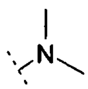 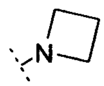 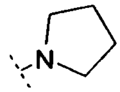

Bevorzugt steht $R_3$ für -$C_{1-8}$-Aliphat, -$C_{3-8}$-Cycloaliphat, -Aryl, -Heteroaryl; oder für jeweils über eine -$C_{1-3}$-Aliphat-Gruppe gebundenes -Aryl, -Heteroaryl oder -$C_{3-8}$-Cycloaliphat.

Bevorzugt steht $R_3$ für -Ethyl, -Propyl, -Butyl, -Pentyl, -Hexyl, -Heptyl, -Cyclopentyl, -Cyclohexyl, - Phenyl, -Naphthyl, -Anthracenyl, -Thiophenyl, -Bezothiophenyl, - Furyl, -Thienyl, -Thiazolyl, -Benzofuranyl, -Benzodioxolanyl, -Indolyl, -Indanyl, -Benzodioxanyl, -Pyrrolyl, -Pyridyl, -Pyrimidyl oder -Pyrazinyl, jeweils unsubstituiert oder ein- oder mehrfach substituiert; über eine gesättigte, unverzweigte -$C_{1-3}$-Aliphat-Gruppe gebundenes -Cyclopentyl,- Cyclohexyl, - Phenyl, -Naphthyl, -Anthracenyl, -Thiophenyl, -Bezothiophenyl, - Furyl, -Thienyl, -Thiazolyl, -Benzofuranyl, -Benzodioxolanyl, -Indolyl, -Indanyl, -Benzodioxanyl, -Pyrrolyl, -Pyridyl, -Pyrimidyl oder -Pyrazinyl, jeweils unsubstituiert oder ein- oder mehrfach substituiert.

Bevorzugter steht $R_3$ für -Ethyl, -Propyl, -Butyl, -Pentyl, -Hexyl, -Heptyl, -Cyclopentyl, -Cyclohexyl, -Phenyl, -Benzyl, -Naphthyl, -Anthracenyl, -Thiophenyl, -Benzothiophenyl, -Furyl, -Thienyl, -Thiazolyl, -Benzofuranyl, -Benzodioxolanyl, -Indolyl, -Indanyl, -Benzodioxanyl, -Pyrrolyl, -Pyridyl, -Pyrimidyl oder -Pyrazinyl, jeweils unsubstituiert oder ein- oder mehrfach substituiert; über eine gesättigte, unverzweigte -$C_{1-3}$-Aliphat-Gruppe gebundenes -$C_{5-6}$-Cycloaliphat, -Phenyl, -Naphthyl, -Anthracenyl, -Thiophenyl, -Benzothiophenyl, -Pyridyl, -Furyl, -Thienyl, -Thiazolyl, -Benzofuranyl, -Benzodioxolanyl, -Indolyl, -Indanyl, -Benzodioxanyl, -Pyrrolyl, -Pyrimidyl, -Triazolyl oder -Pyrazinyl, jeweils unsubstituiert

oder ein- oder mehrfach substituiert.

Bevorzugter steht $R_3$ für -Propyl, -Butyl, -Pentyl, -Hexyl, -Phenyl, -Thiophenyl, -Furyl, -Thienyl, -Thiazolyl, -Naphthyl, -Benzyl, -Benzofuranyl, -Indolyl, -Indanyl, -Benzodioxanyl, -Benzodioxolanyl, -Pyridyl, -Pyrimidyl, -Pyrazinyl, -Triazolyl oder -Benzothiophenyl, jeweils unsubstituiert oder ein- oder mehrfach substituiert; über eine gesättigte, unverzweigte -$C_{1-3}$-Aliphat-Gruppe gebundenes -Phenyl, -Furyl, -Thienyl oder -Thiazolyl, jeweils unsubstituiert oder ein- oder mehrfach substituiert.

Noch bevorzugter steht $R_3$ für -Propyl, -Butyl, -Pentyl, -Hexyl, -Phenyl, -Phenethyl, -Thiophenyl, -Pyridyl, -Triazolyl, -Benzothiophenyl oder -Benzyl, jeweils substituiert oder unsubstituiert, besonders bevorzugt für -Propyl, -3-Methoxypropyl, -Butyl, -Pentyl, -Hexyl, -Phenyl, -3-Methylphenyl, -3-Fluorphenyl, -Benzo[1,3]-dioxolyl, -Thienyl, -Benzothiophenyl, -4-Chlorbenzyl, -Benzyl, -3-Chlorbenzyl, -4-Methylbenzyl, -2-Chlorbenzyl, -4-Fluorbenzyl, -3-Methylbenzyl, -2-Methylbenzyl, -3-Fluorbenzyl, -2-Fluorbenzyl, -1-Methyl-1,2,4-triazolyl oder -Phenethyl.

Ganz besonders bevorzugt steht $R_3$ für -Butyl, -Ethyl, -3-Methoxypropyl, -Benzothiophenyl, -Phenyl, -3-Methylphenyl, -3-Fluorphenyl, -Benzo[1,3]-dioxolyl, -Benzyl, -1-Methyl-1,2,4-triazolyl, -Thienyl oder -Phenethyl.

Am bevorzugtesten steht $R_3$ für -Phenyl, -Benzyl oder -Phenethyl, jeweils unsubstituiert oder am Ring ein- oder mehrfach substituiert; -$C_{1-5}$-Aliphat, -$C_{4-6}$-Cycloaliphat, -Pyridyl, -Thienyl,- Thiazolyl, -Imidazolyl, -1,2,4-Triazolyl oder -Benzimidazolyl, unsubstituiert oder ein- oder mehrfach substituiert.

Insbesondere bevorzugt steht $R_3$ für -Phenyl, -Benzyl, -Phenethyl, -Thienyl, -Pyridyl, -Thiazolyl, -Imidazolyl, -1,2,4 Triazolyl, -Benzimidazolyl oder -Benzyl, unsubstituiert oder ein- oder mehrfach substituiert mit -F, -Cl, -Br, -CN, -$CH_3$, -$C_2H_5$, -$NH_2$, -$NO_2$, -SH, -$CF_3$, -OH, -$OCH_3$, -$OCH_5$ oder -$N(CH_3)_2$; -Ethyl, -n-Propyl, -2-Propyl, -Allyl, -n-Butyl, -iso-Butyl, -sec.-Butyl, -tert.-Butyl, -n-Pentyl, -iso-Pentyl, -neo-Pentyl, -n-Hexyl, -Cyclopentyl oder -Cyclohexyl, jeweils unsubstituiert oder ein- oder mehrfach substituiert mit -OH, -$OCH_3$ oder -$OC_2H_5$, wobei vorzugsweise -Thienyl, -Pyridyl, -Thiazolyl, -Imidazolyl, -1,2,4 Triazolyl und -Benzimidazolyl unsubstituiert sind.

In einer bevorzugten Ausführungsform steht $R_3$ für -$C_{1-6}$-Aliphat, -Aryl (bevorzugt -Phenyl) oder Heteroaryl (bevorzugt -Thienyl, -Thiazolyl oder -Pyridyl), wobei -Aryl und -Heteroaryl jeweils unsubstituiert oder ein- oder mehrfach substituiert sind mit Substituenten unabhängig voneinander ausgewählt aus -F, -Cl, -Br, -I, -$CH_3$, -$OCH_3$ und -OH.

Insbesondere bevorzugt steht $R_3$ für-Phenyl, unsubstituiert oder einfach substituiert mit -F, -Cl, -CN, -$CH_3$; -Thienyl; -Ethyl, -n-Propyl oder -n-Butyl, unsubstituiert oder ein- oder mehrfach substituiert mit -$OCH_3$, -OH oder -$OC_2H_5$, insbesondere mit -$OCH_3$.

Bevorzugt steht $R_4$ für -H, -F, -Cl, -Br, -I, -Aryl, -Heteroaryl, -C(=O)H, -C(=O)-$C_{1-8}$-Aliphat, -C(=O)-$C_{3-12}$-Cycloaliphat, -C(=O)-Aryl, -C(=O)-Heteroaryl, -C(=O)-$C_{1-8}$-Aliphat-$C_{3-12}$-Cycloaliphat, -C(=O)-$C_{1-8}$-Aliphat-Aryl, -C(=O)-$C_{1-8}$-Aliphat-Heteroaryl, -C(=O)O-$C_{1-8}$-Aliphat, -C(=O)O-$C_{3-12}$-Cycloaliphat, -C(=O)O-Aryl, -C(=O)O-Heteroaryl, -C(=O)O-$C_{1-8}$-Aliphat-$C_{3-12}$-Cycloaliphat, -C(=O)O-$C_{1-8}$-Aliphat-Aryl, -C(=O)O-$C_{1-8}$-Aliphat-Heteroarl, -CN, -C(=O)$NH_2$, -C(=O)-NH-$C_{1-8}$-Aliphat, -C(=O)NH-$C_{3-12}$-Cycloaliphat, -C(=O)NH-Aryl, -C(=O)-NH-Heteroaryl, -C(=O)-NH-$C_{1-8}$-Aliphat-$C_{3-12}$-Cycloaliphat, -C(=O)NH-$C_{1-8}$-Aliphat-Aryl, -C(=O)NH-$C_{1-8}$-Aliphat-Heteroaryl, -C(=O)N($C_{1-8}$-Aliphat)$_2$, -C(=O)N($C_{3-12}$-Cycloaliphat)$_2$, -C(=O)N(Aryl)$_2$, -C(=O)N-(Heteroaryl)$_2$, -C(=O)N($C_{1-8}$-Aliphat-$C_{3-12}$-Cycloaliphat)$_2$, -C(=O)-N($C_{1-8}$-Aliphat-Aryl)$_2$, -C(=O)-N($C_{1-8}$-Aliphat-Heteroaryl)$_2$, -OH, -O$C_{1-8}$-Aliphat, -O$C_{3-12}$-Cycloaliphat, -OAryl, -OHeteroaryl, -O$C_{1-8}$-Aliphat-$C_{3-12}$-Cycloaliphat, -O$C_{1-8}$-Aliphat-Aryl, -O$C_{1-8}$-Aliphat-Heteroaryl, -OC(=O)H, -OC(=O)-$C_{1-8}$-Aliphat, -OC(=O)-$C_{3-12}$-Cycloaliphat, -OC(=O)-Aryl, -OC(=O)-Heteroary), -OC(=O)-$C_{1-8}$-Aliphat-$C_{3-12}$-Cycloaliphat, -OC(=O)-$C_{1-8}$-Aliphat-Aryl, -OC(=O)-$C_{1-8}$-Aliphat-Heteroaryl, -OC(=O)O-$C_{1-8}$-Aliphat, -OC(=O)O-$C_{3-12}$-Cycloaliphat, -OC(=O)O-Aryl, -OC(=O)O-Heteroaryl, -OC(=O)O-$C_{1-8}$-Aliphat-$C_{3-12}$-Cycloaliphat, -OC(=O)-O-$C_{1-8}$-Aliphat-Aryl,- OC(=O)-O-$C_{1-8}$-Aliphat-Heteroaryl, -OC(=O)NH-$C_{1-8}$-Aliphat, -OC(=O)-NH-$C_{3-12}$-Cycloaliphat, -OC(=O)NH-Aryl, -OC(=O)NH-Heteroaryl, -OC(=O)NH-$C_{1-8}$-Aliphat-$C_{312}$-Cycloaliphat, -OC(=O)NH-$C_{1-8}$-Aliphat-Aryl, -OC(=O)NH-$C_{1-8}$-Aliphat-Heteroaryl, -OC(=O)-N($C_{1-8}$-Aliphat)$_2$, -OC(=O)N($C_{3-12}$-Cycloaliphat)$_2$, -OC(=O)N(Aryl)$_2$, -OC(=O)-N(Heteroaryl)$_2$, -OC(=O)N($C_{1-8}$-Aliphat-$C_{3-12}$-Cycloaliphat)$_2$, -OC(=O)N($C_{1-8}$-Aliphat-Aryl)$_2$, -OC(=O)N($C_{1-8}$-Aliphat-Heteroaryl)$_2$, -$NH_2$, -$NO_2$, -NH-$C_{1-8}$-Aliphat, -NH-$C_{3-12}$-Cycloaliphat, -NH-Aryl, -NH-Heteroaryl, -NH-$C_{1-8}$-Aliphat-$C_{3-12}$-Cycloaliphat, -NH-$C_{1-8}$-Aliphat-Aryl, -NH-$C_{1-8}$-Aliphat-Heteroaryl, -N($C_{1-8}$-Aliphat)$_2$, -N($C_{3-12}$-Cycloaliphath, -N(Aryl)$_2$, -N(Heteroaryl)$_2$, -N($C_{1-8}$-Aliphat-$C_{312}$-Cycloaliphat)$_2$, -N($C_{1-8}$-Aliphat-Aryl)$_2$, -N($C_{1-8}$-Aliphat-Heteroaryl)$_2$, -NHC(=O)-$C_{1-8}$-Aliphat, -NHC(=O)-$C_{3-12}$-Cycloaliphat, -NHC(=O)-Aryl, -NHC(=O)-Heteroaryl, -NHC(=O)-$C_{1-8}$-Aliphat-$C_{3-12}$-Cycloaliphat, -NHC(=O)-$C_{1-8}$-Aliphat-Aryl, -NHC(=O)-$C_{1-8}$-Aliphat-Heteroaryl, -NHC(=O)-O-$C_{1-8}$-Aliphat, -NHC(=O)O-$C_{3-12}$-Cycloaliphat, -NHC(=O)O-Aryl, -NHC(=O)O-Heteroaryl, -NHC(=O)O-$C_{1-8}$-Aliphat-$C_{3-12}$-Cycloaliphat, -NHC(=O)O-$C_{1-8}$-Aliphat-Aryl, -NHC(=O)O-$C_{1-8}$-Aliphat-Heteroaryl, -NHC(=O)NH-$C_{1-8}$-Aliphat, -NHC(=O)NH-$C_{3-12}$-Cycloaliphat, -NHC(=O)-NH-Aryl, -NHC(=O)-NH-Heteroaryl, -NHC(=O)NH-$C_{1-8}$-Aliphat-$C_{3-12}$-Cycloaliphat, -NHC(=O)-NH-$C_{1-8}$-Aliphat-Aryl, -NHC(=O)NH-$C_{1-8}$-Aliphat-Heteroaryl, -NHC(=O)N($C_{1-8}$-Aliphat)$_2$. -NHC(=O)N($C_{3-12}$-Cycloaliphat)$_2$, -NHC(=O)N(Aryl)$_2$, -NHC(=O)-N(Heteroaryl)$_2$, -NHC(=O)-N($C_{1-8}$-Aliphat-$C_{3-12}$-Cycloaliphath, -NHC(=O)N($C_{1-8}$-Aliphat-Aryl)$_2$, -NHC(=O)N($C_{1-8}$-Aliphat-Heteroaryl)$_2$, -SH, -S$C_{1-8}$-Aliphat, -S$C_{3-12}$-Cycloaliphat, -SAryl, -SHeteroaryl, -S$C_{1-8}$-Aliphat-$C_{312}$-Cycloaliphat, -S$C_{1-8}$-Aliphat-Aryl, -S$C_{1-8}$-Aliphat-Heteroaryl, -S(=O)$_{1-2}$$C_{1-8}$-Aliphat, -S(=O)$_{12}$$C_{3-12}$-Cycloaliphat, -S(=O)$_{1-2}$Aryl, -S(=O)$_{1-2}$Heteroaryl, -S(=O)$_{1-2}$$C_1$8-Aliphat-$C_{3-12}$-Cycloaliphat, -S(=O)$_{1-2}$$C_{1-8}$-Aliphat-Aryl, -S(=O)$_{1-2}$$C_{1-8}$-Aliphat-Heteroaryl, -S(=O)$_{1-2}$OH, -S(=O)$_{1-2}$O$C_{18}$-Aliphat,

$-S(=O)_{1-2}OC_{3-12}$-Cycloaliphat, $-S(=O)_{1-2}$OAryl, $-S(=O)_{1-2}$OHeteroaryl, $-S(=O)_{1-2}OC_{1-8}$-Aliphat-$C_{3-12}$-Cycloaliphat, $-S(=O)_{1-2}OC_{1-8}$-Aliphat-Aryl, $-S(=O)_{1-2}OC_{1-8}$-Aliphat-Heteroaryl, $-S(=O)_{1-2}NH_2$, $-S(=O)_{1-2}NHC_{1-8}$-Aliphat, $-S(=O)_{1-2}NHC_{3-12}$-Cycloaliphat, $-S(=O)_{1-2}$NHAryl, $-S(=O)_{1-2}$NHHeteroaryl, $-S(=O)_{1-2}NHC_{1-8}$-Aliphat-$C_{3-12}$-Cycloaliphat, $-S(=O)_{1-2}NHC_{1-8}$-Aliphat-Aryl, $-S(=O)_{1-2}NHC_{1-8}$-Aliphat-Heteroaryl, $-S(=O)_{1-2}N(C_{1-8}$-Aliphat$)_2$, $-S(=O)_{1-2}N(C_{3-12}$-Cycloaliphat$)_2$, $-S(=O)_{1-2}N(Aryl)_2$, $-S(=O)_{1-2}N(Heteroaryl)_2$, $-S(=O)_{1-2}N(C_{1-8}$-Aliphat-$C_{3-12}$-Cycloaliphat$)_2$, $-S(=O)_{1-2}N(C_{1-8}$-Aliphat-Aryl$)_2$, $-S(=O)_{1-2}N(C_{1-8}$-Aliphat-Heteroaryl$)_2$, $-OS(=O)_{1-2}C_{1-8}$-Aliphat, $-OS(=o)_{1-2}C_{3-12}$-Cycloaliphat, $-OS(=O)_{1-2}$Aryl, $-OS(=O)_{1-2}$Heteroaryl, $-OS(=O)_{1-2}C_{1-8}$-Aliphat-$C_{3-12}$-Cycloaliphat, $-OS(=O)_{1-2}C_{1-8}$-Aliphat-Aryl, $-OS(=O)_{1-2}C_{1-8}$-Aliphat-Heteroaryl, $-OS(=O)_{1-2}OH$, $-OS(=O)_{1-2}OC_{1-8}$-Aliphat, $-OS(=O)_{1-2}OC_{3-12}$-Cycloaliphat, $-OS(=O)_{1-2}$OAryl, $-OS(=O)_{1-2}$OHeteroaryl, $-OS(=O)_{1-2}OC_{1-8}$-Aliphat-$C_{3-12}$-Cycloaliphat, $-OS(=O)_{1-2}OC_{1-8}$-Aliphat-Aryl, $-OS(=O)_{1-2}OC_{1-8}$-Aliphat-Heteroaryl, $-OS(=O)_{1-2}NH_2$, $-OS(=O)_{1-2}NHC_{1-8}$-Aliphat, $-O-S(=O)_{1-2}NHC_{3-12}$-Cycloaliphat, $-OS(=O)_{1-2}$NHAryl, $-OS(=O)_{1-2}$NHHeteroaryl, $-OS(=O)_{1-2}NHC_{18}$-Aliphat-$C_{3-12}$-Cycloaliphat, $-OS(=O)_{1-2}NHC_{1-8}$-Aliphat-Aryl, $-OS(=O)_{1-2}NHC_{1-8}$-Aliphat-Heteroaryl, $-OS(=O)_{1-2}N(C_{1-8}$-Aliphat$)_2$, $-OS(=O)_{1-2}N(C_{3-12}$-Cycloaliphat$)_2$, $-OS(=O)_{1-2}N(Aryl)_2$,- $OS(=O)_{1-2}N(Heteroaryl)_2$, $-OS(=O)_{1-2}N(C_{1-8}$-Aliphat-$C_{3-12}$-Cycloaliphat$)_2$, $-OS(=O)_{1-2}N(C_{1-8}$-Aliphat-Aryl$)_2$ oder $-OS(=O)_{1-2}N(C_{1-8}$-Aliphat-Heteroaryl$)_2$.

Bevorzugter ist $R_4$, -Aryl, -Heteroaryl, -O-$C_{1-8}$-Aliphat, -O-$C_{312}$-Cycloaliphat, -O-Aryl, -O-Heteroaryl, -NH-$C_{1-8}$-Aliphat, -NH-$C_{3-12}$-Cycloaliphat, -NH-Aryl, -NH-Heteroaryl, $-N(C_{1-8}$-Aliphat$)_2$, $-N(C_{3-12}$-Cycloaliphat$)_2$, $-N(Aryl)_2$, $-N(Heteroaryl)_2$, -S-$C_{1-8}$-Aliphat, -S-$C_{3-12}$-Cycloaliphat, -S-Aryl oder -S-Heteroaryl.

Noch bevorzugter steht $R_4$ für -Aryl, -Heteroaryl, -O-$C_{1-8}$-Aliphat, -O-$C_{3-12}$-Cycloaliphat, -O-Aryl, -O-Heteroaryl, -NH-$C_{1-8}$-Aliphat, -NH-$C_{3-12}$-Cycloaliphat, -NH-Aryl, -NH-Heteroaryl, $-N(C_{1-8}$-Aliphat$)_2$, $-N(C_{3-12}$-Cycloaliphat$)_2$, $-N(Aryl)_2$, $-N(Heteroaryl)_2$, -S-$C_{1-8}$-Aliphat, -S-$C_{3-12}$-Cycloaliphat oder -S-Aryl oder -S-Heteroaryl.

Besonders bevorzugt steht $R_4$ für -Aryl (vorzugsweise Phenyl. ggf. substituiert), -O-Aryl (vorzugsweise -O-Phenyl, ggf. substituiert) oder -Heteroaryl (vorzugsweise -Indolyl oder -Indanyl, jeweils ggf. substituiert). In einer besonders bevorzugten Ausführungsform steht $R_4$ für-Aryl, -Heteroaryl, -O-Aryl, -O-Heteroaryl, -O-$C_{3-12}$-Cycloaliphat, -NH-Aryl, -NH-Heteroaryl, -NH-$C_{3-12}$-Cycloaliphat, $-N(Aryl)_2$, $-N(Heteroaryl)_2$, $-N(C_{3-12}$-Cycloaliphat$)_2$, -S-Aryl, -S-Heteroaryl oder -S-$C_{3-12}$-Cycloaliphat; wobei -Aryl, -Heteroaryl besonders bevorzugt sind.

Bevorzugte Beispiele für $R_4$ sind nachfolgend abgebildet:

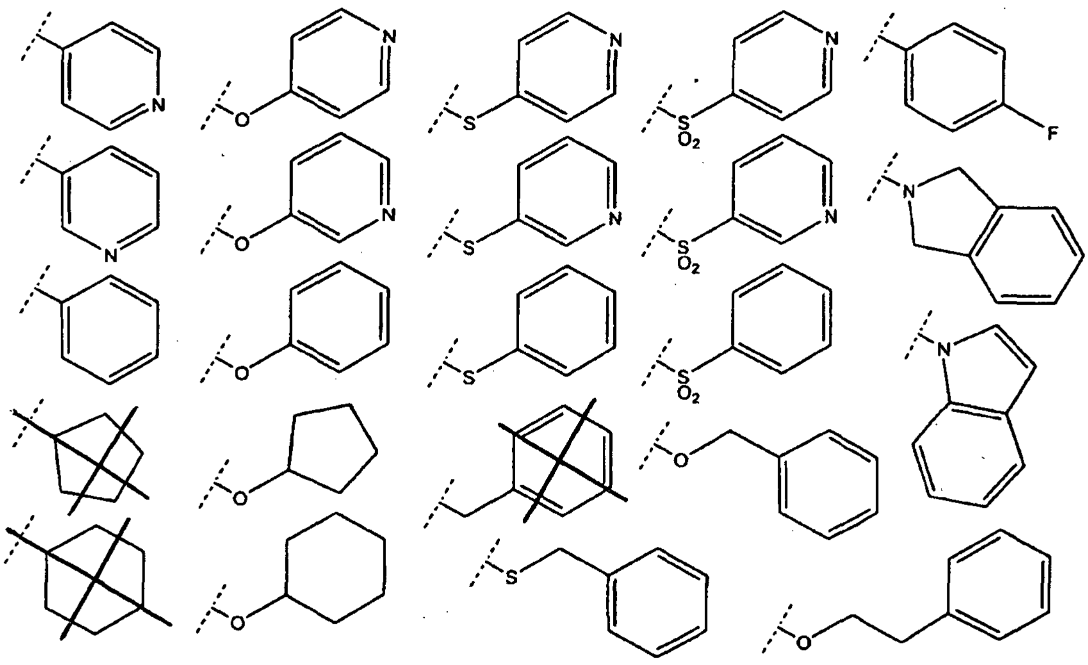

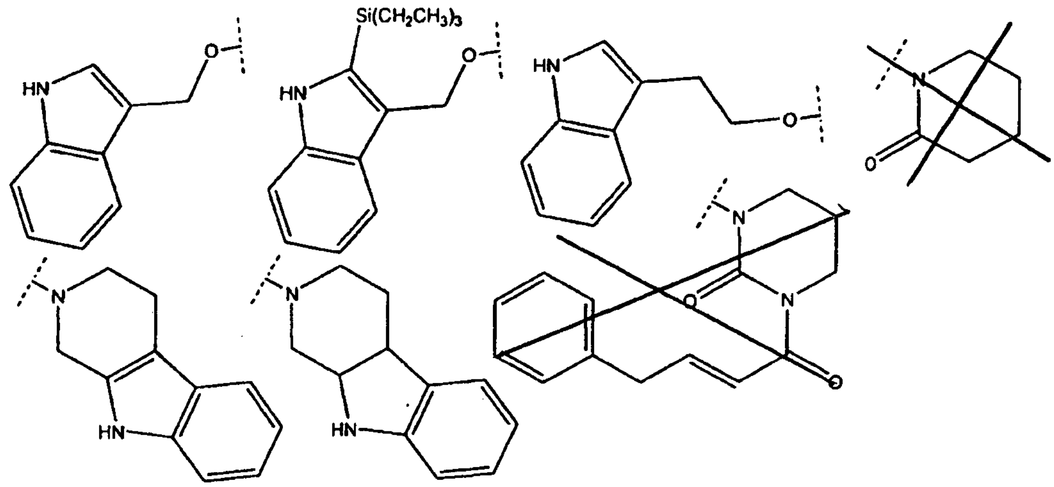

Bevorzugt steht $R_5$ für -H, -$C_{1-8}$-Aliphat, -$C_{3-12}$-Cycloaliphat, -Aryl, -Heteroaryl, -$C_{1-8}$-Aliphat-$C_{3-12}$-Cycloaliphat, -$C_{1-8}$-Aliphat-Aryl, -$C_{1-8}$-Aliphat-Heteroaryl, -C(=O)H, -C(=O)-$C_{1-8}$-Aliphat, -C(=O)-$C_{3-12}$-Cycloaliphat, -C(=O)-Aryl, -C(=O)-Heteroaryl, -C(=O)-$C_{1-8}$-Aliphat-$C_{3-12}$-Cycloaliphat, -C(=O)-$C_{1-8}$-Aliphat-Aryl, -C(=O)-$C_{1-8}$-Aliphat-Heteroaryl, -C(=O)O-$C_{1-8}$-Aliphat, -C(=O)O-$C_{3-12}$-Cycloaliphat, -C(=O)O-Aryl, -C(=O)O-Heteroaryl, -C(=O)O-$C_{1-8}$-Aliphat-$C_{3-12}$-Cycloaliphat, -C(=O)O-$C_{1-8}$-Aliphat-Aryl, -C(=O)O-$C_{1-8}$-Aliphat-Heteroarl, -CN, -C(=O)$NH_2$, -C(=O)-NH-$C_{1-8}$-Aliphat, -C(=O)NH-$C_{3-12}$-Cycloaliphat, -C(=O)NH-Aryl, -C(=O)NH-Heteroaryl, -C(=O)-NH-$C_{1-8}$-Aliphat-$C_{3-12}$-Cycloaliphat, -C(=O)NH-$C_{1-8}$-Aliphat-Aryl, -C(=O)NH-$C_{1-8}$-Aliphat-Heteroaryl, -C(=O)N($C_{1-8}$-Aliphat)$_2$, -C(=O)N($C_{3-12}$-Cycloaliphat)$_2$, -C(=O)N(Aryl)$_2$, -C(=O)N-(Heteroaryl)$_2$, -C(=O)N($C_{1-8}$-Aliphat-$C_{3-12}$-Cycloaliphat)$_2$, -C(=O)N($C_{1-8}$-Aliphat-Aryl)$_2$ oder -C(=O)-N($C_{1-8}$-Aliphat-Heteroaryl)$_2$.

Bevorzugter ist $R_5$ ausgewählt aus -H, -$C_{1-8}$-Aliphat, -$C_{3-12}$-Cycloaliphat, -$C_{1-8}$-Aliphat-$C_{3-12}$-Cycloaliphat, -$C_{1-8}$-Aliphat-Aryl, -$C_{1-8}$-Aliphat-Heteroaryl, -Aryl und -Heteroaryl.

Bevorzugte Beispiele für $R_5$ # -H sind nachfolgend abgebildet:

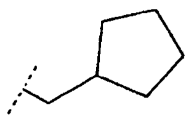
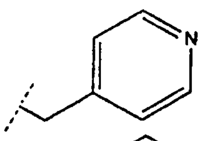
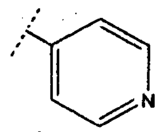
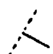
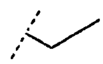
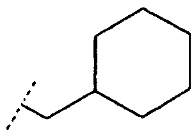
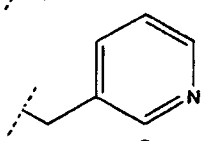
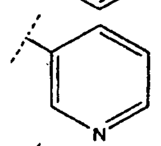
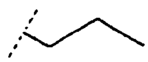
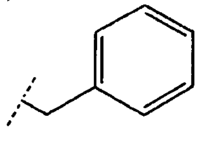
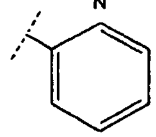

In einer bevorzugten Ausführungsform steht $R_5$ für -H.

Besonders bevorzugte Ausführungsformen der erfindungsgemäßen Verbindungen sind in nachfolgender Tabelle zusammengefasst:

| | bevorzugt | bevorzugter | noch bevorzugter |
|---|---|---|---|
| $R_1$ | -H oder -$C_{1-8}$-Aliphat | -H oder -$C_{1-8}$-Alkyl | -H oder -$CH_3$ |
| $R_2$ | -H oder -$C_{1-8}$-Aliphat | -H oder -$C_{1-8}$-Alkyl | -H oder -$CH_3$ |
| $R_3$ | -$C_{1-8}$-Aliphat; Aryl ggf. substituiert; oder -Heteroaryl ggf. substituiert | -$C_{1-8}$-Alkyl; Phenyl ggf. substituiert; oder -Thienyl ggf. substituiert | -Butyl, -Phenyl, Methoxy-Phenyl, oder Fluor-Phenyl |

(fortgesetzt)

| | bevorzugt | bevorzugter | noch bevorzugter |
|---|---|---|---|
| $R_4$ | -Aryl, -O-Aryl, -S-Aryl, -Heteroaryl, -O-$C_{1-8}$-Aliphat-Aryl, -O-$C_{3-12}$-Cycloaliphat | -Phenyl, -O-Phenyl, -S-Phenyl, jeweils ggf. substituiert; Indolyl ggf. substituiert; Isoindolyl ggf. substituiert; -O-$C_{3-12}$-Cycloalkyl | -Phenyl, -O-Phenyl, -S-Phenyl, jeweils ggf. substituiert; Indolyl ggf. substituiert; Isoindolyl ggf. substituiert; -O-$C_{3-12}$-Cycloalkyl |
| $R_5$ | -H | -H | -H |
| $Y_1$, $Y_1'$, $Y_2$, $Y_2'$, $Y_3$, $Y_3'$, $Y_4$, $Y_4'$ | -H | -H | -H |

Zum Zwecke der Beschreibung werden Kohlenwasserstoffreste unterteilt in aliphatische Kohlenwasserstoffreste einerseits und aromatische Kohlenwasserstoffreste andererseits.

Aliphatische Kohlenwasserstoffreste werden ihrerseits unterteilt in nicht-cyclische aliphatische Kohlenwasserstoffreste einerseits (= "Aliphat") und cyclische aliphatische Kohlenwasserstoffreste, d.h. alicyclische Kohlenwasserstoffreste, andererseits (= "Cycloaliphat"). Cycloaliphaten können monocyclisch oder multicyclisch sein. Alicyclische Kohlenwasserstoffreste ("Cycloaliphat") umfassen sowohl reine aliphatische Carbocyclen als auch aliphatische Heterocyclen, d.h. - sofern nicht ausdrücklich spezifiziert - umfasst "Cycloaliphat" reine aliphatische Carbocyclen (z.B. Cyclohexyl), reine aliphatische Heterocyclen (z.B. Piperidyl oder Piperazyl) sowie nicht-aromatische, multicyclische, ggf. gemischte Systeme (z.B. Decalinyl, Decahydrochinolinyl).

Aromatische Kohlenwasserstoffe werden ihrerseits unterteilt in carbocyclische aromatische Kohlenwasserstoffe einerseits (= "Aryl") und heterocyclische aromatische Kohlenwasserstoffe andererseits (= "Heteroaryl").

Die Zuordnung von multicyclischen, wenigstens teilaromatischen Systemen richtet sich vorzugsweise danach, ob wenigstens ein aromatischer Ring des multicyclischen Systems wenigstens ein Heteroatom (üblicherweise N, O oder S) im Ring aufweist. Ist wenigstens ein solches Heteroatom in diesem Ring vorhanden, handelt es sich bevorzugt um ein "Heteroaryl" (selbst wenn ggf. als zusätzlich vorhandener Cyclus des multicyclischen Systems ein weiterer carbocyclischer aromatischer oder nicht-aromatischer Ring mit oder ohne Heteroatom vorhanden ist); ist in keinem der ggf. mehreren aromatischen Ringe des multicyclischen Systems ein solches Heteroatom vorhanden, handelt es sich bevorzugt um "Aryl" (selbst wenn in einem ggf. zusätzlich vorhandenen, nicht-aromatischen Cyclus des multicyclischen Systems ein Ringheteroatom vorhanden ist).

Innerhalb der multicyclischen Substituenten gilt demnach bevorzugt folgende Priorität bei der Zuordnung: Heteroaryl > Aryl > Cycloaliphat. Der folgende Substituent wird daher vorzugsweise als "Aryl" aufgefasst:

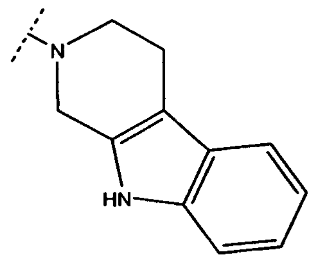

Zum Zwecke der Beschreibung werden einbindige und mehrbindige, z.B. zweibindige Kohlenwasserstoffreste nicht begrifflich unterschieden, d.h. "$C_{1-3}$-Aliphat" umfasst, je nach Sinnzusammenhang, z.B. sowohl -$C_{1-3}$-Alkyl, -$C_{1-3}$-Alkenyl und -$C_{1-3}$-Alkinyl, als auch z.B. -$C_{1-3}$-Alkylen-, -$C_{1-3}$-Alkenylen- und $C_{1-3}$-Alkinylen-.

Bevorzugt ist Aliphat jeweils ein verzweigter oder unverzweigter, gesättigter oder ein ein- oder mehrfach ungesättigter, unsubstituierter oder ein- oder mehrfach substituierter, aliphatischer Kohlenwasserstoffrest. Soweit Aliphat ein- oder mehrfach substituiert ist, sind die Substituenten unabhängig voneinander ausgewählt aus der Gruppe bestehend ausgewählt aus der Gruppe bestehend aus -F, -Cl, -Br, -I, -CN, -$NO_2$, -CHO, =O, -$R_0$, -C(=O)$R_0$, -C(=O)-OH, -C(=O)$OR_0$, -C(=O)$NH_2$, -C(=O)$NHR_0$, -C(=O)$N(R_0)_2$, -OH, -$OR_0$, -OC(=O)H, -OC(=O)$R_0$, -OC(=O)$OR_0$, -OC(=O)-$NHR_0$, -OC(=O)$N(R_0)_2$, -SH, -$SR_0$, -$SO_3H$, -$S(=O)_{1-2}$-$R_0$, -$S(=O)_{1-2}NH_2$, -$NH_2$, -$NHR_0$, -$N(R_0)_2$, -$N^+(R_0)_3$, -$N^+(R_0)_2O^-$, -NHC(=O)$R_0$, -NHC(=O)$OR_0$, -NHC(=O)$NH_2$, -NHC(=O)$NHR_0$, -NH-C(=O)$N(R_0)_2$, -$Si(R_0)_3$, -$PO(OR_0)_2$. So umfasst "Aliphat" acyclische gesättigte oder ungesättigte Kohlenwasserstoffreste, die verzweigt oder geradkettig sein können, d.h.

Alkanyle, Alkenyle und Alkinyle. Dabei weisen Alkenyle mindestens eine C=C-Doppelbindung und Alkinyle mindestens eine C≡C-Dreifachbindung auf. Bevorzugte unsubstituierte einbindige Aliphaten umfassen $-CH_3$, $-CH_2CH_3$, $-CH_2CH_2CH_3$, $-CH(CH_3)_2$, $-CH_2CH_2-CH_2CH_3$, $-CH(CH_3)CH_2CH_3$, $-CH_2CH(CH_3)_2$, $-C(CH_3)_3$, $-CH_2CH_2CH_2-CH_2CH_3$ und $-CH_2CH_2-CH_2CH_2CH_2CH_3$; aber auch $-CH=CH_2$, $-C≡CH$, $-CH_2CH=CH_2$, $-CH=CHCH_3$, $-CH_2C≡CH$, $-C≡CCH_3$ und $-CH=CHCH=CH_2$. Bevorzugte unsubstituierte zweibindige Aliphaten umfassen $-CH_2-$, $-CH_2CH_2-$, $-CH_2CH(CH_3)-$, $-CH(CH_3)-CH_2-$, $-CH_2CH_2CH_2-$, $-CH(CH_3)CH_2CH_2-$,- $CH_2CH(CH_3)-CH_2-$, $-CH_2CH_2CH(CH_3)-$, $-CH-(CH_2CH_3)CH_2-$ und $-CH_2CH_2-CH_2CH_2-$; aber auch $-CH=CH-$, $-C≡C-$, $-CH_2CH=CH-$, $-CH=CHCH_2-$, $-CH_2C≡C-$ und $-C≡CCH_2-$. Bevorzugte substituierte einbindige Aliphaten umfassen $-CH_2F$, $-CHF_2$, $-CF_3$, $-CH_2CF_3$, $-CF_2CF_3$, $-CH_2OH$, $-CH_2CH_2OH$, $-CH_2CHOHCH_3$, $-CH_2OCH_3$ und $CH_2CH_2OCH_3$. Bevorzugte substituierte zweibindige Aliphaten umfassen $-CF_2-$, $-CF_2CF_2-$, $-CH_2CHOH-$, $-CHOHCH_2-$ und $-CH_2CHOHCH_2-$. Besonders bevorzugt sind Methyl, Ethyl, n-Propyl und n-Butyl.

Bevorzugt ist Cycloaliphat jeweils ein gesättigter oder ein ein- oder mehrfach ungesättigter, unsubstituierter oder ein- oder mehrfach substituierter, aliphatischer (d.h. nicht aromatischer), mono- oder multicyclischer Kohlenwasserstoffrest. Die Zahl der Ringkohlenstoffatome liegt vorzugsweise im angegebenen Bereich (d.h. ein "$C_{3-8}$-"Cycloaliphat weist vorzugsweise 3, 4, 5, 6, 7 oder 8 Ringkohlenstoffatome auf). Zum Zwecke der Beschreibung ist "$C_{3-8}$-Cycloaliphat" bevorzugt ein cyclischer Kohlenwasserstoff mit 3, 4, 5, 6, 7 oder 8 Ringkohlenstoffatomen, gesättigt oder ungesättigt, aber nicht aromatisch, wobei ggf. ein oder zwei Kohlenstoffatome unabhängig voneinander durch ein Heteroatom S, N oder O ersetzt sind. Soweit Cycloalkyl ein- oder mehrfach substituiert ist, sind die Substituenten unabhängig voneinander ausgewählt aus der Gruppe bestehend aus -F, -Cl, -Br, -I, -CN, $-NO_2$, -CHO, =O, $-R_0$, $-C(=O)R_0$, -C(=O)OH, $-C(=O)OR_0$, $-C(=O)NH_2$, $-C(=O)NHR_0$, $-C(=O)N(R_0)_2$, -OH, $-OR_0$, -OC(=O)H, $-OC(=O)R_0$, $-OC(=O)OR_0$, $-OC(=O)NHR_0$, $-OC(=O)-N(R_0)_2$, -SH, $-SR_0$, $-SO_3H$, $-S(=O)_{1-2}-R_0$, $-S(=O)_{1-2}NH_2$, $-NH_2$, $-NHR_0$, $-N(R_0)_2$, $-N^+(R_0)_3$, $-N^+(R_0)_2O^-$, $-NHC(=O)R_0$, $-NHC(=O)OR_0$, $-NHC(=O)NH_2$, $-NHC(=O)NHR_0$, $-NH-C(=O)N(R_0)_2$, $-Si(R_0)_3$, $-PO(OR_0)_2$. Vorteilhaft ist $C_{3-8}$-Cycloaliphat aus der Gruppe ausgewählt bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl. Ist Cycloaliphat durch $R_0$ substituiert und $R_0$ steht für Aryl oder Heteroaryl, so kann dieser Aryl- bzw. Heteroaryl-Substituent über eine Bindung an Cycloaliphat gebunden sein, er kann jedoch auch über zwei vicinale Ringatome des Cycloaliphaten gebunden, d.h. annelliert sein.

Bevorzugt wird im Zusammenhang mit "Aliphat" bzw. "Cycloaliphat" unter "ein- oder mehrfach substituiert" die ein- oder mehrfache Substitution, z.B. einfache, zweifache, dreifache oder vierfache Substitution, eines oder mehrerer Wasserstoffatome durch -F, -Cl, -Br, -I, -OH, $-OC_{1-6}$-Alkyl, $-OC(=O)C_{1-6}$-Alkyl, -SH, $-NH_2$, $-NHC_{1-6}$-Alkyl, $-N(C_{1-6}\text{-Alkyl})_2$, $-C(=O)OC_{1-6}$-Alkyl oder -C(=O)OH verstanden. Bevorzugt sind Verbindungen, wobei "Aliphat substituiert" oder "Cycloaliphat substituiert" Aliphat oder Cycloaliphat substituiert mit -F, -Cl, -Br, -I, -CN, $-CH_3$, $-C_2H_5$, $-NH_2$, $-NO_2$, -SH, $-CF_3$, -OH, $-OCH_3$, $-OC_2H_5$ oder $-N(CH_3)_2$ bedeutet. Besonders bevorzugte Substituenten sind -F, -Cl, -OH, -SH, $-NH_2$ und -C(=O)OH.

Unter mehrfach substituierten Resten sind solche Reste zu verstehen, die entweder an verschiedenen oder an gleichen Atomen mehrfach, z. B. zwei- oder dreifach, substituiert sind, beispielsweise dreifach am gleichen C-Atom, wie im Falle von $-CF_3$ oder $-CH_2CF_3$, oder an verschiedenen Stellen, wie im Falle von $-CH(OH)-CH=CH-CHCl_2$. Die Mehrfachsubstitution kann mit dem gleichen oder mit verschiedenen Substituenten erfolgen. Ggf. kann ein Substituent auch seinerseits substituiert sein; so umfasst -OAliphat u.a. auch $-OCH_2CH_2O-CH_2CH_2-OH$. Bevorzugt ist es, wenn Aliphat oder Cycloaliphat substituiert sind mit -F, -Cl, -Br, -I, -CN, $-CH_3$, $-C_2H_5$, $-NH_2$, $-NO_2$, -SH, $-CF_3$, -OH, $-OCH_3$, $-OC_2H_5$ oder $-N(CH_3)_2$. Ganz besonders bevorzugt ist es, wenn Aliphat oder Cycloaliphat substituiert sind mit -OH, $-OCH_3$ oder $-OC_2H_5$.

Bevorzugt steht Aryl jeweils unabhängig für ein carbocyclisches Ringsystem mit mindestens einem aromatischen Ring, aber ohne Heteroatome in diesem Ring, wobei die Aryl-Reste ggf. mit weiteren gesättigten, (partiell) ungesättigten oder aromatischen Ringsystemen kondensiert sein können, welche ihrerseits ein oder mehrere Heteroringatome aufweisen können, jeweils unabhängig voneinander ausgewählt aus N, O und S, und wobei jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können. Bevorzugte Aryle sind Phenyl, Naphthyl, Anthracenyl, Phenanthrenyl, Fluoranthenyl, Fluorenyl, Indanyl und Tetralinyl. Besonders bevorzugt sind Phenyl und Naphthyl. Soweit Aryl ein- oder mehrfach substituiert ist, können die Aryl-Substituenten gleich oder verschieden und in jeder beliebigen und möglichen Position des Aryls sein, und sind unabhängig voneinander ausgewählt aus der Gruppe bestehend aus -F, -Cl, -Br, -I, -CN, $-NO_2$, -CHO, =O, $-R_0$, $-C(=O)R_0$, -C(=O)OH, $-C(=O)OR_0$, $-C(=O)-NH_2$, $-C(=O)NHR_0$, $-C(=O)N(R_0)_2$, -OH, $-O(CH_2)_{1-2}O-$, $-OR_0$, -OC(=O)H, $-OC(=O)R_0$, $-OC(=O)OR_0$, $-OC(=O)-NHR_0$, $-OC(=O)N(R_0)_2$, -SH, $-SR_0$, $-SO_3H$, $-S(=O)_{1-2}-R_0$, $-S(=O)_{1-2}NH_2$, $-NH_2$, $-NHR_0$, $-N(R_0)_2$, $-N^+(R_0)_3$, $-N^+(R_0)_2O^-$, $-NHC(=O)R_0$, $-NHC(=O)OR_0$, $-NHC(=O)NH_2$, $-NHC(=O)NHR_0$, $-NH-C(=O)N(R_0)_2$, $-Si(R_0)_3$, $-PO(OR_0)_2$. Bevorzugte substituierte Aryle sind 2-Fluor-Phenyl, 3-Fluor-Phenyl, 4-Fluor-Phenyl, 2,3-Difluor-Phenyl, 2,4-Difluor-Phenyl, 3,4-Difluor-Phenyl, 2-Chlor-Phenyl, 3-Chlor-Phenyl, 4-Chlor-Phenyl, 2,3-Dichlor-Phenyl, 2,4-Dichlor-Phenyl, 3,4-Dichlor-Phenyl, 2-Methoxy-Phenyl, 3-Methoxy-Phenyl, 4-Methoxy-Phenyl, 2,3-Dimethoxy-Phenyl, 2,4-Dimethoxy-Phenyl, 3,4-Dimethoxy-Phenyl, 2-Methyl-Phenyl, 3-Methyl-Phenyl, 4-Methyl-Phenyl, 2,3-Dimethyl-Phenyl, 2,4-Dimethyl-Phenyl und 3,4-Dimethyl-Phenyl.

Bevorzugt steht Heteroaryl für einen 5-, 6- oder 7-gliedrigen cyclischen aromatischen Rest, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann. Bevorzugt ist "Heteroaryl" ausgewählt aus der Gruppe bestehend aus Pyrrolyl, Indolyl, Furyl (Furanyl), Benzofuranyl, Thienyl (Thiophenyl), Benzothienyl, Benzothiadiazolyl, Benzooxadiazolyl, Benzothiazolyl, Benzooxazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, wobei die Bindung über jedes beliebige und mögliche Ringglied des Heteroaryl-Restes erfolgen kann. Soweit Heteroaryl ein- oder mehrfach substituiert ist, können die Heteroaryl-Substituenten gleich oder verschieden und in jeder beliebigen und möglichen Position des Heteroaryls sein, und sind unabhängig voneinander ausgewählt aus der Gruppe bestehend aus -F, -Cl, -Br, -I, -CN, $-NO_2$, -CHO, =O, $-R_0$, $-C(=O)R_0$, -C(=O)OH, $-C(=O)OR_0$, $-C(=O)-NH_2$, $-C(=O)NHR_0$, $-C(=O)N(R_0)_2$, -OH, $-O(CH_2)_{1-2}O-$, $-OR_0$, -OC(=O)H, $-OC(=O)R_0$, $-OC(=O)OR_0$, $-OC(=O)NHR_0$, $-OC(=O)-N(R_0)_2$, -SH, $-SR_0$, $-SO_3H$, $-S(=O)_{1-2}-R_0$, $-S(=O)_{1-2}NH_2$, $-NH_2$, $-NHR_0$, $-N(R_0)_2$, $-N^+(R_0)_3$, $-N^+(R_0)_2O^-$, $-NH-C(=O)R_0$, $-NHC(=O)OR_0$, $-NHC(=O)NH_2$, $-NHC(=O)NHR_0$, $-NH-C(=O)N(R_0)_2$, $-Si(R_0)_3$, $-PO(OR_0)_2$.

In Bezug auf "Aryl" oder "Heteroaryl" versteht man unter "ein- oder mehrfach substituiert" die ein- oder mehrfache, z.B. zwei-, drei- vier- oder fünffache, Substitution eines oder mehrerer Wasserstoffatome des Ringsystems.

Besonders bevorzugt sind die Substituenten von Aryl und Heteroaryl jeweils unabhängig voneinander ausgewählt aus -F, -Cl, -Br, -I, -CN, -CHO, $-CO_2H$, $-NH_2$, $-NO_2$, $-NHR_0$, $-N(R_0)_2$, $-N^+(R_0)_3$, $-N^+(R_0)_2O^-$, -SH, $-SR_0$, -OH, $-OR_0$, $-C(=O)R_0$, $-CO_2R_0$, $-C(=O)NH_2$, $-C(=O)NHR_0$, $-C(=O)N(R_0)_2$, $-S(=O)_{1-2}R_0$, $-S(=O)_{1-2}NH_2$, $-SO_3H$, =O oder $-R_0$. Bevorzugte Substituenten sind -F, -Cl, -Br, -I, -OH, $-OC_{1-6}$-Alkyl, $-O-C(=O)-C_{1-6}$-Alkyl, -SH, $-NH_2$, $-NHC_{1-6}$-Alkyl, $-N(C_{1-6}$-Alkyl$)_2$, $-C(=O)OC_{1-6}$-Alkyl oder -C(=O)OH. Bevorzugt sind Verbindungen, wobei "Aryl substituiert" oder "Heteroaryl substituiert" Aryl oder Heteroaryl substituiert mit -F, -Cl, -Br, -I, -CN, $-CH_3$, $-C_2H_5$, $-NH_2$, $-NO_2$, -SH, $-CF_3$, -OH, $-OCH_3$, $-OC_2H_5$ oder $-N(CH_3)_2$ bedeutet. Besonders bevorzugte Substituenten sind -F, -Cl, -OH, -SH, $-NH_2$ und -C(=O)OH.

Die erfindungsgemäßen Verbindungen können in Form eines einzelnen Stereoisomers oder deren Mischung, der freien Verbindungen und/oder ihrer physiologisch verträglichen Salze und/oder Solvate vorliegen.

Die erfindungsgemäßen Verbindungen können je nach Substitutionsmuster chiral oder achiral sein.

Bei den erfindungsgemäßen Verbindungen kann es sich je nach Substitution in Bezug auf den Cyclohexanring um Isomere handeln, bei denen das Substitutionsmuster in 1,4-Position (1-Position: $>C(NR_1R)R_3$; 4-Position: $>COHR_5CH_2R_4$) auch mit syn/anti bezeichnet werden kann. "Syn/anti-Isomere" sind eine Untergruppe der Stereoisomere (Konfigurationsisomere).

In einer bevorzugten Ausführungsform beträgt der Diastereomerenüberschuss des syn-Isomers wenigstens 50 %de, bevorzugter wenigstens 75 %de, noch bevorzugter wenigstens 90%de, am bevorzugtesten wenigstens 95%de und insbesondere wenigstens 99%de. In einer anderen bevorzugten Ausführungsform beträgt der Diastereomerenüberschuss des anti-Isomers wenigstens 50 %de, bevorzugter wenigstens 75 %de, noch bevorzugter wenigstens 90%de, am bevorzugtesten wenigstens 95%de und insbesondere wenigstens 99%de.

Geeignete Methoden zur Trennung der Isomere (Diastereomere) sind dem Fachmann bekannt. Als Beispiele können Säulenchromatographie, präparative HPLC und Kristallisationsverfahren genannt werden.

Sind die erfindungsgemäßen Verbindungen chiral, so liegen sie vorzugsweise als Racemat oder in angereicherter Form eines Enantiomers vor. In einer bevorzugten Ausführungsform beträgt der Enantiomerenüberschuss (ee) des S-Enantiomers wenigstens 50%ee, bevorzugter wenigstens 75%ee, noch bevorzugter wenigstens 90%ee, am bevorzugtesten wenigstens 95%ee und insbesondere wenigstens 99%ee. In einer anderen bevorzugten Ausführungsform beträgt der Enantiomerenüberschuss (ee) des R-Enantiomers wenigstens 50%ee, bevorzugter wenigstens 75%ee, noch bevorzugter wenigstens 90%ee, am bevorzugtesten wenigstens 95%ee und insbesondere wenigstens 99%ee.

Geeignete Methoden zur Trennung der Enantiomere sind dem Fachmann bekannt. Als Beispiele können präparative HPLC an chiralen stationären Phasen und Überführung in diastereomere Intermediate genannt werden. Die Überführung in diastereomere Intermediate kann beispielsweise als Salzbildung mit Hilfe chiraler, enantiomerenreiner Säuren erfolgen. Nach der Trennung der so gebildeten Diastereomere kann das Salz dann wieder in die freie Base oder ein anderes Salz überführt werden.

Sofern nicht ausdrücklich spezifiziert umfasst jeder Verweis auf die erfindungsgemäßen Verbindungen alle Isomere (z.B. Stereoisomere, Diastereomere, Enantiomere) in beliebigem Mischungsverhältnis.

Sofern nicht ausdrücklich spezifiziert umfasst jeder Verweis auf die erfindungsgemäßen Verbindungen die freien Verbindungen (d.h. die Formen, welche nicht als Salz vorliegen) und alle physiologisch verträglichen Salze.

Zum Zwecke der Beschreibung liegen physiologisch verträgliche Salze der erfindungsgemäßen Verbindungen vor als Salze mit Anionen oder Säuren der jeweiligen Verbindung mit anorganischen bzw. organischen Säuren, die

physiologisch - insbesondere bei Anwendung im Menschen und/oder Säugetier - verträglich sind.

Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, α-Liponsäure, Acetylglycin, Acetylsalicylsäure, Hippursäure und/oder Asparaginsäure. Besonders bevorzugt sind das Hydrochlorid, das Citrat und das Hemicitrat.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

Nachfolgend werden jeweils bevorzugte Ausführungsformen der erfindungsgemäßen Verbindungen erläutert. Sofern nicht ausdrücklich spezifiziert gelten alle jeweils zuvor erläuterten Definitionen der Substituenten und deren jeweilige bevorzugte Ausführungsformen entsprechend und werden daher nicht wiederholt.

Bevorzugte Ausführungsformen der erfindungsgemäßen Verbindungen der allgemeinen Formel (1) haben die allgemeine Formel (2), (3), (4), (5), (6), (7), (8) oder (9):

(2)

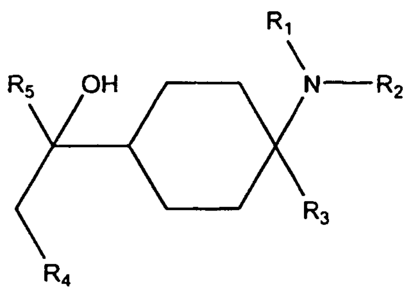

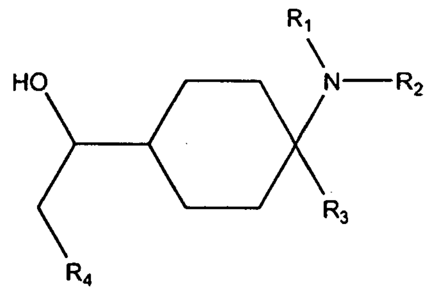

(3)

(4)

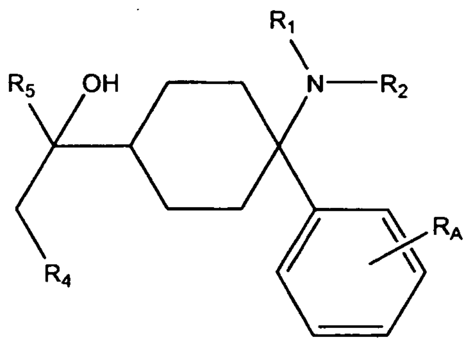

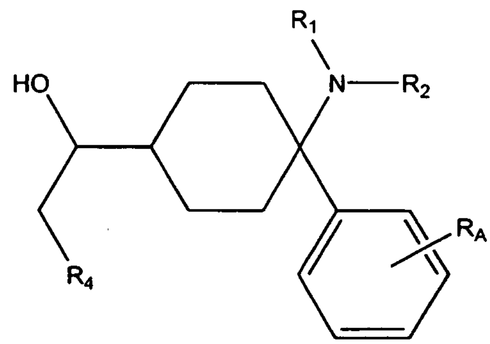

(5)

(6)

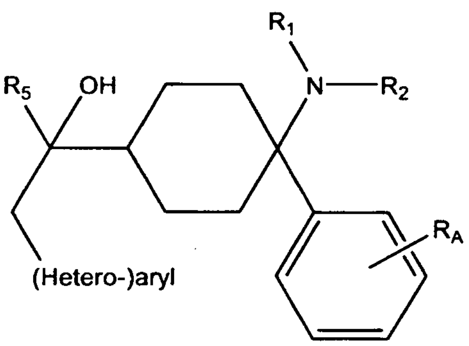

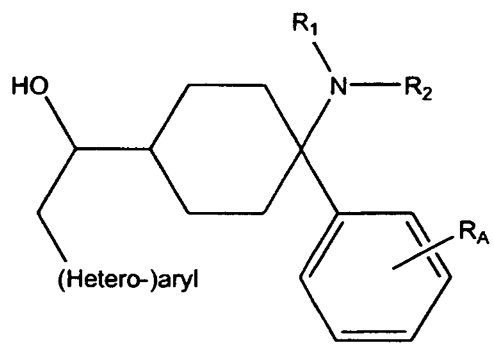

(7)

(8) 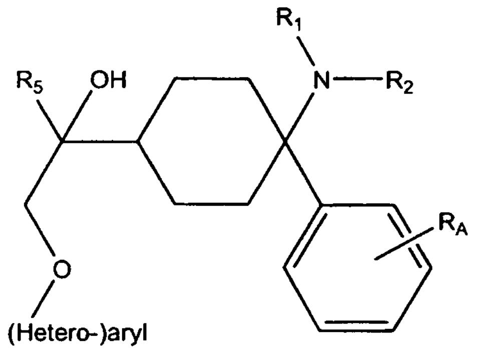 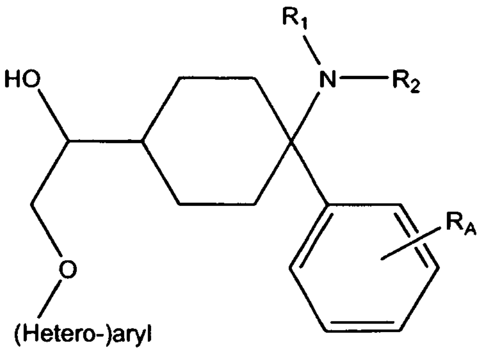 (9)

worin, soweit vorhanden,
$R_A$ für -H, -F, -Cl, -CN, $-NO_2$ oder $-OCH_3$ steht; und
(Hetero-)aryl für Heteroaryl oder Aryl steht, jeweils unsubstituiert oder ein- oder mehrfach substituiert.

Die erfindungsgemäßen Verbindungen sind durch Substituenten definiert, beispielsweise durch $R_1$, $R_2$ und $R_3$ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise $Y_1$ = $-R_0$ wobei $R_0$ = $-C_{1-8}$-Aliphat (Substituent der 1. Generation), so kann $-C_{18}$-Aliphat seinerseits substituiert sein, z.B. mit $-OR_0$ wobei $R_0$ = -Aryl (Substituent der 2. Generation). Es ergibt sich daraus die funktionelle Gruppe $-C_{1-8}$-Aliphat-OAryl. -Aryl kann dann seinerseits erneut substituiert sein, z.B. mit -Cl (Substituent der 3. Generation). Es ergibt sich daraus dann insgesamt die funktionelle Gruppe $-C_{1-8}$-Aliphat-OAryl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform die funktionellen Gruppen für $R_0$ bis $R_5$ jeweils ggf. substituiert sein, die jeweiligen Substituenten können dann ihrerseits jedoch nicht erneut substituiert sein.

In einer anderen bevorzugten Ausführungsform können bereits die Substituenten der 1. Generation nicht erneut substituiert sein, d.h. es gibt dann weder Substituenten der 2. noch Substituenten der 3. Generation. Mit anderen Worten können in dieser Ausführungsform die funktionellen Gruppen für $R_0$ bis $R_5$ jeweils nicht substituiert sein.

Ganz besonders bevorzugt sind Verbindungen aus der Gruppe:

- 1-(4-(Dimethylamino)-4-phenylcyclohexyl)-2-phenylethanol;
- 1-(4-(Dimethylamino)-4-phenylcyclohexyl)-2-phenoxyethanol;
- 1-(4-(Dimethylamino)-4-phenylcyclohexyl)-2-(1H-indol-1-yl)ethanol;
- 1-(4-(Dimethylamino)-4-phenylcyclohexyl)-2-(isoindolin-2-yl)ethanol,
- 1-(4-(Dimethylamino)-4-phenylcyclohexyl)-2-(4-fluorphenyl)ethanol;
- 1-(4-(Dimethylamino)-4-(3-fluorphenyl)cyclohexyl)-2-phenylethanol;
- 1-(4-(Dimethylamino)-4-(3-methoxyphenyl)cyclohexyl)-2-phenylethanol;
- 1-(4-(Dimethylamino)-4-(thiophen-2-yl)cyclohexyl)-2-phenylethanol;
- 1-(4-Butyl-4-(dimethylamino)cyclohexyl)-2-phenylethanol;
- 1-Cyclopentyl-2-(4-(dimethylamino)-4-phenylcyclohexyl)-3-phenylpropan-2-ol;
- 1-(4-(dimethylamino)-4-phenylcyclohexyl)-1-phenyl-2-(pyridin-4-yl)ethanol;
- 1-(4-(Dimethylamino)-4-phenylcyclohexyl)-2-(phenylthio)ethanol;
- 1-(4-(Dimethylamino)-4-phenylcyclohexyl)-2-(phenylsulfonyl)ethanol;
- 2-(Cyclohexyloxy)-1-(4-(dimethylamino)-4-phenylcyclohexyl)ethanol;
- 2-(Benzyloxy)-1-(4-(dimethylamino)-4-phenylcyclohexyl)ethanol;
- 1-(4-(Dimethylamino)-4-phenylcyclohexyl)-2-phenethoxyethanol;
- 2-((1*H*-Indol-3-yl)methoxy)-1-(4-(dimethylamino)-4-phenylcyclohexyl)ethanol;
- 2-(2-(1*H*-Indol-3-yl)ethoxy)-1-(4-(dimethylamino)-4-phenylcyclohexyl)ethanol;
- 1-(4-(Dimethylamino)-4-phenylcyclohexyl)-2-((2-(triethylsilyl)-1*H*-indol-3-yl)methoxy)-ethanol;
- 2-(4,4a-Dihydro-1*H*-pyrido[3,4-b]indol-2(3*H*,9*H*,9a*H*)-yl)-1-(4-(dimethylamino)-4-(3-fluorphenyl)cyclohexyl)ethanol;
- 2-(4-(Dimethylamino)-4-phenylcyclohexyl)-1-phenylpropan-2-ol;
- 2-(4(Dimethylamino)-4-phenylcyclohexyl)-1,3-diphenylpropan-2-ol;
- 2-(4-(Dimethylamino)-4-phenylcyclohexyl)-1-phenyl-3-(pyridin-2-yl)propan-2-ol;

- 2-(4-(Dimethylamino)-4-phenylcyclohexyl)-1-phenyl-3-(pyridin-3-yl)propan-2-ol; und
- 2-(4-(Dimethylamino)-4-phenylcyclohexyl)-1-phenyl-3-(pyridin-4-yl)propan-2-ol;

und deren physiologisch verträgliche Salze und/oder Solvate.

Weitere bevorzugte Verbindungen sind

- 2-(9-(Benzolsulfonyl)-2,3,4,9-tetrahydro-1H-beta-carbolin-2-yl)-1-(4-dimethylamino-4-phenylcyclohexyl)ethanol;
- 2-(2,3-Dihydro-1H-isoindol-2-yl)-1-(4-(dimethylamino)-4-phenylcyclohexyl)ethanol;
- 2-Cyclohexyloxy-1-(4-dimethylamino-4-thiophen-2-ylcyclohexyl)ethanol;
- 2-(4-Dimethylamino-4-phenylcyclohexyl)-1-phenoxy-propan-2-ol; und

und deren physiologisch verträgliche Salze.

Die erfindungsgemäßen Verbindungen wirken beispielsweise auf den im Zusammenhang mit verschiedenen Erkrankungen relevanten ORL1-Rezeptor, sodass sie sich als pharmazeutischer Wirkstoff in einem Arzneimittel eignen.

Ein weiterer Gegenstand der Erfindung betrifft daher Arzneimittel, welche wenigstens eine erfindungsgemäße Verbindung enthalten, sowie ggf. geeignete Zusatz- und/oder Hilfsstoffe und/oder ggf. weitere Wirkstoffe.

Die erfindungsgemäßen Verbindungen weisen eine vergleichbare Affinität zum $\mu$-Opioid- bzw. zum ORL1-Rezeptor auf wie die Verbindungen, die als Beispielverbindungen in WO 2004043967 offenbart sind. Im Vergleich zu diesen Verbindungen weisen sie jedoch eine höhere Löslichkeit auf und eignen sich daher besonders für die Arzneimittelentwicklung.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfinungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens einer erfindungsgemäßen Verbindung appliziert.

Für alle vorstehenden Formen der erfindungsgemäßen Arzneimittel ist es besonders bevorzugt, wenn das Arzneimittel neben wenigstens einer erfindungsgemäßen Verbindung noch einen weiteren Wirkstoff, insbesondere ein Opioid, vorzugsweise ein starkes Opioid, insbesondere Morphin, oder ein Anästhetikum, vorzugsweise Hexobarbital oder Halothan, enthält.

In einer bevorzugten Form des Arzneimittels liegt eine enthaltene erfindungsgemäße Verbindung als reines Diastereomer und/oder Enantiomer vor.

Der ORL1-Rezeptor wurde insbesondere im Schmerzgeschehen identifiziert. Entsprechend können erfindungsgemäße Verbindungen zur Herstellung eines Arzneimittels zur Behandlung von Schmerz, insbesondere von akutem, neuropathischem oder chronischem Schmerz, verwendet werden.

Ein weiterer Gegenstand der Erfindung betrifft daher die Verwendung einer erfindungsgemäßen Verbindung zur Herstellung eines Arzneimittels zur Behandlung von Schmerz, insbesondere von akutem, viszeralem, neuropathischem oder chronischem Schmerz.

Ein weiterer Gegenstand der Erfindung betrifft die Verwendung einer erfindungsgemäßen Verbindung zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem

Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn eine verwendete Verbindung als reines Diastereomer und/oder Enantiomer, als Razemat oder als nichtäquimolare oder äquimolare Mischung der Diastereomere und/oder Enantiomere vorliegt.

Ein weiterer Gegenstand der Erfindung betrifft ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis einer erfindungsgemäßen Verbindung, oder eines erfindungsgemäßen Arzneimittels.

Ein weiterer Gegenstand der Erfindung betrifft ein Verfahren zur Herstellung der erfindungsgemäßen Verbindungen wie in der folgenden Beschreibung und Beispielen ausgeführt. Insbesondere geeignet ist dabei ein Verfahren zur Herstellung einer erfindungsgemäßen Verbindung, wobei Verbindungen der allgemeinen Formel I durch Addition von geeigneten Nucleophilen an geeignete Carbonylverbindungen erhalten werden können. Im Falle, dass $R^5$ ungleich -H ist, können entweder $R^5$ oder aber $R^4$-$CH_2$- in verschiedener Abfolge eingeführt werden (Schema 1):

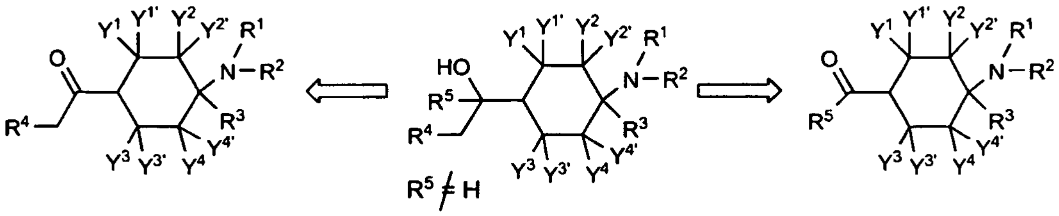


II Ia III

**Schema 1**

Die Ketone II bzw. III können dabei in einer zwischengeschalteten Synthese durch Addition von geeigneten Kohlenstoff-Nucleophilen an Aldehyde VI eingeführt werden. Anschließend kann der erhaltene Alkohol V mittels dem Fachmann geläufigen Oxidationsmethoden in die Ketone II bzw. III überführt werden. Alternativ dazu können Carbonylverbindungen des Typs Weinreb-Amid (IV) durch Substitution mit Kohlenstoff-Nucleophilen in Ketone übergeführt werden (Schema 2):

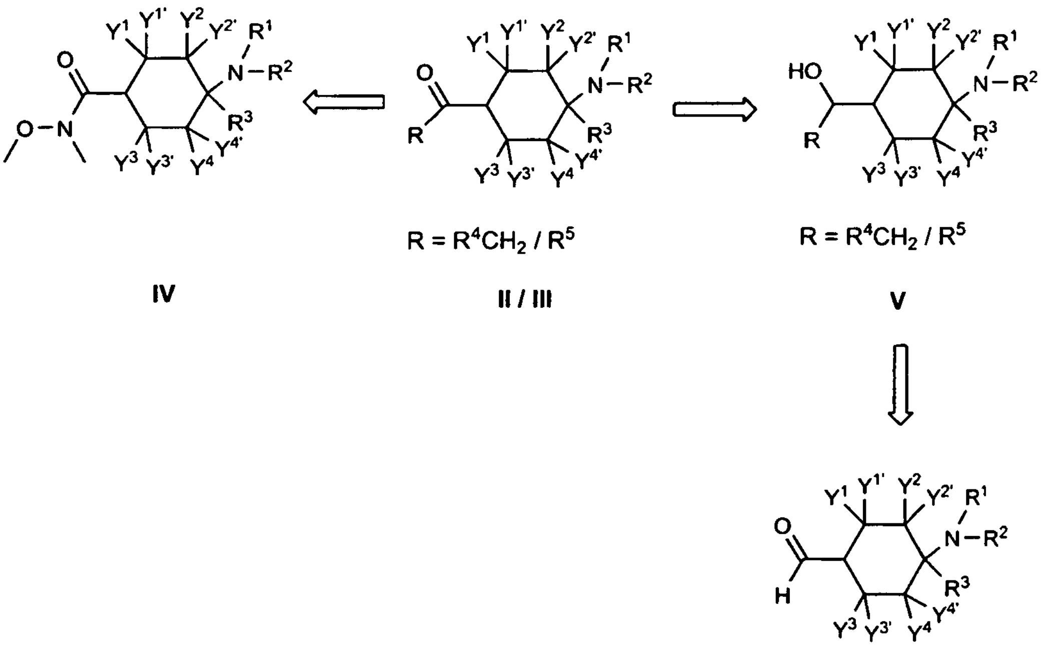

R = $R^4CH_2$ / $R^5$ R = $R^4CH_2$ / $R^5$

IV II / III V

**Schema 2**

VI

Die Herstellung von Weinreb-Amiden ist dem Fachmann bekannt.

Im Falle, dass $R^5$ identisch mit $R^4$-$CH_2$- ist, können Verbindungen der allgemeinen Formel I auch durch Addition von mindestens 2 Äquivalenten eines geeigneten Kohlenstoff-Nucleophils an entsprechende Carbonsäureester oder andere geeignete Carbonylverbindungen erhalten werden.

Im Falle, dass $R^5$ gleich -H ist, entfallen die Zwischenstufen V und VI.

Ein alternatives Verfahren zur Herstellung von Verbindungen der allgemeinen Formel I besteht in der ringöffnenden Substitution mittels geeigneter Nucleophile, $R^4$ enthaltend, an terminalen Epoxiden VII (Schema 3). Dabei kann vorteilhaft von acetalgeschützten Vorstufen VIII, bevorzugt von Ketalen, ausgegangen werden. Das durch ringöffnende Substitution resultierende Ketal VIII wird zu IX entschützt. In einem weiteren Schritt wird eine geeignete Schutzgruppe an der Alkoholfunktion eingeführt, z.B. ein anderes Acetal, resultierend in Verbindungen der allgemeinen Formel X. Die zuvor erhaltenen Ketofunktionen werden nach dem Fachmann bekannten Verfahren in Aminonitrile XI überführt, die anschließend nach geläufigen Methoden mit Kohlenstoff-Nucleophilen in Amine des Typs XII überführt werden. Anschließend werden durch Entfernung der acetalischen Schutzgruppe am Alkohol die Verbindungen der allgemeinen Formel I gewonnen.

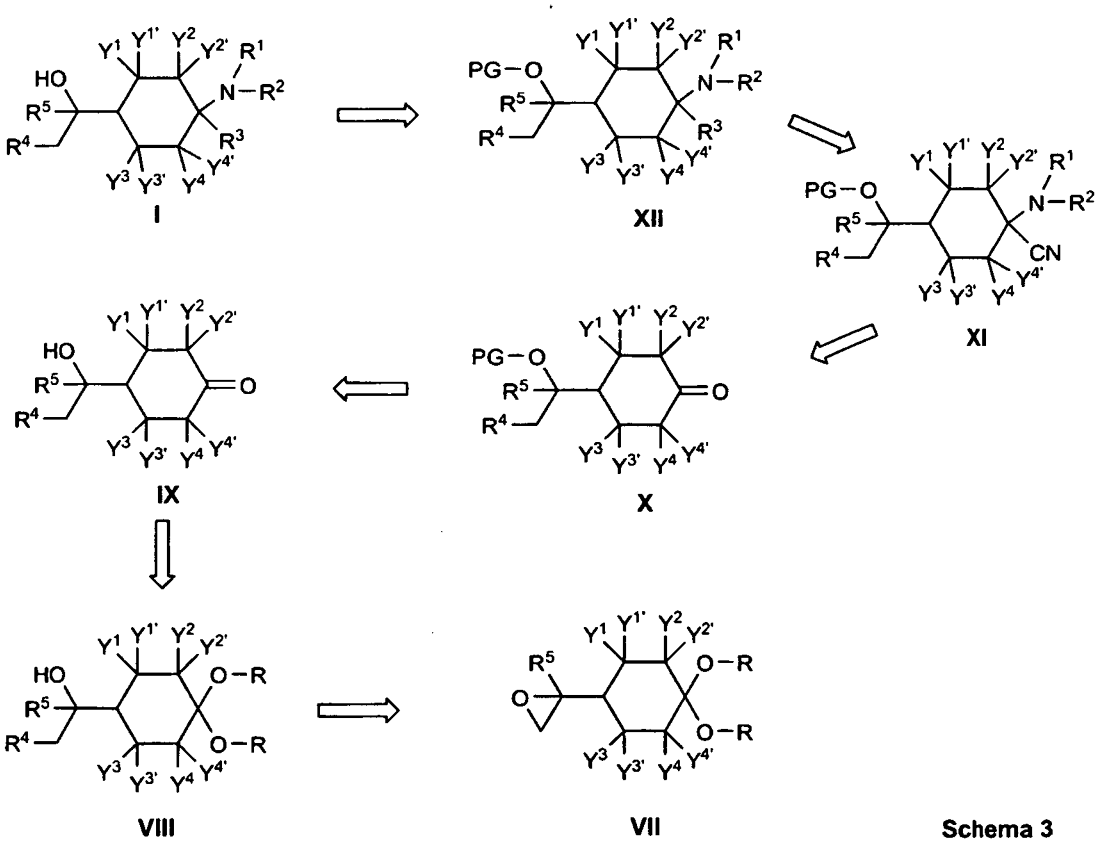
I, XII, XI, IX, X, VIII, VII

**Schema 3**

Besonders vorteilhaft ist dieses Verfahren für den Fall, dass $R^4$ über ein Heteroatom gebunden ist, d.h. $R^4$ mit einem Heteroatom beginnt, ausgewählt aus N, S und O.

Terminale Epoxide des Typs VII können nach dem Fachmann bekannten Verfahren hergestellt werden, z.B. durch Addition von Methyliden an geeignete Carbonylverbindungen III oder z.B. durch Epoxidierung von entsprechenden Olefinen XIV (Schema 4):

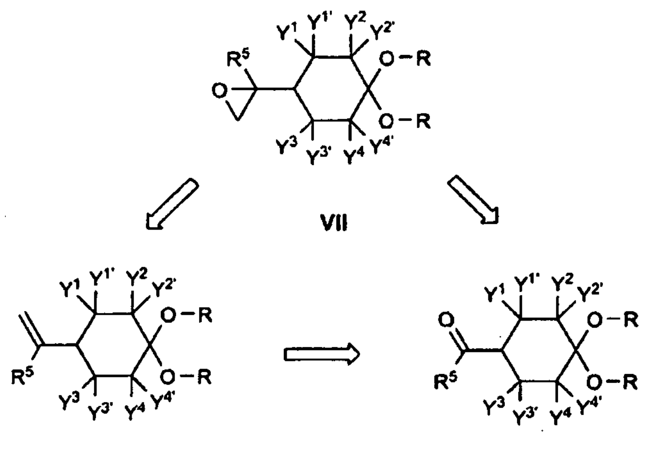

VII

XIV

III

**Schema 4**

Bezüglich weiterer Einzelheiten zur Synthese der erfindungsgemäßen Verbindungen kann vollumfänglich verweisen werden auf WO2002/090317, WO2002/90330, WO2003/008370, WO2003/008371, WO2003/080557, WO2004/043899, WO2004/043900, WO2004/043902, WO2004/043909, WO2004/043949, WO2004/043967, WO2005/063769, WO2005/066183, WO2005/110970, WO2005/110971, WO2005/110973, WO2005/110974, WO2005/110975, WO2005/110976, WO2005/110977, WO2006/018184, WO2006/108565, WO2007/079927, WO2007/079928, W02007/079930, WO2007/079931, WO2007/124903, WO2008/009415 und WO2008/009416.

Beispiele

Die folgenden Beispiele dienen zur näheren Erläuterung der Erfindung, sind jedoch nicht einschränkend auszulegen.

Die Ausbeuten der hergestellten Verbindungen sind nicht optimiert. Alle Temperaturen sind unkorrigiert. Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei),"rac." racemisch, "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt. Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Laufmitteln für chromatographische Untersuchungen sind stets in Volumen/Volumen angegeben.

$^{1}$H-NMR: Varian Mercury 400BB, 400 MHz oder Varian Mercury 300 BB, 300 MHz; $^{13}$C-NMR: Varian Mercury 400BB, 100 MHz oder Varian Mercury 300 BB, 75 MHz; interner Standard: TMS, chemische Verschiebungen in ppm; br: breites Signal $^{19}$F-NMR: Varian Mercury 400BB, 376.8 MHz
interner Standard: $CFCl_3$

LC-MS: Agilent LC-MS 1200 Rapid Resolution mit MSD6140 Gradient: Zeit 0 min: 95 % Wasser (+ 1 % Ameisensäure) / 5 % Methanol (+ 1 % Ameisensäure)→Zeit 5.4 min: 0 % Wasser / 100 % Methanol (+ 1 % Ameisensäure) Säulentemperatur: 50 °C; Injektionsvolumen: 5 μL; Flussrate 0.8 mL/min; Fragmentorspannung: 100 V [pos/neg]; Detektion: MM-ES + APCI + DAD (254 nm); Säule: SB-C18, 2.1 mmx30 mm, 3.5 Mikron.

| Methoden-Nr. | Dauer [min] | Flussrate [mUmin] |
|---|---|---|
| 1 | 7 | 0,8 |
| 7 | 7,5 | 0,8 |
| 8 | 7 | 0,8 |

Fortsetzung Methodentabelle LC/MS

| Methoden-Nr. | Gradient | Säulen-Temp. [°C] | Wellenlänge [nm] | UV-Scan | Massenbereich | pos / neg | Fragmentierung M |
|---|---|---|---|---|---|---|---|
| 1 | 5-100 | 30 | 254 | * | 100-800 | */* | 50 |
| 7 | 5-100 | 50 | 254 | * | 100-600 | */* | 100 |
| 8 | 5-100 | 80 | 254 | * | 80-800 | */* | 100 |

Beispiel 1:

1-(4-Dimethylamino-4-(3-fluor-phenyl)cyclohexyl)-2-phenyl-ethanol

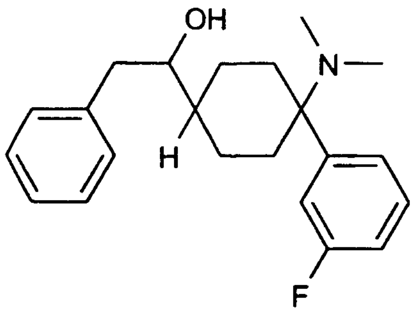

Stufe 1:

4-Dimethylamino-4-(3-fluorphenyl)cyclohexancarbaldehyd

Eine Suspension von Methoxymethyltriphenylphosphoniumchlorid (2.18 g, 6.36 mmol) in wasserfreiem Tetrahydrofuran (5 mL) und wasserfreiem N,N-Dimethylformamid (5 mL) wurde unter Argon mit einer 60 % Dispersion von Natriumhydrid in Mineralöl (254 mg, 6.36 mmol) versetzt. Das Gemisch wurde 2 h bei Raumtemperatur gerührt. Anschließend wurde innerhalb von 30 min eine Lösung von 4-Dimethylamino-4-(3-fluorphenyl)cyclohexanon (1.0 g, 4.24 mmol) in wasserfreiem Tetrahydrofuran (5 mL) und wasserfreiem N,N-Dimethylformamid (5 mL) zugetropft und über Nacht bei Raumtemperatur gerührt. Anschließend wurde unter Eiskühlung 2 M Salzsäure (25 mL) zugetropft und 5 h bei Raumtemperatur gerührt. Danach wurde das Gemisch mit Ethylacetat (5 × 20 ml) und Diethylether (3 × 20 mL) extrahiert. Die wässrige Phase wurde mit 4 M Natronlauge auf pH 11 gestellt und mit Ethylacetat (4 × 20 mL) extrahiert. Die vereinigten organischen Extrakte aus der alkalischen Lösung wurden mit Natriumsulfat getrocknet und i. Vak. eingeengt.
Ausbeute: 1.49 g (>100 %), braunes Öl
1 H-NMR(DMSO-d6): Es handelt sich um ein Diastereoisomerengemisch. Alle charakteristischen Signale konnten identifiziert werden.

Stufe 2:

1-(4-Dimethylamino-4-(3-fluor-phenyl)cyclohexyl)-2-phenyl-ethanol

Eine Lösung von 4-Dimethylamino-4-(3-fluorphenyl)cyclohexancarbaldehyd (1.49 g, 5.97 mmol) in wasserfreiem Tetrahydrofuran (30 mL) wurde unter Eiskühlung tropfenweise mit einer 2 M Lösung von Benzylmagnesiumchlorid in Tetrahydrofuran (6.00 mL, 12 mmol) versetzt. Das Gemisch wurde 2 d bei Raumtemperatur gerührt, dann unter Eiskühlung mit gesättigter Ammoniumchlorid-Lösung (30 mL) versetzt. Das Tetrahydrofuran wurde i. Vak. entfernt und der Rückstand mit 4 M Natronlauge auf pH 8 gestellt. Die wässrige Suspension wurde mit Diethylether (3 × 30 mL) extrahiert. Die vereinigten organischen Phasen wurden mit Natriumsulfat getrocknet und i. Vak. eingeengt. Das Rohprodukt (2.18 g) wurde durch Flashchromatographie mit Dichlormethan / Methanol [95:5 + 1 % NH3 (32 % in H2O)] gereinigt. Das verunreinigte polare Diastereoisomer (500 mg) wurde erneut durch Flashchromatographie mit Dichlormethan / Methanol [95:5 + 1 % NH3 (32 % in H2O)] gereinigt.
Ausbeute: 125 mg (6 %), weißer Feststoff

Schmelzpunkt: 170°C
1H-NMR(DMSO-d6): 0.56-1.10 (m, 2H); 1.20-1.60 (m, 4H); 1.74 (br d, 1H, J = 13.0 Hz); 1.91 (s, 6H); 2.46 (m, 1 H, vom DMSO-Signal überlagert); 2.56-2.70 (m, 3H); 3.27 (m, 1 H); 4.23 (d, 1H, J = 6.0 Hz); 7.02-7.25 (m, 8H); 7.41 (dd, 1 H, J = 7.9 und 14.5 Hz).
13C-NMR (DMSO-d6): 23.2; 25.8; 32.4; 32.7; 37.9; 40.7; 42.8; 61.0; 74.5; 112.8 (d, J = 21 Hz); 114.6 (d, J = 21 Hz); 123.9; 125.3; 127.8; 129.2; 140.2; 162.2 (d, J = 242 Hz).

Beispiel 2:

1-(4-Butyl-4-dimethylaminocyclohexyl)-2-phenylethanol

Durch Ersatz von 4-Dimethylamino-4-(3-fluorphenyl)cyclohexanon durch 4-Butyl-4-dimethylaminocyclohexanon in Beispiel 1, Stufe 1 und anschließende analoge Umsetzung in Stufe 2 wurde Beispiel 2 erhalten:

$^{1}$H-NMR ($CDCl_3$): 0.90 (3H, t, J = 7.1 Hz); 1.14-1.49 (9H, m); 1.56-1.82 (6H, m); 2.28 (6H, s); 2.63 (1H, dd, J = 8.9, 13.6 Hz); 2.88 (1H, dd, J = 4.7, 13.6 Hz); 3.66 (1H, m); 7.16-7.22 (3H, m); 7.27-7.29 (2H, m). Das OH-Proton konnte nicht identifiziert werden.
$^{13}$C-NMR ($CDCl_3$): 14.0; 21.8; 23.5; 23.7 (2C); 26.7 (2C); 31.1; 31.7; 31.8; 37.0 (2C); 41.0; 42.3; 76.4; 126.0; 128.3 (2C); 129.3 (2C); 139.3.
LC-MS (Methode 8): $[M+H]^+$: m/z = 304.3, $R_t$ = 2.4 min.

Beispiel 3 und Beispiel 4

(1-(4-Dimethylamino-4-phenylcyclohexyl)-2-phenylethanol Hydrochlorid, unpolareres Diastereomer)

(1-(4-Dimethylamino-4-phenylcyclohexyl)-2-phenylethanol, polareres Diastereomer)

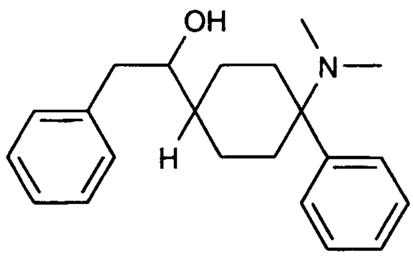

Durch Ersatz von 4-Dimethylamino-4-(3-fluorphenyl)cyclohexancarbaldehyd durch 4-Dimethylamino-4-phenylcyclohexancarbaldehyd in Beispiel 1, Stufe 2 wurden analog die Beispiele 3 und 4 erhalten:

1-((4-Dimethylamino-4-phenylcyclohexyl)-2-phenylethanol (unpolares Diastereoisomer) Ausbeute: 170 mg (16 %), gelbliches Öl
1 H-NMR (CDCl3): 1.35-1.90 (m, 6H); 2.05 (s, 6H); 2.58-2.72 (m, 3H); 2.94-3.06 (m, 1 H); 3.42-3.50 (m, 1 H); 3.72 (br s, 1 H); 4.20-4.23 (m, 1 H); 7.02-7.95 (m, 10H).

1-((4-Dimethylamino-4-phenylcyclohexyl)-2-phenylethanol (polares Diastereoisomer) Ausbeute: 142 mg (14 %), weißer Feststoff
Schmelzpunkt: 161-166 °C
1H-NMR (CDCl3): 0.99-1.16 (m, 2H); 1.46-1.78 (m, 5H); 1.89-1.98 (m, 1H); 2.07 (s, 6H); 2.49 (dd, 1H, J = 9.5 und 13.5 Hz); 2.76 (br d, 1H, J = 12.5 Hz); 2.80 (dd, 1H, J = 13.7, 3.2 Hz); 3.40 (ddd, 1 H, J = 9.6, 6.4, 3.3 Hz); 7.11-7.43 (m, 10H).

Stufe 2

(1-(4-Dimethylamino-4-phenylcyclohexyl)-2-phenylethanol-Hydrochlorid, unpolareres Diastereomer)

1-(4-Dimethylamino-4-phenylcyclohexyl)-2-phenylethanol (unpolares Diastereoisomer, 140 mg, 0.41 mmol) wurde mit einer 7.5 M Lösung von Chlorwasserstoff in Diethylether (25 mL) versetzt. Die überstehende Lösung wurde dekantiert, der Niederschlag wurde i. Vak. im Exsikkator über Kaliumhydroxid getrocknet.
Ausbeute: 80 mg (51 %), weißer Feststoff
Schmelzpunkt: 235 °C
1 H-NMR (DMSO-d6): 1.29-1.44 (m, 3H); 1.76-1.90 (m, 1H); 1.92-2.04 (m, 1H); 2.24-2.38 (m, 2H); 2.43 (t, 6H, J = 5.3 Hz); 2.45-2.58 (m, 3H, teilweise von DMSO-Signal überlagert); 2.90 (dd, 1H, J = 3.1 und 13.7 Hz); 3.80-3.88 (m, 1 H); 4.44 (s, 1 H); 7.13-7.19 (m, 1 H); 7.22-7.29 (m, 4H); 7.47-7.55 (m, 3H); 7.66-7.71 (m, 2H); 10.48 (s, 1 H).

Beispiel 5

1-(4-Dimethylamino-4-phenylcyclohexyl)-2-(4-fluorphenyl)ethanol

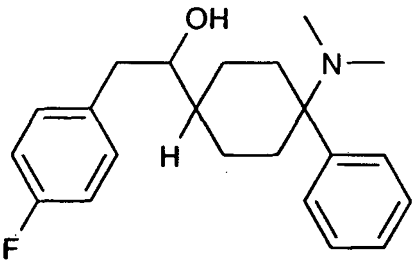

Durch Ersatz von Benzylmagnesiumchlorid durch 4-Fluorbenzylmagnesiumchlorid in Beispiel 3 und 4, Stufe 1 wurde analog das Beispiel 5 erhalten:

Schmelzpunkt: 75 °C
1 H-NMR(CDCI3): 1.32-1.56 (m, 3H); 1.58-1.84 (m, 6H); 2.05 (s, 6H); 2.66 (m, 2H); 2.95 (d, 1 H, J = 13.8 Hz); 3.68 (m, 1 H); 6.96-7.05 (m, 2H); 7.18-7.40 (m, 7H).
13C-NMR (DMSO-d6): 23.1; 24.1; 32.8; 32.9; 37.8; 40.3; 42.5; 58.9; 76.4; 115.2 (d, J = 21 Hz); 126.4; 126.6; 127.2; 130.7 (d, J = 8 Hz); 134.9; 139.6; 161.6 (d, J = 244 Hz).
LC-MS (Methode 8): [M+H]+: m/z = 342.3, Rt = 2.4 min.

Beispiel 6 und Beispiel 7

1-(4-Dimethylamino-4-thiophen-2-yl-cyclohexyl)-2-phenylethanol (polares Diastereomer) und 1-(4-Dimethylamino-4-thiophen-2-yl-cyclohexyl)-2-phenylethanol (unpolares Diastereomer)

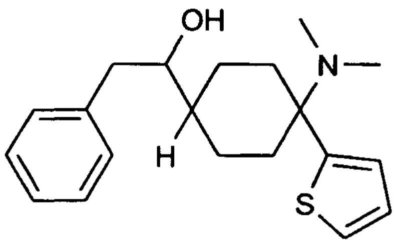

Stufe 1:

1-(1,4-Dioxaspiro[4.5]dec-8-yl)-2-phenylethanol

Eine Lösung von 1,4-Dioxaspiro[4,5]decan-8-carbaldehyd (4.42 g, 25.9 mmol) in wasserfreiem Tetrahydrofuran

(30 mL) wurde unter Eiskühlung mit einer 2 M Lösung von Benzylmagnesiumchlorid (26 mL, 52 mmol) tropfenweise versetzt. Das Gemisch wurde über Nacht bei Raumtemperatur gerührt. Anschließend wurde das Reaktionsgemisch unter Eiskühlung mit gesättigter Ammoniumchlorid-Lösung (10 mL) und Wasser (10 mL) versetzt. Das Lösungsmittel wurde i. Vak. eingeengt. Der Rückstand wurde mit Diethylether (3 × 10 mL) extrahiert. Die vereinigten organischen Phasen wurden mit Natriumsulfat getrocknet und i. Vak. eingeengt.
Ausbeute: 2.23 g (32 %), weißer Feststoff
Schmelzpunkt: 86 °C
1 H-NMR (DMSO-d6): 1.15-1.85 (m, 9H); 2.54 (dd, 1H, J = 13.6, 8.4 Hz); 2.72 (dd, 1 H, J = 13.6, 4.2 Hz); 3.45 (m, 1 H); 3.83 (s, 4H); 4.38 (d, 1 H; J = 6.0 Hz); 7.10-7.30 (m, 5H).

In analogen Ansätzen mit 3 bis 48,11 mmol unter Einsatz von 2 bis 4 Moläquivalenten Benzylmagnesiumchlorid wurden Ausbeuten von 26 bis 47 % erzielt.

Stufe 2:

4-(1-Hydroxy-2-phenylethyl)cyclohexanon

Zu einer Lösung von 1-(1,4-Dioxaspiro[4.5]dec-8-yl)-2-phenylethanol (2.98 g, 11.3 mmol) in Tetrahydrofuran (30 mL) wurde 2 M Salzsäure (30 mL) gegeben. Die Lösung wurde über Nacht bei 50 °C gerührt. Das Gemisch wurde mit 4 M Natronlauge alkalisch gestellt, die Phasen wurden getrennt und die wässrige Phase mit Dichlormethan (3 × 20 mL) extrahiert. Die vereinigten organischen Phasen wurden mit Natriumsulfat getrocknet und i. Vak. eingeengt.
Ausbeute: 2.52 g (100 %), farbloses Öl
1 H-NMR (DMSO-d6): 1.40-1.80 (m, 3H); 1.90 (m, 1 H); 2.08 (m, 1 H); 2.14-2.42 (m, 4H); 2.61 (dd, 1H, J = 8.2 und 13.6 Hz); 2.76 (dd, 1H, J = 4.6 und 13.6 Hz); 3.59 (m, 1 H); 4.57 (d, 1 H, J = 5.9 Hz); 7.13-7.32 (m, 5H).

Stufe 3:

4-[1-(1-Ethoxy-ethoxy)-2-phenylethyl]cyclohexanon

Eine Lösung von 4-(1-Hydroxy-2-phenylethyl)cyclohexanon (2.52 g, 11.5 mmol) in wasserfreiem Dichlormethan (50 mL) wurde mit Ethylvinylether (998 mg, 1.32 mL, 13.8 mmol) und Pyridiniumtosylat (44 mg, 0.17 mmol) versetzt und über Nacht bei Raumtemperatur gerührt. Das Gemisch wurde mit Dichlormethan (20 mL) versetzt und mit Wasser, 5 % Natriumhydrogencarbonat-Lösung und Natriumchloridlösung (je 50 mL) gewaschen. Die vereinigten organischen Phasen wurden mit Natriumsulfat getrocknet und i. Vak. eingeengt.
Ausbeute: 2.93 g, orangefarbenes Öl
1 H-NMR (DMSO-d6): Das Spektrum enthält alle erwarteten Signale.
Es handelt sich um ein Gemisch von zwei Diastereoisomeren.

Stufe 4:

1-Dimethylamino-4-[1-(1-ethoxy-ethoxy)-2-phenylethyl]cyclohexancarbonitril

Ein Gemisch aus 4 M Salzsäure (2.61 mL) und Methanol (1.56 mL) wurde unter Eiskühlung mit 40 % wässriger Dimethylaminlösung (6.05 mL 47.9 mmol) versetzt. Zu diesem Gemisch wurde eine Lösung von 4-[1-(1-Ethoxy-ethoxy)-2-phenylethyl]cyclohexanon (2.91 g, 10.0 mmol) in Methanol (6 mL) und Tetrahydrofuran (3 mL) gegeben. Dem Gemisch wurde danach Kaliumcyanid (1.56 g, 24.1 mmol) zugesetzt, über Nacht bei Raumtemperatur gerührt, anschließend mit Wasser (150 mL) versetzt und mit Diethylether (4 × 50 mL) extrahiert. Die vereinigten organischen Phasen wurden mit Natriumsulfat getrocknet und i. Vak. eingeengt. Der Rückstand wurde in Dichlormethan (50 mL) aufgenommen und mit Wasser (30 mL) gewaschen. Die organische Phase wurde erneut mit Natriumsulfat getrocknet und i. Vak. eingeengt.
Ausbeute: 3.18 g, orangefarbenes Öl
1 H-NMR (DMSO-d6): Das Spektrum enthält alle erwarteten Signale. Es handelt sich um ein Gemisch von Diastereoisomeren.

Stufe 5:

{4-[1-(1-Ethoxy-ethoxy)-2-phenylethyl]-1-thiophen-2-yl-cyclohexyl}dimethylamin

Zu einer Lösung von 1-Dimethylamino-4-[1-(1-ethoxy-ethoxy)-2-phenylethyl]cyclohexan-carbonitril (1.06 g, 3.1 mmol) in Tetrahydrofuran (15 mL) wurde unter Eiskühlung und Argonatmosphäre eine 1 M Lösung von 2-Thienylmagnesiumbromid in Tetrahydrofuran (9.25 mL, 9.25 mmol) getropft. Das Gemisch wurde 48 h bei Raumtemperatur gerührt und anschließend mit Wasser und gesättigter Ammoniumchloridlösung (je 10 mL) versetzt. Die Phasen wurden getrennt und die wässrige Phase mit Diethylether (3 × 10 mL) extrahiert. Die vereinigten organischen Phasen wurden mit Natriumsulfat getrocknet und i. Vak. eingeengt. Ausbeute: 1.12 g, gelbliches Öl
1 H-NMR (DMSO-d6): Das Spektrum enthält alle erwarteten Signale. Es handelt sich um ein Gemisch von Diastereoisomeren

Stufe 6:

1-(4-Dimethylamino-4-thiophen-2-yl-cyclohexyl)-2-phenylethanol

Zu einer Lösung von {4-[1-(1-Ethoxy-ethoxy)-2-phenylethyl]-1-thiophen-2-yl-cyclohexyl}dimethylamin (1.10 g, 2.73 mmol) in Tetrahydrofuran (20 mL) wurde 2 M Salzsäure (20 mL) gegeben und das Reaktionsgemisch über Nacht bei Raumtemperatur gerührt. Anschließend wurde mit 4 M Natronlauge alkalisch gestellt und mit Dichlormethan (4× 10 mL) extrahiert. Die vereinigten organischen Phasen wurden mit Natriumsulfat getrocknet und i. Vak. eingeengt. Das Rohprodukt (956 mg) wurde durch Flashchromatographie mit Cyclohexan / Ethylacetat (3:2) und dann Methanol gereinigt. Die beiden so gewonnenen Produktfraktionen wurden jeweils in Diethylether aufgenommen und mit 2 M Salzsäure (20 mL) versetzt. Die Phasen wurden getrennt. Die sauren wässrigen Phasen wurde mit Diethylether (3 × 10 mL) extrahiert und danach mit 4 M Natronlauge alkalisch gestellt. Die wässrigen Phasen wurden mit Dichlormethan (4 × 10 mL) extrahiert. Die vereinigten organischen Phasen wurden mit Natriumsulfat getrocknet und i. Vak. eingeengt.

polares Diastereoisomer

Ausbeute: 466 mg (46 % bezogen auf die eingesetzte Stufe 2), beigefarbener Feststoff Schmelzpunkt: 85 °C
1 H-NMR (DMSO-d6): 1.22-1.75 (m, 7H); 2.01 (s, 6H); 2.40-2.46 (m, 2H); 2.55 (dd, 1 H, J = 13.6 und 8.6 Hz); 2.75 (dd, 1 H, J = 13.6 und 4.0 Hz); 3.45 (m, 1 H); 4.35 (d, 1H, J = 6.1 Hz); 6.90 (d, 1 H, J = 3.5 Hz); 7.03 (dd, 1 H, J = 3.5 und 5.1 Hz); 7.14-7.30 (m, 5H); 7.37 (d, 1 H, J = 5.1 Hz).
13C-NMR (DMSO-d6): 22.1; 23.9; 35.1; 35.3; 37.5; 40.6; 42.8; 58.1; 75.1; 122.8; 123.5; 125.3; 126.0; 127.7; 129.3; 140.4; 145.2.
LC-MS (Methode 7): [M+H]+: m/z = 330.3, Rt = 2.8 min.

unpolares Diastereoisomer

Ausbeute: 16 mg (1 % bezogen auf die eingesetzte Stufe 2), gelbes Öl 1 H-NMR (CDCl3): 1.43-1.82 (m, 8H); 2.12 (s, 6H); 2.51 (d, 2H, J = 13.8 Hz); 2.63 (dd, 1H, J = 9.5 und 13.6 Hz); 2.97 (dd, 1H, J = 3.4 und 13.6 Hz); 3.70 (m, 1 H); 6.87 (dd, 1 H, J = 1.1 und 3.4 Hz); 7.03 (dd, 1H, J = 3.6 und 5.1 Hz); 7.20-7.27 (m, 4H); 7.30-7.36 (m, 2H). LC-MS (Methode 1): [M+H]+: m/z = 330.3, Rt = 3.3 min.

<u>Beispiel 8</u>

1-(4-Butyl-4-dimethylaminocyclohexyl)-2-phenylethanol

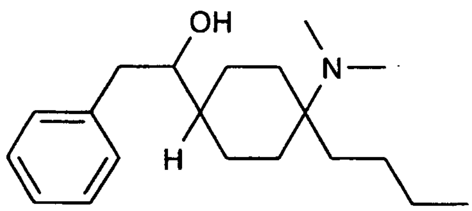

Bei analoger Durchführung zu Beispiel 6 und 7 unter Verwendung der Stufe 4 und mit Ersatz von Thienylmagnesiumbromid durch Butylmagnesiumbromid in Stufe 5 wurde das Beispiel 8 erhalten.

Stufe 5:

{1-Butyl-4-[1-(1-ethoxy-ethoxy)-2-phenylethyl]cyclohexyl}dimethylamin Zu einer Lösung von 1-Dimethylamino-4-[1-(1-ethoxy-ethoxy)-2-phenylethyl]cyclohexan-carbonitril (1.06 g, 3.1 mmol) in Tetrahydrofuran (15 mL) wurde unter Eiskühlung und Argonatmosphäre eine 2 M Lösung von n-Butylmagnesiumchlorid in Tetrahydrofuran (4.62 mL, 9.25 mmol) getropft. Das Gemisch wurde 48 h bei Raumtemperatur gerührt und danach mit Wasser und gesättigter Ammoniumchloridlösung (je 10 mL) versetzt. Die Phasen wurden getrennt und die wässrige mit Diethylether (3 × 10 mL) extrahiert. Die vereinigten organischen Phasen wurden mit Natriumsulfat getrocknet und i. Vak. eingeengt.
Ausbeute: 1.12 g, gelbliches Öl
1 H-NMR (DMSO-d6): Das Spektrum enthält alle erwarteten Signale.
Es handelt sich um ein Gemisch von Diastereoisomeren.

Stufe 6:

1-(4-Butyl-4-dimethylaminocyclohexyl)-2-phenylethanol

Eine Lösung von {1-Butyl-4-[1-(1-ethoxy-ethoxy)-2-phenylethyl]cyclohexyl}dimethylamin (1.09 g, 2.90 mmol) in wasserfreiem Tetrahydrofuran (20 mL) wurde mit 2 M Salzsäure (20 mL) versetzt und 3 d bei Raumtemperatur gerührt. Das Tetrahydrofuran wurde i. Vak. entfernt, die saure wässrige Lösung mit Diethylether (3 × 10 mL) extrahiert und danach mit 4 M Natronlauge alkalisch gestellt. Die alkalische wässrige Phase wurde mit Dichlormethan (4 x 10 mL) extrahiert. Die vereinigten organischen Phasen wurden mit Natriumsulfat getrocknet und i. Vak. eingeengt. Das Rohprodukt (646 mg) wurde durch Flashchromatographie mit Dichlormethan / Methanol (9:1→8:2-0:1) gereinigt. Das so gewonne Produkt lag als Hydrochlorid vor. Es wurde in Dichlormethan aufgenommen und die Suspension mit gesättigter Kaliumcarbonatlösung gewaschen. Die organische Phase wurde mit Natriumsulfat getrocknet und i. Vak. eingeengt.
Ausbeute: 306 mg (35 % bezogen auf die eingesetztestufe 2), gelbliches Öl
1H-NMR (DMSO-d6): 0.86 (t, 3H, J = 7.0 Hz); 1.45-1.58 (m, 13H); 1.66-1.75 (m, 2H); 2.13 (s, 6H); 2.52 (dd, 1H, J = 8.5 und 13.3 Hz, vom DMSO-Signal teilweise überlagert); 2.72 (dd, 1 H, J = 4.1 und 13.6 Hz); 3.38 (m, 1 H); 4.26 (d, 1H, J = 6.0 Hz); 7.10-7.30 (m, 5H). 13C-NMR (DMSO-d6): 13.9; 21.6; 23.4; 23.5; 26.4; 30.8; 32.0; 32.1; 36.8; 40.6; 43.0; 55.5; 75.3; 125.4; 127.8; 129.1; 140.3.
LC-MS (Methode 7): [M+H]+: m/z = 304.5, Rt = 2.9 min.

<u>Beispiel 9</u>

1-[4-Dimethylamino-4-(3-methoxyphenyl)cyclohexyl]-2-phenylethanol

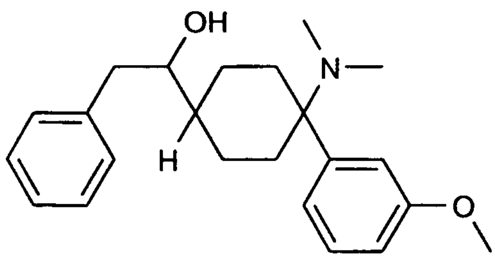

Bei analoger Durchführung zu Beispiel 6 und 7 unter Verwendung der Stufe 4 und mit Ersatz von Thienylmagnesiumbromid durch 3-Methoxyphenylmagnesiumbromid in Stufe 5 wurde das Beispiel 9 erhalten.

Stufe 5:

[4-[1-(1-Ethoxy-ethoxy)-2-phenylethyl]-1-(3-methoxyphenyl)cyclohexyl]dimethylamin

Zu einer Lösung von 1-Dimethylamino-4-[1-(1-ethoxy-ethoxy)-2-phenylethyl]cyclohexancarbonitril (1.06 g, 3.1 mmol) in Tetrahydrofuran (15 mL) wurde unter Eiskühlung und Argonatmosphäre eine 1 M Lösung von 3-Methoxyphenylmagnesiumbromid in Tetrahydrofuran (9.25 mL, 9.25 mmol) getropft. Das Gemisch wurde 48 h bei Raumtemperatur gerührt und danach mit Wasser und gesättigter Ammoniumchloridlösung (je 10 mL) versetzt. Die Phasen wurden getrennt und die wässrige mit Diethylether (3 × 10 mL) extrahiert. Die vereinigten organischen Phasen wurden mit Natriumsulfat getrocknet und i. Vak. eingeengt.
Ausbeute: 1.56 g, gelbliches Öl

1 H-NMR (DMSO-d6): Das Spektrum enthält alle erwarteten Signale.
Es handelt sich um ein Gemisch von Diastereoisomeren.

Stufe 6:

1-[4-Dimethylamino-4-(3-methoxyphenyl)cyclohexyl]-2-phenylethanol

Eine Lösung von WW561 (1.54 g, 3.61 mmol) in wasserfreiem Tetrahydrofuran (20 mL) wurde mit 2 M Salzsäure (20 mL) versetzt und 2 d bei Raumtemperatur gerührt. Das Tetrahydrofuran wurde i. Vak. entfernt, die saure wässrige Lösung mit Diethylether (3 × 10 mL) extrahiert und danach mit 4 M Natronlauge alkalisch gestellt. Die alkalische wässrige Phase wurde mit Dichlormethan (4 × 10 mL) extrahiert. Die vereinigten organischen Phasen wurden mit Natriumsulfat getrocknet und i. Vak. eingeengt. Das Rohprodukt (670 mg) wurde durch Flashchromatographie mit Cyclohexan / Ethylacetat (3:2)→Dichlormethan / Methanol (9:1) gereinigt.
Ausbeute: 163 mg (15 % bezogen auf Stufe 3), weißer Feststoff
Schmelzpunkt: 75 °C 1 H-NMR (DMSO-d6): 1.15-1.52 (m, 7H); 1.95 (s, 6H); 2.52-2.68 (m, 3H); 2.77 (dd, 1 H, J = 3.7 und 13.6 Hz); 3.45 (m, 1 H); 3.74 (s, 3H); 4.33 (d, 1 H, J = 6.1 Hz); 6.78-6.84 (m, 2H); 6.89 (d, 1 H, J = 7.9 Hz); 7.11-7.30 (m, 6H).
13C-NMR (DMSO-d6): 22.2; 24.2; 32.8; 32.9; 37.6; 40.5; 42.9; 58.2; 75.0; 110.9; 112.9; 118.8; 125.3; 127.7; 127.9; 129.3; 140.5; 141.5; 158.6.
Eine weitere Fraktion mit verunreinigtem Produkt (350 mg) wurde erneut durch Flashchromatographie mit Dichlormethan / Methanol (95:5) gereinigt, wodurch weitere 90 mg 1-[4-Dimethytamino-4-(3-methoxyphenyl)cyclohexyl]-2-phenylethanol erhalten wurden (8 % bezogen auf Stufe 3), weißer Feststoff
Schmelzpunkt: 72 °C

Beispiel 10

1-[4-Dimethylamino-4-(3-methoxyphenyl)cyclohexyl]-2-phenylethanol

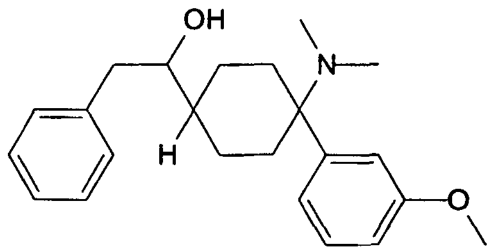

Stufe 1:

4-Dimethylamino-4-(3-methoxyphenyl)cyclohexancarbonitril

Eine Lösung von 4-(Dimethylamino)-4-(3-methoxyphenyl)cyclohexanon (2.47 g, 10 mmol) und Tosylmethylisocyanid (2.54 g, 13 mmol) in wasserfreiem 1,2-Dimethoxyethan (40 mL) und wasserfreiem Ethanol (2 mL) wurde auf -30 °C gekühlt. Anschließend wurde eine Lösung von Kalium-tert-butylat (2.70 g, 24 mmol) in wasserfreiem Tetrahydrofuran (20 mL) so zugetropft, dass die Innentemperatur nicht über 5 °C stieg. Das Gemisch wurde 1 h bei 0 °C, 24 h bei Raumtemperatur und anschließend 5 h unter Rückfluss gerührt. Das Reaktionsgemisch wurde auf Raumtemperatur gekühlt und filtriert. Der Filterrückstand wurde mit 1,2-Dimethoxyethan gewaschen. Das Filtrat wurde i. Vak. eingeengt, der Rückstand in Diethylether aufgenommen und die Lösung mit Wasser (3 × 20 mL) sowie gesättigter Natriumchlorid-Lösung (20 mL) gewaschen. Die organische Phase wurde mit Natriumsulfat getrocknet und i. Vak. eingeengt. Das Rohprodukt (1.76 g) wurde durch Flashchromatographie mit Ethylacetat und dann Ethylacetat / Methanol (9:1→8:2) gereinigt.

unpolares Diastereoisomer

Ausbeute: 401 mg (15 %), gelbliches Öl
1 H-NMR (DMSO-d6): 1.60-1.90 (m, 6H); 1.94 (s, 6H); 2.22-2.34 (m, 2H); 2.81 (m, 1 H); 3.74 (s, 3H); 6.78-6.90 (m, 3H);

7.27 (t, 1H, J = 7.9 Hz).
13C-NMR (DMSO-d6): 24.7; 26.3; 30.5; 37.4; 54.9; 58.7; 111.3; 113.2; 119.3; 122.9; 128.4; 139.2; 158.8.
LC-MS (Methode 8): [M+H]+: m/z = 259.3, Rt = 0.9 min.

polares Diastereoisomer

Ausbeute: 505 mg (19 %), gelbliches Öl
1 H-NMR (DMSO-d6): 1.33-1.52 (m, 2H); 1.92 (s, 6H); 1.93-2.18 (m, 6H); 2.92 (m, 1 H); 3.76 (s, 3H); 6.80-6.94 (m, 3H); 7.30 (t, 1H, J = 7.9 Hz).
13C-NMR (DMSO-d6): 24.9; 26.4; 30.3; 37.7; 54.8; 59.2; 111.4; 113.6; 119.4; 122.8; 128.4; 138.7; 158.8.
LC-MS (Methode 8): [M+H]+: m/z = 259.2, Rt = 1.0 min.

Stufe 2:

1-[4-Dimethylamino-4-(3-methoxyphenyl)cyclohexyl]-2-phenylethanon

Eine Lösung des-polaren Diastereoisomers (240 mg, 0.9 mmol) in wasserfreiem Tetrahydrofuran (5 mL) wurde unter Eiskühlung mit einer 2 M Lösung von Benzylmagnesiumchlorid in Tetrahydrofuran (1.4 mL, 2.8 mmol) versetzt und 3 d bei Raumtemperatur gerührt. Das Reaktionsgemisch wurde mit gesättigter Ammoniumchlorid-Lösung (8 mL) und Wasser (5 mL) versetzt. Das Lösungsmittel wurde i. Vak. entfernt und die wässrige Suspension mit Diethylether (3 $\times$ 20 mL) extrahiert. Die vereinigten organischen Phasen wurden mit Natriumsulfat getrocknet und i. Vak. eingeengt. Das Rohprodukt wurde durch Flashchromatographie mit Dichlormethan / Methanol (95:5) gereinigt.
Ausbeute: 120 mg (36 %), gelbliches Öl
1 H-NMR (CDCl3): 1.33-1.46 (m, 2H); 1.72 (t, 2H, J = 14.5 Hz); 1.87 (d, 2H, J = 11.1 Hz); 2.13 (s, 6H); 2.56 (m, 1 H); 2.70 (d, 2H, J = 12.5 Hz); 3.69 (s, 2H); 3.85 (s, 3H); 6.82-6.94 (m, 4Hz); 7.16 (d, 1 H, J = 7.1 Hz); 7.22-7.38 (m, 4H).

Stufe 3:

1-[4-Dimethylamino-4-(3-methoxyphenyl)cyclohexyl]-2-phenylethanol

Eine Lösung des Produktes aus Stufe 2 (112 mg, 0.3 mmol) in wasserfreiem Methanol (5 mL) wurde bei 0 °C mit Natriumborhydrid (24 mg, 0.6 mmol) versetzt und 3 h bei Raumtemperatur gerührt. Anschließend wurde weiteres Natriumborhydrid (12 mg, 0.3 mmol) zugesetzt und noch 2 h bei Raumtemperatur gerührt. Das Gemisch wurde mit Wasser (20 mL) versetzt. Das Lösungsmittel wurde i. Vak. entfernt und die wässrige Suspension mit Ethylacetat (3 $\times$ 10 mL) extrahiert. Die vereinigten organischen Phasen wurden mit Natriumsulfat getrocknet und i. Vak. eingeengt. Das Rohprodukt (76 mg) wurde durch Flashchromatographie (10 g, 20 $\times$ 1.1 cm) mit Dichlormethan / Methanol [(95:5) +1 % Ammoniaklösung (30 % in H2O)] gereinigt.
Ausbeute: 57 mg (50 %), gelblicher Feststoff
Schmelzpunkt: 120 °C
1 H-NMR (DMSO-d6): 0,84-1.12 (m, 2H); 1.18-1.58 (m, 6H); 1.72 (d, 1 H, J = 12.5 Hz); 1.91 (s, 6H); 2.40-2.50 (m, 1H); 2.54-2.70 (m, 2H); 3.26 (m, 1 H); 3.75 (s, 3H); 4.20 (d, 1H, J = 6.0 Hz); 6.79-6.92 (m, 3H); 7.10-7.33 (m, 6H).
13C-NMR (DMSO-d6): 23.5; 25.9; 32.5; 32.7; 37.9; 40.7; 42.9; 61.1; 74.6; 110.7; 114.4; 120.3; 125.3; 127.8; 128.4; 129.1; 138.4; 140.1; 158.9.

<u>Beispiel 11:</u>

1-(4-(Dimethylamino)-4-(3-fluorphenyl)cyclohexyl)-2-(5-(phenylsulfonyl)-3,4-dihydro-1H-pyrido[4,3-b]indol-2(5H)-yl)ethanol

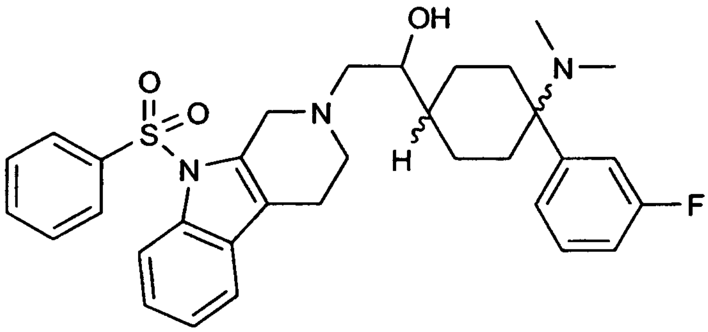

Stufe 1:

1,3,4,9-Tetrahydro-β-carbolin-2-carbonsäurebenzylester

Eine Suspension von 2,3,4,9-Tetrahydro-1H-β-carbolin (4.43 g, 25.7 mmol) und 4-N,N-Dimethylaminopyridin (266 mg) in wasserfreiem Tetrahydrofuran (30 mL) wurde unter Eiskühlung mit einer Lösung von N-(Benzyloxycarbonyloxy)succinimid (9.61 g, 38.6 mmol) in wasserfreiem Tetrahydrofuran (30 mL) versetzt. Die Suspension wurde 16 h bei Raumtemperatur gerührt und anschließend das Tetrahydrofuran i. Vak. entfernt. Der Rückstand wurde in Ethylacetat (20 mL) gelöst und mit Wasser (2 × 20 mL) gewaschen. Die organische Phase wurde mit Natriumsulfat getrocknet und i. Vak. eingeengt. Das Rohprodukt (11.0 g) wurde durch Flashchromatographie mit Cyclohexan / Ethylacetat (4:1) gereinigt. Ausbeute: 6.64 g (84 %), weißer Feststoff
1 H-NMR (DMSO-d6): 2.71 (t, 2H, J = 5.6 Hz); 3.76 (t, 2H, J = 5.3 Hz); 4.64 (br s, 2H); 5.14 (s, 2H); 6.96 (ddd, 1 H, J = 8.0, 7.1 und 1.1 Hz); 7.04 (ddd, 1 H, J = 8.2, 7.1 und 1.2 Hz);
7.25-7.44 (m, 7H); 10.78 und 10.84 (2 s, 1 H).

Stufe 2:

9-Benzolsulfonyl-1,3,4,9-tetrahydro-β-carbolin-2-carbonsäurebenzylester

Eine Lösung des Produkts aus Stufe 1 (6.61 g, 21.6 mmol) in wasserfreiem Dichlormethan (100 mL) wurde mit pulverisiertem Natriumhydroxid (1.73 g, 43.3 mmol) und Tetra-n-butylammoniumhydrogensulfat (111 mg) versetzt und 1 h bei Raumtemperatur gerührt. Der Suspension wurde unter Eiskühlung Benzolsulfonylchlorid (4.21 g, 3.07 mL, 23.8 mmol) zugesetzt. Das Gemisch wurde 16 h bei Raumtemperatur gerührt und anschließend mit Wasser und Dichlormethan (je 50 mL) versetzt. Die organische Phase wurde abgetrennt und mit Natriumchlorid-Lösung (40 mL) gewaschen. Die organische Phase wurde mit Natriumsulfat getrocknet und i. Vak. eingeengt. Der Rückstand wurde durch Flashchromatographie mit Cyclohexan / Ethylacetat (4:2) gereinigt.
Ausbeute: 5.66 g (58 %), weißer Feststoff
Schmelzpunkt: 153 °C 1 H-NMR (DMSO-d6): 2.67 (t, 2H, J = 5.5 Hz); 3.73 (t, 2H, J = 4.7 Hz); 4.97 (br s, 2H); 5.16 (s, 2H); 7.26 (dt, 1 H, J = 7.4 und 1.0 Hz); 7.31-7.94 (m, 12H); 8.01 (br d, J = 8.1 Hz).

Stufe 3:

9-Benzolsulfonyl-2,3,4,9-tetrahydro-1H-β-carbolin

Eine Suspension des Produkts aus Stufe 2 (4.14 g, 9.27 mmol) in Eisessig (20.7 mL) wurde mit 33 % Bromwasserstoff in Eisessig (20.7 mL) versetzt und 1 h bei Raumtemperatur gerührt. Das Gemisch wurde in Diethylether (500 mL) gegossen. Das ausgefallene Hydrobromid wurde abgesaugt, mit Diethylether gewaschen und im Exsikkator über Kaliumhydroxid getrocknet. Das Salz (3.60 g) wurde mit gesättigter Kaliumcarbonat-Lösung (100 mL) versetzt und das resultierende Gemisch mit Dichlormethan (3 × 25 mL) extrahiert. Die vereinigten organischen Phasen wurden mit Natriumsulfat getrocknet und i. Vak. eingeengt.
Ausbeute: 2.51 g (86 %), weißer Feststoff
Schmelzpunkt: 190-195 °C
1H-NMR (DMSO-d6): 2.53 (t, 2H, J = 5.5 Hz); 2.91 (t, 2H, J = 5.5 Hz); 4.11 (s, 2H); 7.20-7.33 (m, 2H); 7.40-7.43 (m, 1H); 7.52-7.60 (m, 2H); 7.62-7.70 (m, 1H); 7.84-7.90 (m, 2H); 7.96-8.04 (m, 1 H).

Stufe 4:

2-(9-Benzolsulfonyl-1,3,4,9-tetrahydro-β-carbolin-2-yl)-1-(1,4-dioxaspiro[4.5]dec-8-yl)ethanol

Eine Lösung des Produkts aus Stufe 3 (2.51 g, 8.05 mmol) und 8-Oxiranyl-1,4-dioxa-spiro[4.5]decan (1.34 g, 7.30 mmol) in wasserfreiem Tetrahydrofuran (50 mL) wurde mit Calciumtrifluormethansulfonat (1.22 g, 3.60 mmol) versetzt und 48 h bei Raumtemperatur gerührt. Das Tetrahydrofuran wurde i. Vak. entfernt. Der Rückstand wurde in Dichlormethan (40 mL) aufgenommen und mit 25 % Kaliumcarbonat-Lösung (2 × 25 mL) gewaschen. Die organische Phase wurde mit Natriumsulfat getrocknet und i. Vak. eingeengt. Das Rohprodukt (4.31 g) wurde durch Flashchromatographie (mit Cyclohexan / Ethylacetat (1:2) gereinigt.
Ausbeute: 2.33 g (64 %), weißer Feststoff
Schmelzpunkt: 158 °C
1 H-NMR (DMSO-d6): 1.22-1.48 (m, 5H); 1.57 (s, 1 H); 1.62-1.78 (m, 3H); 2.48-2.68 (m, 4H); 2.72-2.90 (m, 2H); 3.51 (m, 1 H); 3.83 (s, 4H); 3.95 (d, 1H, J = 17.0 Hz); 4.03 (d, 1 H, J = 17.0 Hz); 4.38 (d, 1H, J = 4.4 Hz); 7.25 (dt, 1 H, J = 7.3 und 1.2 Hz); 7.31 (ddd, 1 H, J = 8.4, 7.3 und 1.5 Hz); 7.40-7.45 (m, 1 H); 7.51-7.60 (m, 2H); 7.64-7.71 (m, 1 H); 7.82-7.89 (m, 2H); 7.99-8.03 (m, 1H).

Stufe 5:

4-[2-(9-Benzolsulfonyl-1,3,4,9-tetrahydro-β-carbolin-2-yl)-1-hydroxyethyl]cyclohexanon

Eine Lösung des Produkts aus Stufe 4 (748 mg, 1.50 mmol) in Tetrahydrofuran (30 ml) wurde mit 2 M Salzsäure (30 mL) versetzt und 16 h bei Raumtemperatur gerührt. Das Gemisch wurde mit 4 M Natronlauge alkalisch gestellt und mit Ethylacetat (3 x 35 mL) extrahiert. Die vereinigten organischen Phasen wurden mit Natriumsulfat getrocknet und i. Vak. eingeengt.
Ausbeute: 658 mg (96 %), beigefarbener Feststoff
Schmelzpunkt: 174 °C
1 H-NMR (DMSO-d6): 1.40-1.64 (m, 2H); 1.76-1.98 (m, 2H); 2.02 (m, 1 H); 2.15-2.26 (m, 2H); 2.27-2.45 (m, 2H); 2.54-2.70 (m, 4H); 2.77-2.93 (m, 2H); 3.64 (m, 1H); 3.98 (d, 1H, J = 17.0 Hz); 4.06 (d, 1 H, J = 17.0 Hz); 4.56 (d, 1 H, J = 4.4 Hz); 7.25 (dt, 1 H, J = 7.3 und 1.1 Hz); 7.32 (ddd, 1 H, J = 8.6, 7.3 und 1.4 Hz); 7.44 (m, 1 H); 7.52-7.60 (m, 2H); 7.67 (m, 1 H); 7.83-7.89 (m, 2H); 8.02 (br d, 1 H, J = 7.8 Hz).
LC-MS (Methode 7): [M+H]+: m/z = 453.2, Rt = 2.7 min.

Stufe 6:

4-[2-(9-Benzolsulfonyl-1,3,4,9-tetrahydro-β-carbolin-2-yl)-1-(tert-butyldimethylsilanyloxy)-ethyl]cyclohexanon

Eine Lösung des Produkts aus Stufe 6 (553 mg, 1.22 mmol), Imidazol (250 mg, 3.66 mmol) und tert-Butyldimethylchlorsilan (275 mg, 1.83 mmol) in wasserfreiem N,N-Dimethylformamid (20 mL) wurde 48 h bei Raumtemperatur gerührt. Das Reaktionsgemisch wurde i. Vak. eingeengt. Der Rückstand wurde mit 25 % Kaliumcarbonat-Lösung (30 mL) versetzt und mit Dichlormethan (3 × 35 mL) extrahiert. Die vereinigten organischen Phasen wurden mit Natriumsulfat getrocknet und i. Vak. eingeengt. Das Rohprodukt (1.3 g) wurde durch Flashchromatographie (85 g, 20 × 3.8 cm) mit Cyclohexan / Ethylacetat (2:1) gereinigt.
Ausbeute: 588 mg (85 %), weißer Feststoff
Schmelzpunkt: 150-152 °C
1 H-NMR (DMSO-d6): 0.03 (s, 3H); 0.04 (s, 3H); 0.85 (s, 9H); 1.44-1.66 (m, 2H); 1.86-2.06 (m, 3H); 2.14-2.25 (m, 2H); 2.28-2.47 (m, 2H); 2.55-2.70 (m, 4H); 2.71-2.92 (m, 2H); 3.83 (m, 1 H); 3.89 (d, 1 H, J = 16.8 Hz); 4.00 (d, 1 H, J = 16.8 Hz); 7.26 (dt, 1 H, J = 7.3 und 1.2 Hz); 7.32 (ddd, 1 H, 8.6, 7.3 und 1.4 Hz); 7.42-7.48 (m, 1 H); 7.52-7.60 (m, 2H); 7.64-7.72 (m, 1 H); 7.80-7.88 (m, 2H); 8.00-8.06 (m, 1 H).

Stufe 7:

4-[2-(9-Benzofsulfonyl-1,3,4,9-tetrahydro-β-carbolin-2-yl)-1-(tert-butyldimethylsilanyloxy)-ethyl]-1-dimethylamino-cyclohexancarbonitril

Ein Gemisch von 4 M Salzsäure (256 μL) und Methanol (153 μL) wurde unter Eiskühlung mit 40 % wässriger Dimethylamin-Lösung (596 μL, 4.73 mmol) versetzt. Dem Gemisch wurden eine Lösung des Produkts aus Stufe 6 (557 mg, 0.98 mmol) in Methanol (3 mL) und Tetrahydrofuran (3 mL) zugesetzt. Anschließend wurden Kaliumcyanid (153

mg, 2.36 mmol) und Wasser (2 mL) zugegeben. Die Suspension wurde über Nacht bei Raumtemperatur gerührt und danach mit Wasser (20 mL) verdünnt. Das Gemisch wurde mit Diethylether (5 × 20 mL) extrahiert. Die vereinigten organischen Phasen wurden mit Natriumsulfat getrocknet und i. Vak. eingeengt.
Ausbeute: 609 mg (100 %), gelbliches Öl
Es handelt sich um ein Diastereoisomerengemisch.
1 H-NMR (DMSO-d6): Das Spektrum enthält alle erwarteten Signale.

Stufe 8:

[4-[2-(9-Benzolsulfonyl-1,3,4,9-tetrahydro-β-carbolin-2-yl)-1-(tert-butyldimethylsilanyloxy)-ethyl]-1-(3-fluorphenyl)cyclohexyl]dimethylamin

Eine Lösung des Produkts aus Stufe 7 (521 mg, 0.84 mmol) in wasserfreiem Tetrahydrofuran (20 mL) wurde unter Eiskühlung und Argon mit einer 1 M Lösung von 3-Fluorphenylmagnesiumbromid in Tetrahydrofuran (2.5 mL, 2.5 mmol) versetzt und 2 d bei Raumtemperatur gerührt. Anschließend wurde das Reaktionsgemisch mit gesättigter Ammoniumchlorid-Lösung und Wasser (je 10 mL) versetzt und die Phasen wurden getrennt. Die wässrige Phase wurde mit Diethylether (2 × 30 mL) extrahiert. Die vereinigten organischen Phasen wurden mit Natriumsulfat getrocknet und i. Vak. eingeengt. Der Rückstand (491 mg) wurde durch Flashchromatographie mit Cyclohexan / Ethylacetat (4:1) gereinigt, wodurch das noch mit 3-Fluorphenol verunreinigte Produkt (211 mg) erhalten wurde. Dieses wurde erneut durch Flashchromatographie mit Cyclohexan / tert-Butylmethylether (4:1) gereinigt.
Ausbeute: 149 mg (25 %), weißer Feststoff 1 H-NMR (DMSO-d6): 0.01 (s, 3H); 0.04 (s, 3H); 0.88 (s, 9H); 1.15-1.80 (m, 7H); 1.92 (s, 6H); 2.56-2.56 (m, 6H); 2.56-2.95 (m, 2H); 3.65-3.73 (m, 1H); 3.86 (d, 1H, J = 16.9 Hz); 3.99 (d, 1H, J = 17.0 Hz); 7.00-7.15 (m, 2H); 7.20-7.45 (m, 5H); 7.50-7.59 (m, 2H); 7.62-7.70 (m, 1 H); 7.80-7.87 (m, 2H); 8.01 (d, 1 H, J = 8.2 Hz).
13C-NMR (DMSO-d6): -5.1; -4.9; -4.0; -3.8; 17.9; 20.5; 24.1; 25.8; 25.9; 32.6; 32.7; 37.4; 49.7; 51.6; 58.3; 61.3; 71.3; 74.1; 111.6 (d, J = 22 Hz); 112.6 (d, J = 21 Hz); 113.1; 113.7; 117.1; 118.6; 120.7 (br); 122.4; 122.5; 123.6; 124.4; 126.0; 128.8 (d, J = 8 Hz); 129.3; 129.8; 132.9; 134.5; 135.3; 137.4; 143.1 (d, J = 6 Hz); 161.9 (d, J = 242 Hz).
LC/MS (Methode 8): [M+H]+: m/z = 691.4, Rt = 4.2 min.

Stufe 9:

2-(9-Benzolsulfonyl-1,3,4,9-tetrahydro-β-carbolin-2-yl)-1-[4-dimethylamino-4-(3-fluorphenyl)-cyclohexyl]ethanol

Eine Lösung des Produkts aus Stufe 8 (176 mg, 0.255 mmol) in Tetrahydrofuran (20 mL) wurde mit 2 M Salzsäure (20 mL) versetzt und 1 d bei Raumtemperatur gerührt. Anschließend wurde das Reaktionsgemisch 15 h bei 50 °C und daraufhin weitere 16 h bei Raumtemperatur gerührt. Das Gemisch wurde anschließend mit 2 M Natronlauge (30 mL) alkalisch gestellt und die Phasen wurden getrennt. Die wässrige Phase wurde mit Diethylether (3 × 10 mL) extrahiert. Die vereinigten organischen Phasen wurden mit Natriumsulfat getrocknet und i. Vak. eingeengt. Das Rohprodukt (151 mg) wurde durch Flashchromatographie mit Dichlormethan / Methanol (97:3) gereinigt.
Ausbeute: 35 mg (22 %), farbloses Öl
1 H-NMR (CDCl3): 1.35-1.78 (m, 7H); 1.85 (br d, 1 H, J = 12.4 Hz); 2.04 (s, 6H); 2.48-2.66 (m, 3H); 2.50-2.86 (m, 4H); 3.05 (m, 1 H); 3.65 (t, 1 H, J = 7.8 Hz); 4.02 (d, 1 H, J = 16.4 Hz); 4.13 (d, 1 H, J = 16.4 Hz); 6.95 (t, 1H, J = 8.1 Hz); 7.01 (dd, 1H, J = 11.5 und 1.4 Hz); 7.09 (d, 1H, J = 7.5 Hz); 7.20-7.46 (m, 6H); 7.52 (m, 1 H); 7.77 (m, 2H); 8.12 (d, 1 H, J = 8.1 Hz). 13C-NMR (CDCl3): 20.8; 23.2; 23.8; 32.9; 37.6; 41.9; 49.5; 51.3; 58.7; 60.3; 70.1; 113.1 (d, J = 20 Hz); 113.6 (d, J = 21 Hz); 114.2; 117.3; 118.2; 122.4; 123.6; 124.3; 126.2; 128.5 (d, J = 8 Hz); 129.3; 129. 6; 132.6; 133.6; 136.1; 138.7; 143.0 (br); 162 (d, J = 245 Hz).
LC-MS (Methode 7): [M+H]+: m/z = 576.3, Rt = 2.4 min.

<u>Beispiel 12</u>

2-(1,3-Dihydroisoindol-2-yl)-1-(4-dimethylamino-4-phenylcyclohexyl)ethanol

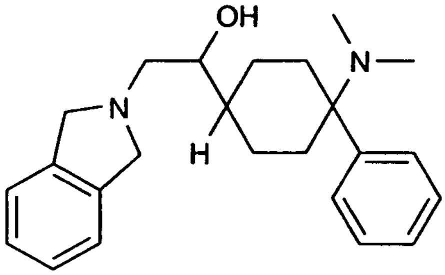

Stufe 1:

2-(1,3-Dihydroisoindol-2-yl)-1-(1,4-dioxaspiro[4.5]dec-8-yl)ethanol

Eine Lösung von 8-Oxiranyl-1,4-dioxaspiro[4.5]decan (1.41 g, 7.66 mmol), Isoindolin (1.00 g, 8.43 mmol) und Calciumtrifluormethansulfonat (1.29 g, 3.8 mmol) in wasserfreiem Acetonitril (60 mL) wurde über Nacht bei Raumtemperatur gerührt. Anschließend wurde das Lösungsmittel im Vakuum eingeengt. Der Rückstand wurde in Ethylacetat (50 mL) aufgenommen und die Lösung mit 25 % Kaliumcarbonatlösung (3 × 50 mL) gewaschen. Die wässrige Phase wurde mit Ethylacetat (3 × 40 mL) extrahiert, die vereinigten organischen Phasen wurden mit Natriumsulfat getrocknet und im Vakuum eingeengt.
Ausbeute: 2.04 g (88 %), beigefarbener Feststoff
1 H-NMR (DMSO-d6): 1.24-1.48 (m, 5H); 1.59 (br s, 1 H); 1.64-1.74 (m, 3H); 2.56 (dd, 1 H, J = 7.4, 12.2 Hz); 2.70 (dd, 1 H, J = 4.7, 12.2 Hz); (m, 2H); 3.44 (m, 1H); 3.83 (s, 4H); 3.83-3.94 (m, 4H); 4.30 (d, 1 H, J= 4.4 Hz); 7.15-7.26 (m, 4 H).

Stufe 2:

4-[2-(1,3-Dihydroisoindol-2-yl)-1-hydroxyethyl]cyclohexanon

Eine Lösung von 2-(1,3-Dihydroisoindol-2-yl)-1-(1,4-dioxaspiro[4.5]dec-8-yl)ethanol (2.05 g, 6.74 mmol) in Aceton (60 mL) wurde mit 2 M Salzsäure (19 mL) versetzt und über Nacht bei Raumtemperatur gerührt. Das Reaktionsgemisch wurde im Vakuum eingeengt, der Rückstand mit 2 M Natronlauge alkalisch gestellt und die Lösung mit Dichlormethan (3 × 40 mL) extrahiert. Die vereinigten organischen Phasen wurden mit Natriumsulfat getrocknet und im Vakuum eingeengt. Das Rohprodukt (1.67 g) wurde durch Flashchromatographie mit Ethylacetat / Methanol (9:1) gereinigt.
Ausbeute: 1.12 g (64 %), beigefarbener Feststoff
Schmelzpunkt: 136 °C
1H-NMR (DMSO-d6): 1.40-1.68 (m, 2H); 1.82-2.10 (m, 3H); 2.14-2.24 (m, 2H); 2.28-2.46 (m, 2H); 2.70 (dd, 1H, J = 7.1, 12.3 Hz); 2.77 (dd, 1H, J = 5.2 Hz); 3.58 (m, 1 H); 3.85-3.97 (m, 4H); 4.54 (br s, 1 H); 7.15-7.25 (m, 4H).

Stufe 3:

4-[2-(1,3-Dihydroisoindol-2-yl)-1-hydroxyethyl]-1-dimethylaminocyclohexancarbonitril

Zu auf 0 °C gekühlter 4 M Salzsäure (706 μL) in Methanol (785 μL) wurde 40 % wässrige Dimethylaminlösung (1.72 ml, 12.5 mmol) gegeben. Anschließend wurde eine Lösung von 4-[2-(1,3-Dihydroisoindol-2-yl)-1-hydroxyethyl]cyclohexanon (732 mg, 2.82 mmol) in Methanol (4 mL) und Tetrahydrofuran (6 mL) zugegeben. Das Gemisch wurde anschließend mit Kaliumcyanid (445 mg, 6.67 mmol) versetzt und über Nacht bei Raumtemperatur gerührt. Das Reaktionsgemisch wurde danach mit Wasser (80 mL) versetzt und mit Diethylether (3 × 20 mL) extrahiert. Die vereinigten organischen Phasen wurden mit Natriumsulfat getrocknet und im Vakuum eingeengt.
Ausbeute: 672 mg (76 %), weißer Feststoff
1 H-NMR (DMSO-d6): 1.20-1.50 (m, 7H); 1.60-1.90 (m, 2H); 2.22 und 2.33 (2 s, 6H); 2.63-2.75 (m, 2H); 3.45 (m, 1 H); 3.82-3.94 (m, 4H); 4.36 und 4.45 (2 d, 1 H, J = jeweils 4.7 Hz); 7.15-7.24 (m, 4H).

Es wurde ein Diastereoisomerengemisch im Verhältnis von ca. 4.5:1 erhalten.

Stufe 4:

2-(1,3-Dihydroisoindol-2-yl)-1-(4-dimethylamino-4-phenylcyclohexyl)ethanol

Eine 2 M Lösung von Phenylmagnesiumchlorid in Tetrahydrofuran (4.25 mL, 8.49 mmol) wurde unter Eiskühlung

tropfenweise mit einer Lösung von 4-[2-(1,3-Dihydroisoindol-2-yl)-1-hydroxyethyl]-1-dimethylaminocyclohexancarbonitril (666 mg, 2.12 mmol) in wasserfreiem Tetrahydrofuran (30 mL) versetzt und über Nacht bei Raumtemperatur gerührt. Das Gemisch wurde anschließend unter Eiskühlung tropfenweise mit gesättigter Ammoniumchloridlösung und Wasser (jeweils 10 mL) versetzt. Das Tetrahydrofuran wurde im Vakuum abdestilliert und der Rückstand mit Diethylether (3 × 30 mL) extrahiert. Die vereinigten organischen Phasen wurden mit Natriumsulfat getrocknet und im Vakuum eingeengt. Das Rohprodukt (761 mg) wurde durch Flashchromatographie mit Chloroform / Methanol (9:1) gereinigt. Erneute Flashchromatographie mit Chloroform (mit 25 % wässriger Ammoniaklösung gesättigt) / Methanol (95:5) ergabe eine Ausbeute von 83 mg (10 %), beigefarbener Feststoff
Schmelzpunkt: 130-135 °C
1 H-NMR (CDCl3): 1.26 (s, 1 H); 1.40-1.90 (m, 7H); 2.05 (s, 6H); 2.58-2.70 (m, 2H); 2.79 (dd, 1H, J = 10.2, 11.8 Hz); 2.84 (dd, 1H, J = 3.5, 11.8 Hz); 3.63 (m, 1 H); 3.93 (d, 2H, J = 11.1 Hz); 4.12 (d, 2H, J = 11.1 Hz); 7.21-7.29 (m, 5H); 7.30-7.39 (m, 4H).

Es wurde ein Diastereoisomerengemisch im Verhältnis von ca. 4:1 erhalten.

Beispiel 13 und Beispiel 14

(1-(4-Dimethylamino-4-phenylcyclohexyl)-2-phenoxyethanol, unpolares Diastereoisomer) (1-(4-Dimethylamino-4-phenylcyclohexyl)-2-phenoxyethanol, polares Diastereoisomer)

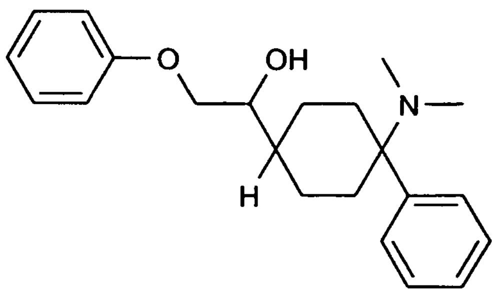

Stufe 1:

8-Oxiranyl-1,4-dioxaspiro[4.5]decan

Eine 60 %ige Dispersion von Natriumhydrid in Mineralöl (1.78 g, 44.59 mmol) wurde in Dimethylsulfoxid (25 mL) aufgenommen und mit Trimethylsulfoxoniumiodid (9.80 g, 44.6 mmol) versetzt. Das Gemisch wurde 45 min bei Raumtemperatur gerührt. Anschließend wurde dem Gemisch eine Lösung von 1,4-Dioxaspiro[4,5]decan-8-carbaldehyd (7.59 g, 44.6 mmol) in Dimethylsulfoxid (20 mL) zugesetzt. Das Reaktionsgemisch wurde 18 h bei 60 °C gerührt. Nach dem Abkühlen wurde das Gemisch in Wasser (100 ml) gegossen und mit Diethylether (4 × 20 mL) extrahiert. Die vereinigten organischen Phasen wurden mit Natriumsulfat getrocknet und im Vakuum eingeengt. Das Rohprodukt (4.64 g) wurde durch Flashchromatographie mit Cyclohexan / Ethylacetat (4:1) gereinigt.
Ausbeute: 1.09 g (13 %), farbloses Öl
1 H-NMR (DMSO-d6): 1.10-1.85 (m, 8H); 2.50 (2H, überlagert vom DMSO-Signal); 2.64 (dd, 1 H, J = 5.0, 4.0 Hz); 2.71 (ddd, 1 H, J = 6.6, 4.0, 2.7 Hz); 3.84 (s, 4H).

Auf analoge Weise wurden folgende Produktchargen gewonnen:

a) aus 1.16 g 1,4-Dioxaspiro[4,5]decan-8-carbaldehyd 247 mg, 20 % d. Th.
b) aus 2.99 g 1,4-Dioxaspiro[4,5]decan-8-carbaldehyd 560 mg, 17 % d. Th.
c) aus 7.6 g 1,4-Dioxaspiro[4,5]decan-8-carbaldehyd 7.34 g, wobei diese Charge große Mengen an Dichlormethan und Cyclohexan enthielt. Der Gehalt an Produkt war maximal ca. 30 % d.Th.

Stufe 2:

1-(1,4-Dioxaspiro[4.5]dec-8-yl)-2-phenoxyethanol

Eine 60 %ige Dispersion von Natriumhydrid in Mineralöl (834 mg, 20.7 mmol) wurde in wasserfreiem N,N-Dimethylformamid (10 mL) aufgenommen und mit Phenol (1.96 g, 20.8 mmol) versetzt. Das Gemisch wurde 15 min bei Raumtemperatur gerührt, anschließend wurde eine Lösung von 8-Oxiranyl-1,4-dioxaspiro[4.5]decan (2.62 g, Gehalt ca. 30 %, ca. 4 mmol) in N,N-Dimethylformamid (6 mL) zugesetzt. Das Reaktionsgemisch wurde 5.5 h bei 120 °C gerührt, dann auf Raumtemperatur abgekühlt, mit Wasser (1 mL) versetzt und im Vakuum eingeengt. Der Rückstand wurde wiederholt mit Toluol versetzt und jeweils wieder im Vakuum eingeengt. Das Rohprodukt (2.9 g) wurde durch Flashchromatographie (200 g, 20 × 5.6 cm) mit Cyclohexan 1 Ethylacetat (4: 1) gereinigt
Ausbeute: 1.01 g (ca. 90 %), farbloses Öl
1 H-NMR (DMSO-d6): 1.30-1.80 (m, 9H); 3.59 (m, 1 H); 3.82-3.88 (s, 4H, überlagert von dd, 1 H); 3.93 (dd, 1 H, J = 4.2, 9.9 Hz); 4.79 (d, 1 H, J = 5.4 Hz); 6.88-6.95 (m, 3H); 7.24-7.30 (m, 2H).

Stufe 3:

4-(1-Hydroxy-2-phenoxyethyl)cyclohexanon

Eine Lösung von 1-(1,4-Dioxaspiro[4.5]dec-8-yl)-2-phenoxyethanol (1.25 g, 4.5 mmol) in Aceton (30 mL) wurde mit 2 M Salzsäure versetzt und 48 h bei Raumtemperatur gerührt. Das Aceton wurde im Vakuum entfernt, der pH-Wert des wässrigen Rückstandes mit 2 M Natronlauge alkalisch gestellt und der wässrige Rückstand mit Dichlormethan (4 × 20 mL) extrahiert. Die vereinigten organischen Phasen wurden mit Natriumsulfat getrocknet und im Vakuum eingeengt.
Ausbeute: 928 mg (88 %), gelbliches Öl
1 H-NMR (DMSO-d6): 1.45-1.65 (m, 2H); 1.88-2.13 (m, 3H); 2.15-2.26 (m, 2H); 2.31-2.45 (m, 2H); 3.72 (m, 1 H); 3.90 (dd, 1 H, J = 6.2, 9.9 Hz); 3.99 (dd, 1H, J = 4.4, 9.9 Hz); 4.96 (d, 1 H, J = 5.4 Hz); 6.90-6.98 (m, 3H); 7.25-7.32 (m, 2H).

Stufe 4:

4-[1-(1-Ethoxy-ethoxy)-2-phenoxyethyl]cyclohexanon

Eine Lösung von 4-(1-Hydroxy-2-phenoxyethyl)cyclohexanon (919 mg, 3.92 mmol) in wasserfreiem Dichlormethan (20 mL) wurde mit Pyridiniumtosylat (15 mg, 0.06 mmol) und Ethylvinylether (339 mg, 450 μL, 4.70 mmol) versetzt und über Nacht bei Raumtemperatur gerührt. Anschließend wurde das Gemisch mit Dichlormethan (20 mL) versetzt und mit Wasser, 5 %iger Natriumhydrogencarbonat-Lösung sowie gesättigter Natriumchlorid-Lösung (je 50 mL) gewaschen. Die organische Phase wurde mit Natriumsulfat getrocknet und im Vakuum eingeengt.
Das Rohprodukt (1.09 g) wurde durch Flashchromatographie mit Cyclohexan / Ethylacetat (4:1) gereinigt.
Ausbeute: 929 mg (77 %), farbloses Öl 1 H-NMR (DMSO-d6): 1.02-1.13 (m, 3H); 1.21 (dd, 3H, J = 5.2, 9.1 Hz); 1.44-1.70 (m, 3H); 1.90-2.30 (m, 4H); 2.32-2.47 (m, 2H); 3.38-3.64 (m, 2H); 3.71-3.88 (m, 1H); 3.94-4.15 (m, 2H); 4.80 und 4.90 (2 q, 1H, J = 5.3 Hz); 6.90-6.94 (m 3H); 7.29 (t, 2H, J = 8.0 Hz).
Das Produkt wurde als Diastereoisomerengemisch erhalten.

Stufe 5:

1-Dimethytamino-4-[1-(1-ethoxy-ethoxy)-2-phenoxyethyl]cyclohexancarbonitril

Ein Gemisch aus 4 M Salzsäure (747 μL) und Methanol (448 μL) wurde unter Eiskühlung mit 40 % wässriger Dimethylaminlösung (1.73 mL, 13.7 mmol) versetzt und zu 4-[1-(1-Ethoxy-ethoxy)-2-phenoxyethyl]cyclohexanon (880 mg, 2.87 mmol) gegeben, bevor Kaliumcyanid (448 mg, 6.88 mg) zugesetzt wurde. Zur Lösungsvermittlung wurde noch Tetrahydrofuran (3 mL) zugegeben. Das Reaktionsgemisch wurde 6 h bei Raumtemperatur gerührt, anschließend mit Wasser (50 mL) versetzt und mit Diethylether (4 × 30 mL) extrahiert. Die vereinigten organischen Phasen wurden im Vakuum eingeengt, der Rückstand in Dichlormethan (30 mL) aufgenommen und mit Wasser (30 mL) gewaschen. Die organische Phase wurde mit Natriumsulfat getrocknet und im Vakuum eingeengt.
Ausbeute: 877 mg (84 %), farbloses Öl
1H-NMR (DMSO-d6): 1.00-1.12 (m, 3H); 1.16-1.24 (m, 3H); 1.30-2.00 (m, 8H); 2.10-2.30 (m, 7H); 3.40-3.70 (m, 3H); 4.00-4.10 (m, 2H); 4.74-4.90 (m, 1 H); 6.90-6.99 (m, 3H); 7.25-7.32 (m, 2H).
Produkt liegt als Gemisch von Diastereoisomeren vor.

Stufe 6:

{4-[1-(1-Ethoxy-ethoxy)-2-phenoxyethyl]-1-phenylcyclohexyl}dimethylamin

Zu einer 2 M Lösung von Phenylmagnesiumchlorid in Tetrahydrofuran (3.6 mL, 7.3 mmol) wurde unter Eiskühlung tropfenweise eine Lösung von 1-Dimethylamino-4-[1-(1-ethoxy-ethoxy)-2-phenoxyethyl]cyclohexancarbonitril (871 mg, 2.4 mmol) in wasserfreiem Tetrahydrofuran (15 mL) gegeben. Das Gemisch wurde über Nacht bei Raumtemperatur gerührt und anschließend mit gesättigter Ammoniumchloridlösung und Wasser (jeweils 5 mL) versetzt. Die Phasen wurden getrennt und die wässrige Phase mit Diethylether (3 × 20 mL) extrahiert. Die vereinigten organischen Phasen wurden mit Natriumsulfat getrocknet und im Vakuum eingeengt.
Ausbeute: 946 mg (99 %), gelbliches Öl
1 H-NMR (DMSO-d6): 0.90-1.26 (m, 6H); 1.26-1.80 (m, 9H); 1.93 (s, 6H); 2.61-2.71 (m, 1H); 3.40-3.69 (m, 2H); 3.93-4.14 (m, 2H); 4.71-4.92 (m, 1H); 6.70-7.70 (m, 10H).
Das Produkt wurde als Diastereoisomeren-Gemisch erhalten.

Stufe 7:

1-(4-Dimethylamino-4-phenylcyclohexyl)-2-phenoxyethanol

Eine Lösung von {4-[1-(1-Ethoxy-ethoxy)-2-phenoxyethyl]-1-phenylcyclohexyl}dimethylamin (892 mg, 2.16 mmol) in Aceton (30 mL) wurde mit 2 M Salzsäure (10 mL) versetzt und über Nacht bei Raumtemperatur gerührt. Anschließend wurde der pH-Wert des Gemisches mit 0.5 M Natronlauge alkalisch gestellt und dann mit Dichlormethan (3 × 30 mL) extrahiert. Die vereinigten organischen Phasen wurden mit Natriumsulfat getrocknet und im Vakuum eingeengt. Das Rohprodukt (1.26 g) wurde durch Flashchromatographie mit Ethylacetat / Methanol (9:1→0:1) gereinigt. 1-(4-Dimethylamino-4-phenylcyclohexyl)-2-phenoxyethanol (unpolares Diastereoisomer) Ausbeute: 317 mg (43 %), gelbliches Öl
1H-NMR (DMSO-d6): 1.22-1.38 (m, 2H); 1.40-1.74 (m, 5H); 1.92 (s, 6H); 2.58-2.74 (m, 2H); 3.56-3.65 (m, 1 H); 3.88 (dd, 1 H, J = 10.0, 6.3 Hz); 3.98 (dd, 1H, J = 10.0, 4.0 Hz); 4.78 (d, 1 H, J = 5.5 Hz); 6.88-6.97 (m, 3H); 7.18-7.37 (m, 7H).
1-(4-Dimethylamino-4-phenylcyclohexyl)-2-phenoxyethanol (polares Diastereoisomer) Ausbeute: 41 mg (5 %), gelblicher Feststoff
Schmelzpunkt: 145-147 °C
1 H-NMR (DMSO-d6): 0.80-1.10 (m, 2H); 1.38-1.54 (m, 4H); 1.72 (br d, 1H, J = 12.8 Hz); 1.89 (s, 6H); 2.58-2.74 (m, 2H); 3.40-3.52 (m, 1 H); 3.77 (dd, 1 H, J = 9.9, 6.1 Hz); 3.84 (dd, 1 H, J = 9.9, 4.4 Hz); 4.67 (br s, 1 H); 6.84-6.92 (m, 3H); 7.20-7.40 (m, 7H).

<u>Beispiel 15:</u>

2-Benzyloxy-1-(4-dimethylamino-4-phenylcyclohexyl)ethanol

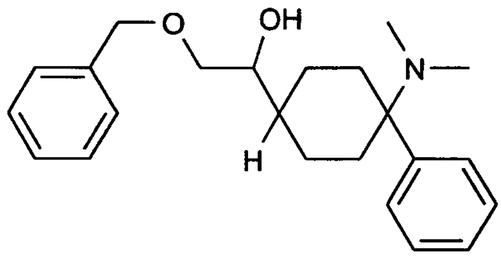

Stufe 1:

Bei Ersatz von Phenol durch Benzylalkohol in Beispiel 13 und 14, Stufe 2 und anschließende analoge Umsetzung wurde Beispiel 15 erhalten:

1 H-NMR (DMSO-d6): 1.15-1.68 (m, 8H); 1.92 (s, 6H); 2.52-2.70 (m, 2H); 3.35-3.50 (m, 2H); 4.49 (m, 3H); 7.18-7.40 (m, 10 H).
13C-NMR (DMSO-d6): 22.3; 23.7; 32.6; 32.7; 37.5; 58.1; 72.1; 72.7; 72.9; 126.1 (br); 126.3 (br); 127.1 (br); 127.4; 128.1; 138.7; 139.7.
Es wurde nur ein Diastereoisomer isoliert.

Beispiel 16

2-(Cyclohexyloxy)-1-(4-(dimethylamino)-4-phenylcyclohexyl)ethanol

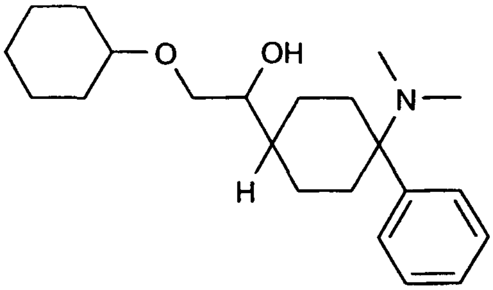

Bei Ersatz von Phenol durch Cyclohexanol in Beispiel 13 und 14, Stufe 2 und anschließende analoge Umsetzung wurde Beispiel 16 erhalten:

1 H-NMR (DMSO-d6): 1.10-1.57 (m, 16H); 1.80 (m, 2H); 1.92 (s, 6H); 2.63 (br d, 2H, J = 13.9 Hz); 3.22 (m, 1 H); 3.28-3.45 (m, 3H); 4.31 (d, 1H, J = 4.7 Hz); 7.23 (m, 1 H); 7.28-7.35 (m, 4H).
13C-NMR (DMSO-d6): 22.7; 23.2; 23.7; 25.3; 31.7; 32.6; 37.4; 58.3; 70.2; 73.0; 76.4; 126.1; 126.5; 127.0; 139.6.
Es wurde nur ein Diastereoisomer isoliert.

Beispiel 17:

2-Cyclohexyloxy-1-(4-dimethylamino-4-thiophen-2-yl-cyclohexyl)ethanol

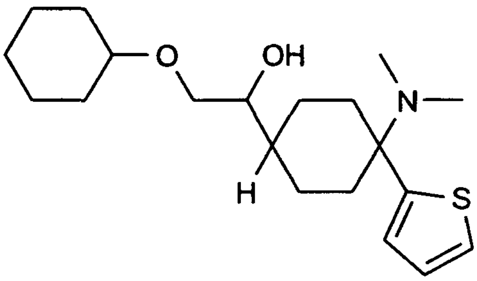

Bei analoger Durchführung zu Beispiel 16 mit Ersatz von Phenylmagnesiumchlorid durch 2-Thienylmagnesiumbromid in Stufe 6 wurde das Beispiel 17 erhalten. 1 H-NMR (DMSO-d6): 1.10-1.57 (m, 15H); 1.80 (m, 2H); 1.99 (s, 6H); 2.42 (d, 2H, J = 13.7 Hz); 3.21 (m, 1 H); 3.27-3.44 (m, 3H); 4.34 (d, 1 H, J = 4.7 Hz); 6.89 (dd, 1H, J = 1.1 und 3.4 Hz); 7.02 (dd, 1 H, J = 3.4 und 5.1 Hz); 7.37 (dd, 1 H, J = 1.1 und 5.1 Hz).
13C-NMR (DMSO-d6): 22.2; 23.2; 23.6; 25.3; 31.7; 35.1; 37.3; 58.1; 70.1; 72.9; 76.4; 122.8; 123.5; 126.0; 145.2.
Es wurde nur ein Diastereoisomer isoliert.

Beispiel 18

1-(4-Dimethylamino-4-phenylcyclohexyl)-2-indol-1-yl-ethanol

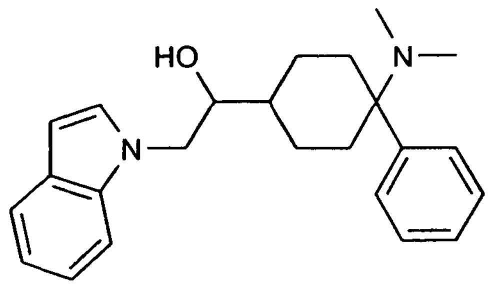

Bei Ersatz von Phenol durch Indol in Beispiel 13 und 14, Stufe 2 und anschließende analoge Umsetzung wurde Beispiel 18 erhalten:

1 H-NMR (CDCl3): 1.44-1.64 (m, 3H); 1.64-1.90 (m, 4H); 2.05 (s, 6H); 2.65-2.74 (m, 2H); 3.89 (ddd, 1H, J = 2.8, 6.6, 9.2 Hz); 4.06 (dd, 1H, J = 9.1, 14.3 Hz); 4.40 (dd, 1 H, J = 2.8, 14.3 Hz); 6.52 (d, 1H, J = 3.1 Hz); 7.11 (m, 1H); 7.18 (d, 1H, J = 3.1 Hz); 7.22 (ddd, 1 H, J = 1.2, 7.1, 8.2 Hz); 7.16-7.42 (m, 6H); 7.64 (d, 1 H, J = 7.9 Hz).

<u>Beispiel 19:</u>

1-(4-Dimethylamino-4-phenylcyclohexyl)-2-phenylsulfanylethanol

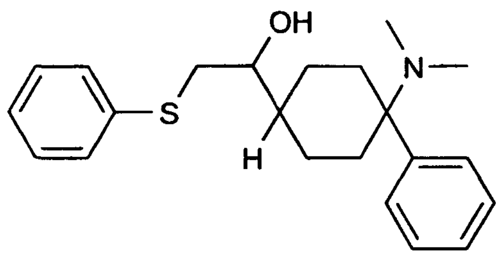

Bei Ersatz von Phenol durch Thiophenol in Beispiel 13 und 14, Stufe 2 und anschließende analoge Umsetzung wurde Beispiel 19 erhalten:

1 H-NMR (CDCl3): 1.48-1.64 (m, 4H); 1.66-1.82 (m, 4H); 2.10 (s, 6 H); 2.60-2.72 (m, 2H); 2.94 (dd, 1 H, J = 8.8 und 13.6 Hz); 3.29 (dd, 1 H, J = 3.4 und 13.6 Hz) 3.64 (m, 1 H); 7.21 (m, 1 H); 7.25-7.43 (m, 9H).
LC-MS (Methode 8): [M+H]+: m/z = 356.2, Rt = 2.6 min.

<u>Beispiel 20:</u>

2-(4-(Dimethylamino)-4-phenylcyclohexyl)-1-phenoxypropan-2-ol

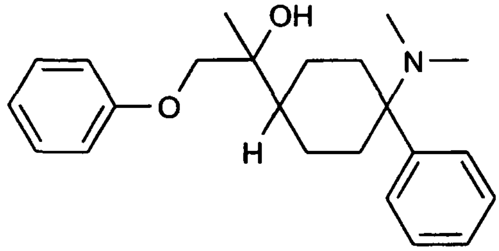

Analog zu Beispiel 13 und 14 wurde Stufe 7 hergestellt, von der ausgehend wie nachfolgend beschrieben die Synthese des Beispiels 20 erfolgte:

Stufe 8:

1-(4-Dimethylamino-4-phenylcyclohexyl)-2-phenoxyethanon

Eine Lösung von 1-(4-Dimethylamino-4-phenylcyclohexyl)-2-phenoxyethanol (800 mg, 2.35 mmol) in wasserfreiem Dichlormethan (25 mL) wurde mit einer 15 % Lösung von Dess-Martin-Periodinan in Dichlormethan (22.9 g, 8.10 mmol) versetzt. Das Gemisch wurde 4 h bei Raumtemperatur und anschließend 1.5 h bei 40 °C gerührt und dann mit Diethylether (100 mL) versetzt. Die Suspension wurde mit 25 % Kaliumcarbonat-Lösung, 5 % Natriumhydrogencarbonat-Lösung, 1 M Natriumthiosulfat-Lösung und Wasser (je 50 mL) gewaschen. Die organische Phase wurde mit Natriumsulfat getrocknet und i. Vak. eingeengt.
Ausbeute: 931 mg, (>100 %), gelblicher Feststoff
1 H-NMR (DMSO-d6): 1.40-1.53 (m, 2H); 1.62-1.72.(m, 2H); 1.75-1.88 (m, 2H); 1.95 (s, 6H); 2.58-2.70 (m, 3H); 4.94 (s, 2H); 6.86-6.90 (m, 2H); 6.91-6.96 (m, 1 H); 7.25-7.30 (m, 3H); 7.33-7.37 (m, 4H).

Stufe 9:

2-(4-Dimethylamino-4-phenylcyclohexyl)-1-phenoxypropan-2-ol

Eine Suspension des Produkts aus Stufe 7 (Rohprodukt, 300 mg, max. 0.89 mmol) in wasserfreiem Tetrahydrofuran (30 mL) wurde unter Argon und Eiskühlung mit einer 3 M Lösung von Methylmagnesiumbromid in Diethylether (8.8 mL, 26.7 mmol) versetzt und 3 h bei Raumtemperatur gerührt. Das Gemisch wurde anschließend unter Eiskühlung tropfenweise mit gesättigter Ammoniumchlorid-Lösung und Wasser (je 10 mL) versetzt und mit Diethylether (3 × 20 mL) extrahiert. Die vereinigten organischen Phasen wurden mit Natriumsulfat getrocknet und i. Vak. eingeengt. Das Rohprodukt (167 mg) wurde durch Flashchromatographie (20 g, 20 × 2.0 cm) mit Ethylacetat / Methanol (9:1) gereinigt.
Ausbeute: 102 mg (32 % bezogen auf Stufe 6), farbloses Öl
1 H-NMR (DMSO-d6): 1.15 (s, 3H); 1.20-1.30 (m, 2H); 1.50-1.70 (m, 5H); 1.92 (s, 6H); 2.71 (br, d, 2H, J = 14.3 Hz); 3.76 (d, 1 H, J = 9.3 Hz); 3.83 (d, 1H, J = 9.3 Hz); 4.38 (s, 1 H); 6.88-6.97 (m, 3H); 7.18-7.40 (m, 7H).
LC-MS (Methode 7): [M+H]+: m/z = 354.3, Rt = 3.3 min.

Vergleichsbeispiel 1

3-{3-[4-(Dimethylamino)-4-phenylcyclohexyl]-3-hydroxyprop-1-inyl}-1*H*-indol-1-carbonsäure-tert-butylester

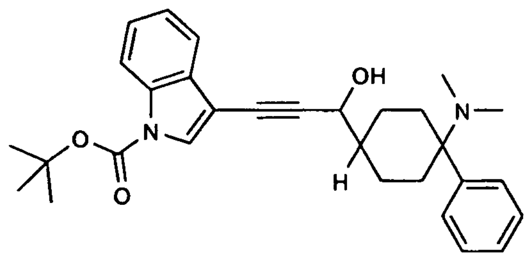

Die Synthese dieser Verbindung sowie die nachfolgenden Daten zur biologischen Aktivität sind in der Literatur beschrieben (WO 04/043900)

Vergleichsbeispiel 2

(4-(Dimethylamino)-4-phenyl-4',9'-dihydro-3'H-spiro[cyclohexan-1,1'-pyrano[3,4-b]indol]-2-yl)methanol

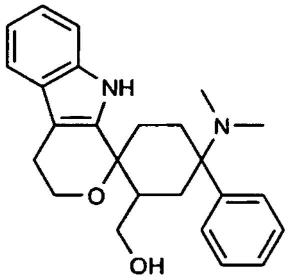

Stufe 1:

4-(Dimethylamino)-4-phenyl-4',9'-dihydro-3'*H*-spiro[cyclohexan-1,1'-pyrano[3,4-*b*]indol]-2-yl)methanol (eins von 4 möglichen racemischen Diastereoisomerenpaaren)

Der 2-(4-(Dimethylamino)-4-phenyl-4',9'-dihydro-3'H-spiro[cyclohexan-1,1'-pyrano[3,4-b]indol]-2-yl)essigsäure-methylester (190 mg, 0,44 mmol) wurde in einem Gemisch aus 2N HCl (20 ml) und Ethanol (20 ml) gelöst und 18 h bei RT gerührt. Zur Aufarbeitung wurde das Ethanol im Vakuum abgezogen, der wäßrige Rückstand mit $NaHCO_3$ neutralisiert und mit 2 N NaOH streng basisch gemacht. Die wäßrige Lösung wurde mit Ethylacetat (3 x 10 ml) extrahiert. Die vereinigten organischen Phasen wurden über $MgSO_4$ getrocknet und anschließend eingeengt. Der erhaltene feste Rückstand erwies sich als eines der vier möglichen Diastereoisomeren des gewünschten Alkohols in reiner Form. Das Produkt wurde so in einer Ausbeute von 153 mg (89 %) mit einem Schmelzpunkt von 219-233 °C (aus Propan-2-ol) erhalten.
$^{13}$C-NMR (101 MHz, DMSO-$d_6$, δ ppm): 22.1, 27.9, 30.5, 31.0, 37.9, 43.9, 59.1, 60.8, 61.6, 73.8, 106.5, 111.0, 117.3, 118.2, 120.4, 126.2, 126.3, 127.59, 127.63, 135.9, 136.6, 137.4 Untersuchungen zur Wirksamkeit der erfindungsgemäßen Verbindungen

*Messung der ORL1-Bindung*

Die Verbindungen wurden in einem Rezeptorbindungsassay mit $^3$H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3$H-Nociceptin/Orphanin FQ betrug bei diesen Versuchen 0.5 nM. Die Bindungsassays wurden mit je 20 μg Membranprotein je 200 μl Ansatz in 50 mM Hepes, pH 7,4, 10 mM $MgCl_2$ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschließende Messung im Szintillationscounter Trilux (Wallac, Finnland), bestimmt. Die Affinität wird in Tabelle 1 als nanomolarer $K_i$-Wert in oder % Inhibition bei c=1 μM angegeben.

*Messung der μ-Bindung*

Die Rezeptoraffinität zum humanen μ-Opiatrezeptor wurde in einem homogenen Ansatz in Mikrotiterplatten bestimmt. Hierzu wurden Verdünnungsreihen des jeweils zu prüfenden Verbindung mit einer Rezeptormembranpräparation (15-40 μg Protein pro 250 μl Inkubationsansatz) von CHO-K1-Zellen, welche den humanen μ-Opiatrezeptor exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 μl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 μmol/l Naloxon zugegeben. Nach Beendigung der neunzigminütigen Inkubationszeit wurden die Mikrotiterplatten für 20 Minuten bei 1000 g abzentrifugiert und die Radioaktivität in einem ß-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen μ-Opiatrezeptor bei einer Konzentration der Prüfsubstanzen von 1 μmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I $IC_{50}$ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten. In einigen Fällen wurde auf die Bestimmung des Ki-Wertes verzichtet und nur die Hemmung bei einer Testkonzentration von 1 μM bestimmt.

*Analgesieprüfung im Tail-Flick-Test an der Ratte*

Die analgetische Wirksamkeit der Testverbindungen von Beispiel 3 wurde im Brennstrahl (Tail-flick)-Test an der Ratte nach der Methode von D'Amour and Smith (J. Pharm. Exp. Ther. 72, 74 79 (1941) untersucht. Dazu wurden weibliche Sprague Dawley mit einem Gewicht zwischen 134 und 189 g verwendet. Die Tiere wurden einzeln in spezielle Testkäfige gesetzt und die Schwanzbasis einem focussierten Wärmestrahl einer Lampe (Tail-flick Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 2,5-5 Sekunden betrug. Vor Gabe einer Testverbindung wurden die Tiere innerhalb von 30 Minuten zweimal vorgetestet und der Mittelwert dieser Messungen als Vortestmittelwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel:

$$[(T_1 - T_0)/(T_2 - T_0)] \times 100$$

Dabei ist die $T_0$ die Latenzzeit vor und $T_1$ die Latenzzeit nach Substanzapplikation, $T_2$ ist die maximale Expositionszeit (12 sec).

Zur Bestimmung der Dosisabhängigkeit wurde die jeweilige Testverbindung in 3 - 5 logarithmisch ansteigenden Dosen, die jeweils die Schwellen- und die maximale Wirkdosis einschlossen, appliziert und die $ED_{50}$-Werte mit Hilfe der Regressionsanalyse bestimmt. Die $ED_{50}$-Berechnung erfolgte im Wirkmaximum, 20 Minuten nach intravenöser Substanzgabe.

*Nephelometrische Löslichkeitsuntersuchung (Phosphatpuffer pH 7.4)*

Diese Methode untersucht die Löslichkeit einer Substanz bei festgelegten Konzentrationen (1 μM, 3 μM, 10 μM, 30 μM und 100 μM) in 10 mM Phosphatpufferlösung bei pH 7.4. Initial wird eine 10 mM Lösung der Substanzen in DMSO benötigt, aus der 100-fach Stammlösungen der oben genannten Konzentrationslevel wiederum in DMSO hergestellt werden, die finale DMSO Konzentration im Versuchsansatz beträgt 1 % (v/v). Das Experiment wird in mehrfach Bestimmung durchgeführt. Nach Zugabe der DMSO Stammlösungen zum Puffer, wird der Ansatz 2h bei 37°C inkubiert, bevor eine Absorptionsbestimmung bei 620 nm stattfindet. Steigt die Absorption der Proben über die der reinen Puffer/DMSO Lösung an, so gilt dies als Indikator für eine Präzipitatbildung. Die untere Löslichkeitsgrenze ("lower bound") ist die Konzentration, die derjenigen mit erster Präzipitatbildung vorangegangen ist (z.B. 3 μM, wenn Präzipitatbildung bei 10 μM detektiert wurde).

*Vergleichsuntersuchungen*

| | % Hemmung (ORL1) [1 μM] | Ki (ORL1) Mittel [nM] | % Hemmung (μ) [1 μM] | Ki(μ) Mittel [nM] | TF Ratte $ED_{50}$ i.v. [μg/kg] | Löslichkeit (pH 7) [μmol/l] |
|---|---|---|---|---|---|---|
| Beispiel 1 | 93 | | 99 | | | |
| Beispiel 3 | | 1,9 | | 3,2 | 31,7 | |
| Beispiel 4 | | 605 | | 2075 | | 100 |
| Beispiel 5 | 97 | | 103 | | | |
| Beispiel 6 | | 0,76 | | 1,3 | | |
| Beispiel 7 | | 0,94 | | 1,8 | | |
| Beispiel 8 | | 400 | | 11 | | |
| Beispiel 9 | | 370 | | 11,3 | | |
| Beispiel 12 | 71 | | 99 | | | |
| Beispiel 13 | | 30 | | 6,5 | | |

(fortgesetzt)

| | % Hemmung (ORL1) [1 $\mu$M] | Ki (ORL1) Mittel [nM] | % Hemmung ($\mu$) [1 $\mu$M] | Ki($\mu$) Mittel [nM] | TF Ratte $ED_{50}$ i.v. [$\mu$g/kg] | Löslichkeit (pH 7) [$\mu$mol/l] |
|---|---|---|---|---|---|---|
| Beispiel 15 | | 130 | | 10,7 | | |
| Beispiel 16 | | 91 | | 12 | | |
| Beispiel 17 | | 45 | | 4,6 | | |
| Beispiel 18 | | 4,1 | | 1,0 | | |
| Vergleich 1 | | 730 | | 86 | | |
| Vergleich 2 | 80 | | 99 | | | 10 |

Aus der vorliegenden Tabelle ergibt sich, dass die erfindungsgemäßen Verbindungen der Beispiele 1, 3, 4, 5, 6, 7, 8, 9, 12, 13, 15, 16, 17 und 18 im Vergleich zu der ähnlichen Struktur der bekannten Verbindung des Vergleichsbeispiels 1 eine überraschend hohe Bindung am ORL1-Rezeptor und mitunter zusätzlich auch am $\mu$-Opioidrezeptor aufweisen. Ferner ist ersichtlich, dass die erfindungsgemäße Verbindung gemäß Beispiel 4 eine rund 10fach bessere Löslichkeit in wässrigen Medien als die Verbindung des Vergleichsbeispiels 2 aufweist.

**Patentansprüche**

**1.** Verbindung der allgemeinen Formel (1)

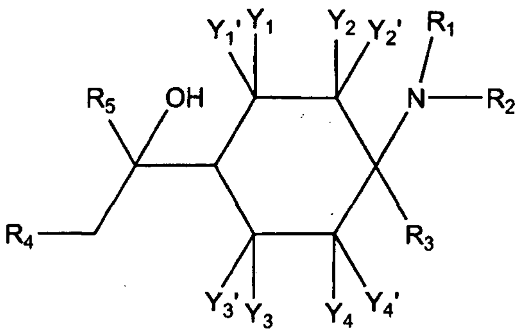

(1)

worin

$Y_1$, $Y_1$', $Y_2$, $Y_2$', $Y_3$, $Y_3$, $Y_4$ und $Y_4$' jeweils unabhängig voneinander ausgewählt sind aus der Gruppe bestehend aus -H, -F, -Cl, -Br, -I, -CN, $-NO_2$, -CHO, $-R_0$, $-C(=O)R_0$, -C(=O)-OH, $-C(=O)OR_0$, $-C(=O)NH_2$, $-C(=O)NHR_0$, $-C(=O)N(R_0)_2$, -OH, $-OR_0$, -OC(=O)H, $-OC(=O)R_0$, $-OC(=O)OR_0$, $-OC(=O)NHR_0$, $-OC(=O)N(R_0)_2$, -SH, $-SR_0$, $-S(=O)_{1-2}R_0$, $-S(=O)_{1-2}OH$, $-S(=O)_{1-2}OR_0$, $-S(=O)_{1-2}NH_2$, $-S(=O)_{1-2}NHR_0$ oder $-S(=O)_{1-2}N(R_0)_2$, $-NH_2$, $-NHR_0$, $-N(R_0)_2$, $-N^+(R_0)_3$, $-N^+(R_0)_2O^-$, $-NHC(=O)R_0$, $-NHC(=O)OR_0$, $-NHC(=O)NH_2$, $-NHC(=O)NHR_0$ und $-NHC(=O)N(R_0)_2$; oder $Y_1$ und $Y_1$', oder $Y_2$ und $Y_2$', oder $Y_3$ und $Y_3$', oder $Y_4$ und $Y_4$ gemeinsam für =O stehen;
$R_0$ jeweils unabhängig für $-C_{1-8}$-Aliphat, $-C_{3-12}$-Cycloaliphat, -Aryl, -Heteroaryl, $-C_{1-8}$-Aliphat-$C_{3-12}$-Cycloaliphat, $-C_{1-8}$-Aliphat-Aryl, $-C_{1-8}$-Aliphat-Heteroaryl. $-C_{3-8}$-Cycloaliphat-$C_{1-8}$-Aliphat, $-C_{3-8}$-Cycloaliphat-Aryl oder $-C_{3-8}$-Cycloaliphat-Heteroaryl steht;
$R_1$ und $R_2$ unabhängig voneinander für -H oder $-C_{1-8}$-Aliphat stehen; oder $R_1$ und $R_2$ zusammen einen Ring bilden und für $-(CH_2)_{2-4}$- stehen;
$R_3$ für $-R_0$ steht;

$R_4$ für -H, -F, -CL, -Br, -I, -Aryl, -Heteroaryl, -C(=O)H, -C(=O)$R_0$, -C(=O)O$R_0$, -CN, -C(=O)$NH_2$, -C(=O)NH$R_0$, -C(=O)N$(R_0)_2$, -OH, -O$R_0$, -OC(=O)H, -OC(=O)$R_0$, -OC(=O)O$R_0$, -OC(=O)NH$R_0$, -OC(=O)-N$(R_0)_2$, -$NH_2$, -NH$R_0$, -N$(R_0)_2$, -$N^+(R_0)_3$, -$N^+(R_0)_2$O-, -NHC(=O)$R_0$, -NHC(=O)O$R_0$, -NHC(=O)NH$R_0$, -NHC(=OFN$(R_0)_2$, -$NO_2$, -SH, -S$R_0$, -S$(=O)_{1-2}R_0$, -S$(=O)_{1-2}$OH, -S$(=O)_{1-2}$O$R_0$, -S$(=O)_{1-2}NH_2$, -S$(=O)_{1-2}$NH$R_0$, -S$(=O)_{1-2}$N$(R_0)_2$, -OS$(=O)_{1-2}R_0$, -OS$(=O)_{1-2}$OH, -OS$(=O)_{1-2}$O$R_0$, -OS$(=0)_{1-2}NH_2$, -OS$(=O)_{1-2}$NH$R_0$ oder -OS$(=O)_{1-2}$N$(R_0)_2$ steht;
$R_5$ für -H, -$R_0$, -C(=O)H, -C(=O)$R_0$, -C(=O)O$R_0$, -CN, -C(=O)$NH_2$, -C(=O)NH$R_0$ oder -C(=O)N$(R_0)_2$ steht;
wobei
"Aliphat" jeweils ein verzweigter oder unverzweigter, gesättigter oder ein- oder mehrfach ungesättigter, unsubstituierter oder ein- oder mehrfach substituierter, aliphatischer Kohlenwasserstoffrest ist;
"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt;
wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -$NO_2$, -CHO, =O, -$R_0$, -C(=O)$R_0$, -C(=O)OH, -C(=O)O$R_0$, -C(=O)$NH_2$, -C(=O)NH$R_0$, -C(=O)N$(R_0)_2$, -OH, -O$R_0$, -OC(=O)H, -OC(=O)$R_0$, -OC(=O)O$R_0$, -OC(=O)NH$R_0$, -OC(=O)-N$(R_0)_2$, -SH, -S$R_0$, -$SO_3$H, -S$(=O)_{1-2}$-$R_0$, -S$(=O)_{1-2}$NH2, -$NH_2$, -NH$R_0$, -N$(R_0)_2$, -$N^+(R_0)_3$, -$N^+(R_0)_2O^-$, -NHC(=O)$R_0$, -NHC$(=O)_O R_0$, -NHC(=O)$NH_2$, -NHC(=O)-NH$R_0$, -NH-C(=O)N$(R_0)_2$, -Si$(R_0)_3$ und -PO$(OR_0)_2$ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können, welche ihrerseits ein oder mehrere Heteroringatome aufweisen können, jeweils unabhängig voneinander ausgewählt aus N, O und S, und wobei jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -$NO_2$, -CHO, =O, -$R_0$, -C(=O)$R_0$, -C(=O)OH, -C(=O)O$R_0$, -C(=O)$NH_2$, -C(=O)NH$R_0$, -C(=O)-N$(R_0)_2$, -OH, -O$(CH_2)_{1-2}$O-, -O$R_0$, -OC(=O)H, -OC(=O)$R_0$, -OC(=O)O$R_0$, -OC(=O)NH$R_0$, -OC(=O)N$(R_0)_2$, -SH, -S$R_0$, -$SO_3$H, -S$(=O)_{1-2}$-$R_0$, -S$(=O)_{1-2}NH_2$, -$NH_2$, -NH$R_0$, -N$(R_0)_2$, -$N^+(R_0)_3$, -$N^+(R_0)_2O^-$, -NH(=O)$R_0$, -NHC(=O)O$R_0$, -NH-C(=O)$NH_2$, -NHC(=O)NH$R_0$, -NHC(=O)N$(R_0)_2$, -Si$(R_0)_3$ und -PO$(OR_0)_2$ verstanden wird; wobei ggf. vorhandene N-Ringatome jeweils oxidiert sein können;
in Form eines einzelnen Stereoisomers oder deren Mischung, der freien Verbindungen und/oder ihrer physiologisch verträglichen Salze.

**2.** Verbindung nach Anspruch 1, wobei $Y_1$, $Y_1$', $Y_2$, $Y_2$', $Y_3$, $Y_3$', $Y_4$ und $Y_4$' jeweils für -H stehen.

**3.** Verbindung nach einem der vorstehenden Ansprüche, wobei $R_3$ ausgewählt ist aus der Gruppe bestehend aus -Phenyl, -Benzyl oder -Phenethyl, jeweils unsubstituiert oder am Ring ein- oder mehrfach substituiert; -$C_{1-5}$-Aliphat, -$C_{4-6}$-Cycloaliphat, -Pyridyl, -Thienyl, -Thiazolyl, -Imidazolyl, -1,2,4-Triazolyl und -Benzimidazolyl, jeweils unsubstituiert oder ein- oder mehrfach substituiert.

**4.** Verbindung nach einem der vorstehenden Ansprüche, wobei $R_5$ für -H steht.

**5.** Verbindung nach Anspruch 1, welche die allgemeine Formel (4), (5), (6), (7), (8) oder (9)

(4) 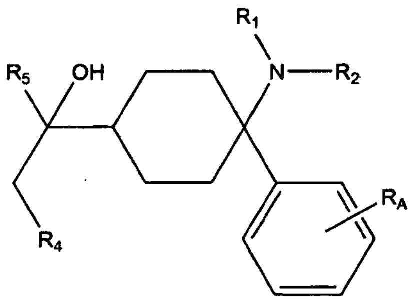 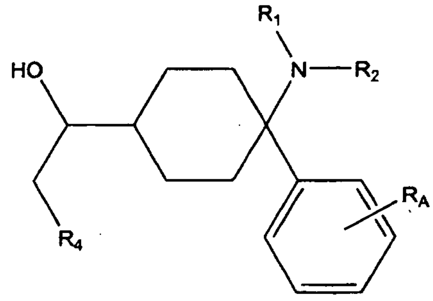 (5)

(6) 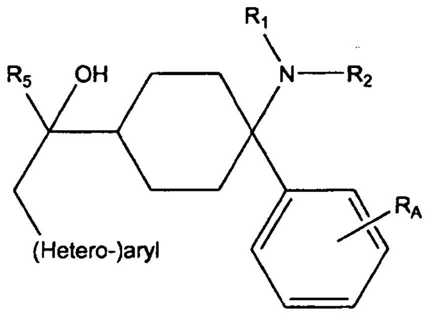 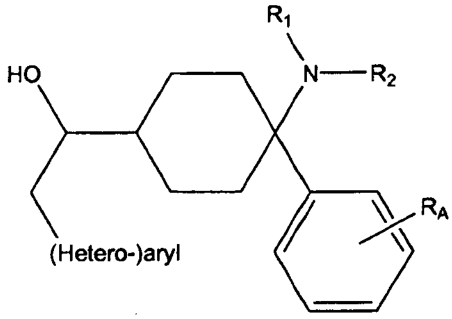 (7)

(8) 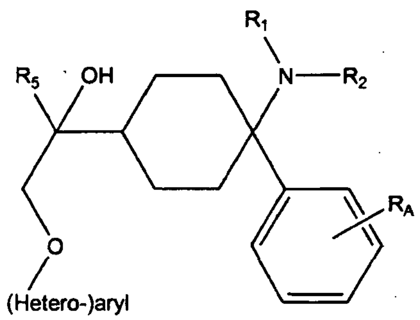 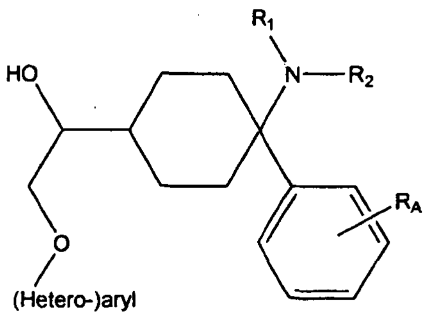 (9)

aufweist, worin, soweit vorhanden,
$R_A$ für -H, -F, -Cl, -CN, -$NO_2$ oder -$OCH_3$ steht; und
(Hetero-)aryl für Heteroaryl oder Aryl steht, jeweils unsubstituiert oder ein- oder mehr-fach substituiert.

**6.** Verbindung nach Anspruch 1, ausgewählt aus der Gruppe bestehend aus

- 1-(4-(Dimethylamino)-4-phenylcyclohexyl)-2-phenylethanol;
- 1-(4-(Dimethylamino)-4-phenylcyclohexyl)-2-phenoxyethanol;
- 1-(4-(Dimethylamino)-4-phenylcyclohexyl)-2-(1H-indol-1-yl)ethanol;
- 1-(4-(Dimethylamino)-4-phenylcyclohexyl)-2-(isoindolin-2-yl)ethanol,
- 1-(4-(Dimethylamino)-4phenylcyclohexyl)-2-(4-fluorphenyl)ethanol;
- 1-(4-(Dimethylamino)-4-(3-fluorphenyl)cyclohexyl)-2-phenylethanol;
- 1-(4-(Dimethylamino)-4-(3-methoxyphenyl)cyclohexyl)-2-phenylethanol;
- 1-(4-(Dimethylamino)-4-(thiophen-2-yl)cyclohexyl)-2-phenylethanol;
- 1-(4-Butyl-4-(dimethylamino)cyclohexyl)-2-phenylethanol;
- 1-Cyclopentyl-2-(4-(dimethylamino)-4-phenylcyclohexyl)-3-phenylpropan-2-ol;
- 1-(4-(Dimethylamino)-4-phenylcyclohexyl)-1-phenyl-2-(pyridin-4-yl)ethanol;
- 1-(4-(Dimethylamino)-4-phenylcyclohexyl)-2-(phenylthio)ethanol;
- 1-(4-(Dimethylamino)-4-phenylcyclohexyl)-2-(phenylsulfonyl)ethanol;
- 2-(Cyclohexyloxy)-1-(4-(dimethylamino)-4-phenylcyclohexyl)ethanol;
- 2-(Benzyloxy)-1-(4-(dimethylamino)-4-phenylcyclohexyl)ethanol;
- 1-(4-(Dimethylamino)-4-phenylcyclohexyl)-2-phenethoxyethanol;

• 2-((1*H*-Indol-3-yl)methoxy)-1-(4-(dimethylamino)-4-phenylcyclohexyl)ethanol;
• 2-(2-(1*H*-Indol-3-yl)ethoxy)-1-(4-(dimethylamino)-4-phenylcyclohexyl)ethanol;
• 1-(4-(Dimethylamino)-4-phenylcyclohexyl)-2-((2-(triethylsilyl)-1*H*-indol-3-yl)-methoxy)ethanol;
• 2-(4,4a-Dihydro-1*H*-pyhdo[3,4-b]indol-2(3*H*,9*H*,9a*H*)-yl)-1-(4-(dimethylamino)-4-(3-fluorphenyl)cyclohexyl)ethanol;
• 2-(4-(Dimethylamino)-4-phenylcyclohexyl)-1-phenylpropan-2-ol;
• 2-(4-(Dimethylamino)-4-phenylcyclohexyl)-1,3-diphenylpropan-2-ol;
• 2-(4-(Dimethylamino)-4-phenylcyclohexyl)-1-phenyl-3-(pyridin-2-yl)propan-2-ol;
• 2-(4-(Dimethylamino)-4-phenylcyclohexyl)-1-phenyl-3-(pyridin-3-yl)propan-2-ol; und
• 2-(4-(Dimethylamino)-4-phenylcyclohexyl)-1-phenyl-3-(pyridin-4-yl)propan-2-ol; und deren physiologisch verträgliche Salze.

**7.** Arzneimittel enthaltend wenigstens eine Verbindung nach einem der Ansprüche 1 bis 6 in Form eines einzelnen Stereoisomers oder deren Mischung, der freien Verbindungen und/oder ihrer physiologisch verträglichen Salze, sowie ggf. geeignete Zusatz- und/ oder Hilfsstoffe und/oder gegebenenfalls weiterer Wirkstoffe.

**8.** Verwendung einer Verbindung nach einem der Ansprüche 1 bis 6 in Form eines einzelnen Stereoisomers oder deren Mischung, der freien Verbindung und/oder ihrer physiologisch verträglichen Salze, zur Herstellung eines Arzneimittels zur Behandlung von Schmerz.

**9.** Verwendung einer Verbindung nach einem der Ansprüche 1 bis 6 in Form eines einzelnen Stereoisomers oder deren Mischung, der freien Verbindungen und/oder ihrer physiologisch verträglichen Salze und/oder Solvate zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

**1.** Compound of the general formula (1)

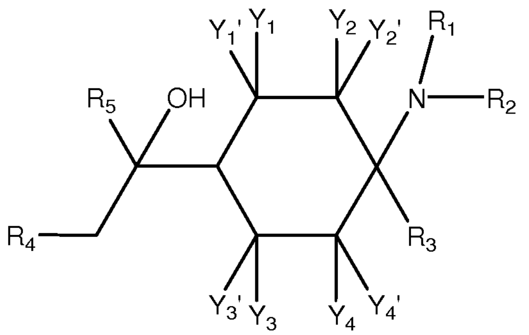

(1)

wherein

$Y_1$, $Y_1$', $Y_2$, $Y_2$', $Y_3$, $Y_3$', $Y_4$ and $Y_4$' in each case independently of each other are chosen from the group consisting of -H, -F, -Cl, -Br, -I, -CN, $-NO_2$, -CHO, $-R_0$, $-C(=O)R_0$, -C(=O)-OH, $-C(=O)OR_0$, $-C(=O)NH_2$, $-C(=O)NHR_0$, $-C(=O)N(R_0)_2$, -OH, $-OR_0$, -OC(=O)H, $-OC(=O)R_0$, $-OC(=O)OR_0$, $-OC(=O)NHR_0$, $-OC(=O)N(R_0)_2$, -SH, $-SR_0$, $-S(=O)_{1-2}R_0$, $-S(=O)_{1-2}OH$, $-S(=O)_{1-2}OR_0$, $-S(=O)_{1-2}NH_2$, $-S(=O)_{1-2}NHR_0$ or $-S(=O)_{1-2}N(R_0)_2$, $-NH_2$, $-NHR_0$, $-N(R_0)_2$, $-N^+(R_0)_3$, $-N^+(R_0)_2O^-$, $-NHC(=O)R_0$, $-NHC(=O)OR_0$, $-NHC(=O)NH_2$, $-NHC(=O)NHR_0$, and-

$NHC(=O)N(R_0)_2$; or $Y_1$ and $Y_1$', or $Y_2$ and $Y_2$', or $Y_3$ and $Y_3$', or $Y_4$ and $Y_4$' together represent =O;
$R_0$ in each case independently represents -$C_{1-8}$-aliphatic, -$C_{3-12}$-cycloaliphatic, -aryl, -heteroaryl, -$C_{1-8}$-aliphatic-$C_{3-12}$-cycloaliphatic, -$C_{1-8}$-aliphatic-aryl, -$C_{1-8}$-aliphatic-heteroaryl, -$C_{3-8}$-cydoaliphatic-$C_{1-8}$-aliphatic, -$C_{3-8}$-cycloaliphatic-aryl or -$C_{3-8}$-cycloaliphatic-heteroaryl;
$R_1$ and $R_2$ independently of each other represent -H or -$C_{1-8}$-aliphatic; or $R_1$ and $R_2$ together form a ring and represent -$(CH_2)_{2-4}$,
$R_3$ represents -$R_0$;
$R_4$ represents -H, -F, -Cl, -Br, -I, -aryl, -heteroaryl, -C(=O)H, -C(=O)$R_0$, -C(=O)O$R_0$, -CN, -C(=O)$NH_2$, -C(=O)NH$R_0$, -C(=O)N$(R_0)_2$, -OH, -O$R_0$, -OC(=O)H, -OC(=O)$R_0$, -OC(=O)O$R_0$, -OC(=O)NH$R_0$, -OC(=O)-N$(R_0)_2$, -$NH_2$, -NH$R_0$, -N$(R_0)_2$, -$N^+(R_0)_3$, -$N^+(R_0)_2O^-$, -NHC(=O)$R_0$, -NHC(=O)O$R_0$, -NHC(=O)NH$R_0$, -NHC(=O)-N$(R_0)_2$, -$NO_2$, -SH, S$R_0$, -S$(=O)_{1-2}R_0$, -S$(=O)_{1-2}$OH, -S$(=O)_{1-2}$O$R_0$, -S$(=O)_{1-2}NH_2$, -S$(=O)_{1-2}$NH$R_0$, -S$(=O)_{12}$N$(R_0)_2$,- OS$(=O)_{1-2}R_0$, -OS$(=O)_{1-2}$OH, -OS$(=O)_{1-2}$O$R_0$, -OS$(=O)_{1-2}NH_2$, -OS$(=O)_{1-2}$NH$R_0$ or -OS$(=O)_{1-2}$N$(R_0)_2$;
$R_5$ represents -H, -$R_0$, -C(=O)H, -C(=O)$R_0$, -C(=O)O$R_0$, -CN, -C(=O)$NH_2$, -C(=O)NH$R_0$ or -C(=O)N$(R_0)_2$;
wherein
"aliphatic" in each case is a branched or unbranched, saturated or mono- or polyunsaturated, unsubstituted or mono- or polysubstituted, aliphatic hydrocarbon radical;
"cycloaliphatic" in each case is a saturated or mono- or polyunsaturated, unsubstituted or mono- or polysubstituted, alicyclic, mono- or multicyclic hydrocarbon radical, the number of ring carbon atoms of which is preferably in the stated range;
wherein with respect to "aliphatic" and "cycloaliphatic", "mono- or polysubstituted" is understood as meaning substitution once or several times of one or more hydrogen atoms, e.g. substitution once, twice, three times or completely, by substituents chosen independently of each other from the group consisting of -F, -Cl, -Br, -I, -CN, -$NO_2$, -CHO, =O, -$R_0$, -C(=O)$R_0$, -C(=O)OH, -C(=O)O$R_0$, -C(=O)$NH_2$, -C(=O)NH$R_0$, -C(=O)N$(R_0)_2$, -OH, -O$R_0$, -OC(=O)H, -OC(=O)$R_0$, -OC(=O)O$R_0$, -OC(=O)NH$R_0$, -OC(=O)-N$(R_0)_2$, -SH, -S$R_0$, -$SO_3$H, -S$(=O)_{1-2}$-$R_0$, -S$(=O)_{1-2}NH_2$, -$NH_2$, -NH$R_0$, -N$(R_0)_2$, -$N^+(R_0)_3$, -$N^+(R_0)_2O^-$, -NHC(=O)$R_0$, -NHC(=O)O$R_0$, -NHC(=O)$NH_2$, -NHC(=O)-NH$R_0$, -NH-C(=O)N$(R_0)_2$, -Si$(R_0)_3$ and -PO$(OR_0)_2$;
"aryl" in each case independently represents a carbocyclic ring system having at least one aromatic ring, but without hetero atoms in this ring, wherein the aryl radicals can optionally be fused with further saturated, (partially) unsaturated or aromatic ring systems, which in their turn can have one or more hetero ring atoms, in each case independently of each other chosen from N, O and S, and wherein each aryl radical can be unsubstituted or mono- or polysubstituted, wherein the substituents on aryl can be identical or different and can be in any desired and possible position of the aryl;
"heteroaryl" represents a 5-, 6- or 7-membered cyclic aromatic radical which contains 1, 2, 3, 4 or 5 hetero atoms, wherein the hetero atoms are identical or different and are nitrogen, oxygen or sulfur and the heterocyclyl can be unsubstituted or mono- or polysubstituted; wherein in the case of substitution on the heterocyclyl the substituents can be identical or different and can be in any desired and possible position of the heteroaryl; and wherein the heterocyclyl can also be part of a bi- or polycyclic system;
wherein with respect to "aryl" and "heteroaryl", "mono- or polysubstituted" is understood as meaning substitution once or several times of one or more hydrogen atoms of the ring system by substituents chosen from the group consisting of -F, -Cl, -Br, -I, -CN, -$NO_2$, -CHO, =O, -$R_0$, -C(=O)$R_0$, -C(=O)OH, -C(=O)O$R_0$, -C(=O)$NH_2$, -C(=O)NH$R_0$, -C(=O)N$(R_0)_2$, -OH, -O$(CH_2)_{1-2}$O-, -O$R_0$, -OC(=O)H, -OC(=O)$R_0$, -OC(=O)O$R_0$, -OC(=O)NH$R_0$, -OC(=O)N$(R_0)_2$, -SH, -S$R_0$, -$SO_3$H, -S$(=O)_{1-2}$-$R_0$, -S$(=O)_{1-2}NH_2$, -$NH_2$, -NH$R_0$, -N$(R_0)_2$, -$N^+(R_0)_3$, -$N^+(R_0)_2O^-$, -NHC(=O)$R_0$, -NHC(=O)O$R_0$, -NHC(=O)$NH_2$, -NHC(=O)NH$R_0$, -NH-C(=O)N$(R_0)_2$, -Si$(R_0)_3$ and -PO$(OR_0)_2$;
wherein N ring atoms optionally present can in each case be oxidized;
in the form of an individual stereoisomer or mixture thereof, the free compounds and/or their physiologically acceptable salts.

**2.** Compound according to claim 1, wherein $Y_1$, $Y_1$', $Y_2$, $Y_2$', $Y_3$, $Y_3$', $Y_4$ and $Y_4$' in each case represent -H.

**3.** Compound according to one of the preceding claims, wherein $R_3$ is chosen from the group consisting of -phenyl, -benzyl or -phenethyl, in each case unsubstituted or mono- or polysubstituted on the ring; -$C_{1-5}$-aliphatic, -$C_{4-6}$-cycloaliphatic, -pyridyl, -thienyl, -thiazolyl, -imidazolyl, -1,2,4-triazolyl and -benzimidazolyl, in each case unsubstituted or mono- or polysubstituted.

**4.** Compound according to one of the preceding claims, wherein $R_5$ represents -H.

**5.** Compound according to claim 1, which has the general formula (4), (5), (6), (7), (8) or (9)

(4) 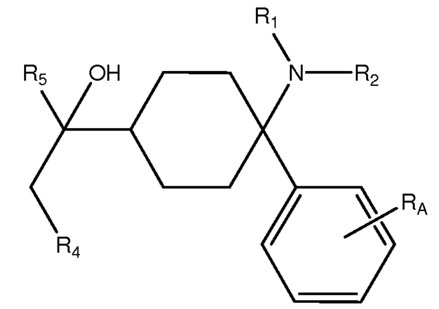 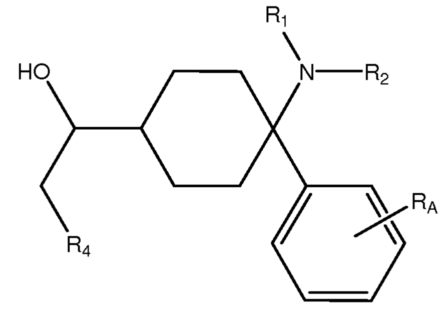 (5)

(6) 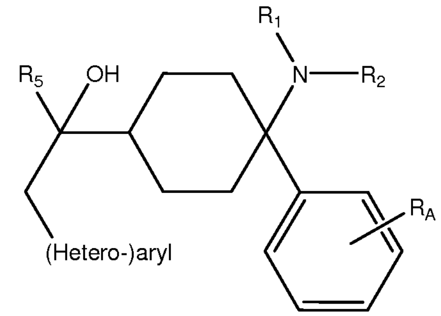 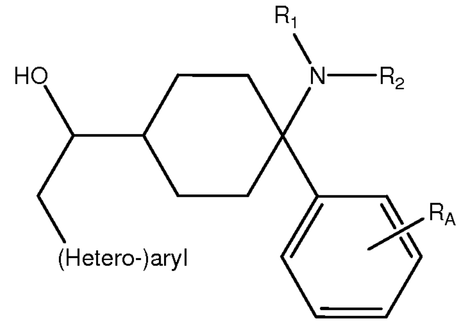 (7)

(8) 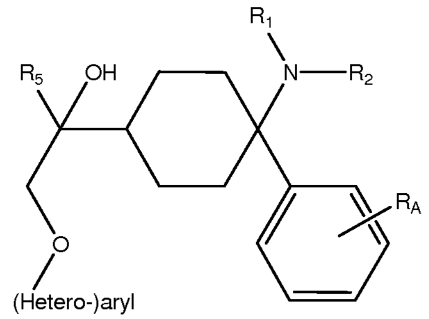 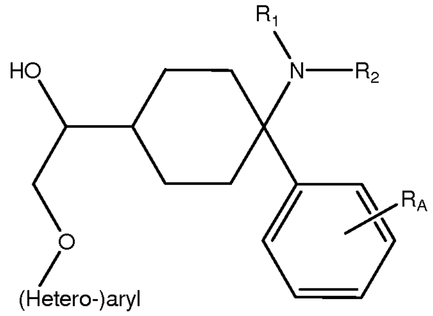 (9)

wherein, if present,
$R_A$ represents -H, -F, -Cl, -CN, $-NO_2$ or $-OCH_3$; and
(Hetero-)aryl represents heteroaryl or aryl, in each case unsubstituted or mono- or polysubstituted.

**6.** Compound according to claim 1, chosen from the group consisting of

- 1-(4-(dimethylamino)-4-phenylcyclohexyl)-2-phenylethanol;
- 1-(4-(dimethylamino)-4-phenylcyclohexyl)-2-phenoxyethanol;
- 1-(4-(dimethylamino)-4-phenylcyclohexyl)-2-(1H-indol-1-yl)ethanol;
- 1-(4-(dimethylamino)-4-phenylcyclohexyl)-2-(isoindolin-2-yl)ethanol,
- 1-(4-(dimethylamino)-4-phenylcyclohexyl)-2-(4-fluorophenyl)ethanol;
- 1-(4-(dimethylamino)-4-(3-fluorophenyl)cyclohexyl)-2-phenylethanol;
- 1-(4-(dimethylamino)-4-(3-methoxyphenyl)cyclohexyl)-2-phenylethanol;
- 1-(4-(dimethylamino)-4-(thiophen-2-yl)cyclohexyl)-2-phenylethanol;
- 1-(4-butyl-4-(dimethylamino)cyclohexyl)-2-phenylethanol;
- 1-cyclopentyl-2-(4-(dimethylamino)-4-phenylcyclohexyl)-3-phenylpropan-2-ol;
- 1-(4-(dimethylamino)-4-phenylcyclohexyl)-1-phenyl-2-(pyridin-4-yl)ethanol;
- 1-(4-(dimethylamino)-4-phenylcyclohexyl)-2-(phenylthio)ethanol;
- 1-(4-(dimethylamino)-4-phenylcyclohexyl)-2-(phenylsulfonyl)ethanol;
- 2-(cyclohexyloxy)-1-(4-(dimethylamino)-4-phenylcyclohexyl)ethanol;
- 2-(benzyloxy)-1-(4-(dimethylamino)-4-phenylcyclohexyl)ethanol;
- 1-(4-(dimethylamino)-4-phenylcyclohexyl)-2-phenethoxyethanol;

• 2-((1*H*-indol-3-yl)methoxy)-1-(4-(dimethylamino)-4-phenylcydohexyl)ethanol;
• 2-(2-(1*H*-indol-3-yl)ethoxy)-1-(4-(dimethylamino)-4-phenylcyclohexyl)ethanol;
• 1-(4-(dimethylamino)-4-phenylcyclohexyl)-2-((2-(triethylsilyl)-1*H*-indol-3-yl)-methoxy)ethanol;
• 2-(4,4a-dihydro-1*H*-pyrido[3,4-b]indol-2(3*H*,9*H*,9a*H*)-yl)-1-(4-(dimethylamino)-4-(3-fluorophenyl)cyclohexyl)ethanol;
• 2-(4-(dimethylamino)-4-phenylcyclohexyl)-1-phenylpropan-2-ol;
• 2-(4-(dimethylamino)-4-phenylcyclohexyl)-1,3-diphenylpropan-2-ol;
• 2-(4-(dimethylamino)-4-phenylcyclohexyl)-1-phenyl-3-(pyridin-2-yl)propan-2-ol;
• 2-(4-(dimethylamino)-4-phenylcyclohexyl)-1-phenyl-3-(pyridin-3-yl)propan-2-ol; and
• 2-(4-(dimethylamino)-4-phenylcyclohexyl)-1-phenyl-3-(pyridin-4-yl)propan-2-ol; and physiologically acceptable salts thereof.

**7.** Medicaments containing at least one compound according to one of claims 1 to 6 in the form of an individual stereoisomer or mixture thereof, the free compounds and/or their physiologically acceptable salts, and optionally suitable additives and/or auxiliary substances and/or optionally further active compounds.

**8.** Use of a compound according to one of claims 1 to 6 in the form of an individual stereoisomer or mixture thereof, the free compound and/or its physiologically acceptable salts, for the preparation of a medicament for treatment of pain.

**9.** Use of a compound according to one of claims 1 to 6 in the form of an individual stereoisomer or mixture thereof, the free compounds and/or their physiologically acceptable salts and/or solvates, for the preparation of a medicament for treatment of anxiety states, of stress and syndromes associated with stress, depression, epilepsy, Alzheimer's disease, senile dementia, general cognitive dysfunctions, learning and memory disorders (as a nootropic), withdrawal symptoms, alcohol and/or drug and/or medicament abuse and/or dependency, sexual dysfunctions, cardiovascular diseases, hypotension, hypertension, tinnitus, pruritus, migraine, impaired hearing, lack of intestinal motility, impaired food intake, anorexia, obesity, locomotor disorders, diarrhoea, cachexia, urinary incontinence or as a muscle relaxant, anticonvulsive or anaesthetic or for co-administration in treatment with an opioid analgesic or with an anaesthetic, for diuresis or antinatriuresis, anxiolysis, for modulation of motor activity, for modulation of neurotransmitter secretion and treatment of neurodegenerative diseases associated therewith, for treatment of withdrawal symptoms and/or for reduction of the addiction potential of opioids.

**Revendications**

**1.** Composé de formule générale (1)

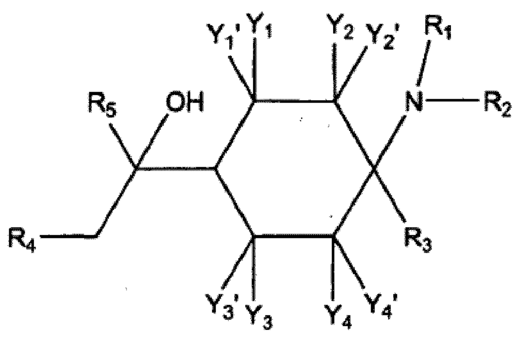

**(1)**

dans laquelle

$Y_1$, $Y_1'$, $Y_2$, $Y_2'$, $Y_3$, $Y_3'$, $Y_4$ et $Y_4'$ sont chacun choisis indépendamment les uns des autres dans le groupe constitué par -H, -F, -Cl, -Br, -I, -CN, $-NO_2$, -CHO, - $R_0$, $-C(=O)R_0$, -C(=O)-OH, $-C(=O)OR_0$, $-C(=O)NH_2$, - $C(=O)NHR_0$, $-C(=O)N(R_0)_2$, -OH, $-OR_0$, -OC(=O)H, $-OC(=O)R_0$, $-OC(=O)OR_0$, $-OC(=O)NHR_0$, $-OC(=O)N(R_0)_2$, -SH, $-SR_0$, - $S(=O)_{1-2}R_0$, $-S-(=O)_{1-2}OH$, $-S(=O)_{1-2}OR_0$, $-S(=O)_{1-2}NH_2$, - $S(=O)_{1-2}NHR_0$ ou $-S(=O)_{1-2}N(R_0)_2$, $-NH_2$, $-NHR_0$, $-N(R_0)_2$, - $N^+(R_0)_3$, $-N^+(R_0)_2O^-$, $-NHC(=O)R_0$, $-NHC(=O)OR_0$, $-NHC(=O)NH_2$, $-NHC(=O)NHR_0$ et $-NHC(=O)N(R_0)_2$ ; ou $Y_1$ et $Y_1'$, ou $Y_2$ et $Y_2'$, ou $Y_3$ et $Y_3'$, ou $Y_4$ 4 et $Y_4'$ représentent ensemble =O ;
les $R_0$ représentent chacun indépendamment aliphatique en $C_{1-8}$, cycloaliphatique en $C_{3-12}$, aryle, hétéroaryle,

aliphatique en $C_{1-8}$-cycloaliphatique en $C_{3-12}$, aliphatique en $C_{1-8}$-aryle, aliphatique en $C_{1-8}$-hétéroaryle, cycloaliphatique en $C_{3-8}$-aliphatique en $C_{1-8}$, cycloaliphatique en $C_{3-8}$-aryle ou cycloaliphatique en $C_{38}$-hétéroaryle ;
$R_1$ et $R_2$ représentent indépendamment l'un de l'autre H ou aliphatique en $C_{1-8}$ ; ou $R_1$ et $R_2$ forment ensemble un cycle et représentent - $(CH_2)_{2-4}$- ;
$R_3$ représente $-R_0$ ;
$R_4$ représente -H, -F, -Cl, -Br, -I, -aryle, - hétéroaryle, -C(=O)H, $-C(=O)R_0$, $-C(=O)OR_0$, -CN, - $C(=O)NH_2$, $-C(=O)NHR_0$, $-C(=O)N(R_0)_2$, -OH, $-OR_0$, -OC(=O)H, $-OC(=O)R_0$, $-OC(=O)OR_0$, $-OC(=O)NHR_0$, $-OC(=O)-N(R_0)_2$, $-NH_2$, $-NHR_0$, $-N(R_0)_2$, $-N^+(R_0)_3$, $-N^+(R_0)_2O^-$, $-NHC(=O)R_0$, - $NHC(=O)OR_0$, $-NHC(=O)NHR_0$, $-NHC(=O)-N(R_0)_2$, $-NO_2$, -SH, - $SR_0$, $-S(=O)_{1-2}R_0$, $-S(=O)_{1-2}OH$, $-S(=O)_{1-2}OR_0$, $-S(=O)_{1-2}NH_2$, - $S(=O)_{1-2}NHR_0$, $-S(=O)_{1-2}N(R_0)_2$, $-OS(=O)_{1-2}R_0$, $-OS(=O)_{1-2}OH$, - $OS(=O)_{1-2}OR_0$, $-OS(=O)_{1-2}NH_2$, $-OS(=O)_{1-2}NHR_0$ ou $-OS(=O)_{12}N(R_0)_2$ ;
$R_5$ représente -H, $-R_0$, -C(=O)H, $-C(=O)R_0$, $-C(=O)OR_0$, -CN, $-C(=O)NH_2$, $-C(=O)NHR_0$ ou $-C(=O)N(R_0)_2$ ;
« aliphatique » étant à chaque fois un radical hydrocarboné aliphatique ramifié ou non ramifié, saturé ou insaturé une ou plusieurs fois, non substitué ou substitué une ou plusieurs fois ;
« cycloaliphatique » étant à chaque fois un radical hydrocarboné alicyclique, mono- ou multicyclique, saturé ou insaturé une ou plusieurs fois, non substitué ou substitué une ou plusieurs fois, dont le nombre d'atomes de carbone de cycle se situe de préférence dans la plage indiquée ;
au regard d'« aliphatique » et de « cycloaliphatique », « substitué une ou plusieurs fois » se rapportant au remplacement une ou plusieurs fois d'un ou de plusieurs atomes d'hydrogène, p. ex. au remplacement une fois, deux fois, trois fois ou complet, par des substituants choisis indépendamment les uns des autres dans le groupe constitué par -F, -Cl, -Br, -I, -CN, $-NO_2$, -CHO, =O, $-R_0$, $-C(=O)R_0$, -C(=O)OH, $-C(=O)OR_0$, $-C(=O)NH_2$, - $C(=O)NHR_0$, $-C(=O)N(R_0)_2$, -OH, $-OR_0$, -OC(=O)H, $-OC(=O)R_0$, $-OC(=O)OR_0$, $-OC(=O)NHR_0$, $-OC(=O)-N(R_0)_2$, -SH, $-SR_0$, - $SO_3H$, $-S(=O)_{1-2}-R_0$, $-S(=O)_{1-2}NH_2$, $-NH_2$, $-NHR_0$, $-N(R_0)_2$, - $N^+(R_0)_3$, $-N(R_0)_2O^-$, -NHC (=O) $R_0$, $-NHC(=O)OR_0$, $-NHC(=O)NH_2$, $-NHC(=O)-NHR_0$, $-NH-C(=O)N(R_0)_2$, $-Si(R_0)_3$ et $-PO(OR_0)_2$ ;
« aryle » représentant à chaque fois indépendamment un système cyclique carbocyclique comprenant au moins un cycle aromatique, mais sans hétéroatome dans ce cycle, les radicaux aryle pouvant éventuellement être condensés avec d'autres systèmes cycliques saturés, (partiellement) insaturés ou aromatiques, qui peuvent de leur côté comprendre un ou plusieurs hétéroatomes de cycle, chacun choisis indépendamment les uns des autres parmi N, O et S, et chaque radical aryle pouvant être non substitué ou substitué une ou plusieurs fois, les substituants de l'aryle pouvant être identiques ou différents et se trouver à toute position quelconque et possible de l'aryle ;
« hétéroaryle » représentant un radical aromatique cyclique de 5, 6 ou 7 éléments, qui contient 1, 2, 3, 4 ou 5 hétéroatomes, les hétéroatomes étant identiques ou différents et étant l'azote, l'oxygène ou le soufre, et l'hétérocycle pouvant être non substitué ou substitué une ou plusieurs fois ; dans le cas de la substitution sur l'hétérocycle, les substituants pouvant être identiques ou différents et se trouver à toute position quelconque et possible de l'hétéroaryle ; et l'hétérocycle pouvant également faire partie d'un système bi- ou polycyclique ;
au regard d'« aryle » et « hétéroaryle », « substitué une ou plusieurs fois » se rapportant au remplacement une ou plusieurs fois d'un ou de plusieurs atomes d'hydrogène du système cyclique par des substituants choisis dans le groupe constitué par -F, -Cl, -Br, -I, -CN, $-NO_2$, -CHO, =O, $-R_0$, $-C(=O)R_0$, -C(=O)OH, $-C(=O)OR_0$, $-C(=O)NH_2$, $-C(=O)NHR_0$, $-C(=O)-N(R_0)_2$, -OH, $-O(CH_2)_{1-2}O-$, - $OR_0$, -OC(=O)H, $-OC(=O)R_0$, $-OC(=O)OR_0$, $-OC(=O)NHR_0$, - $OC(=O)N(R_0)_2$, -SH, $-SR_0$, $-SO_3H$, $-S(=O)_{1-2}-R_0$, $-S(=O)_{12}NH_2$, $-NH_2$, $-NHR_0$, $-N(R_0)_2$, $-N^+(R_0)_3$, $-N^+(R_0)_2O^-$, - $NHC(=O)R_0$, $-NHC(=O)OR_0$, $-NH-C(=O)NH_2$, $-NHC(=O)NHR_0$, - $NHC(=O)N(R_0)_2$, $-Si(R_0)_3$ et $-PO(OR_0)_2$ ; les atomes de cycle N éventuellement présents pouvant à chaque fois être oxydés ;
sous la forme d'un stéréoisomère individuel ou de leur mélange, des composés libres et/ou de leurs sels physiologiquement compatibles.

**2.** Composé selon la revendication 1, dans lequel $Y_1$, $Y_1'$, $Y_2$, $Y_2'$, $Y_3$, $Y_3'$, $Y_4$ et $Y_4'$ représentent chacun -H.

**3.** Composé selon l'une quelconque des revendications précédentes, dans lequel $R_3$ est choisi dans le groupe constitué par phényle, benzyle ou phénéthyle, à chaque fois non substitué ou substitué une ou plusieurs fois sur le cycle ; aliphatique en $C_{1-5}$, cycloaliphatique en $C_{4-6}$, pyridyle, thiényle, thiazolyle, imidazolyle, 1,2,4-triazolyle et benzimidazolyle, à chaque fois non substitué ou substitué une ou plusieurs fois.

**4.** Composé selon l'une quelconque des revendications précédentes, dans lequel $R_5$ représente -H.

**5.** Composé selon la revendication 1, qui présente la formule générale (4), (5), (6), (7), (8) ou (9)

(4) 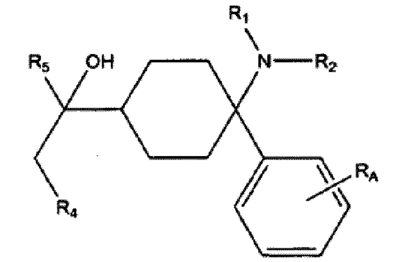 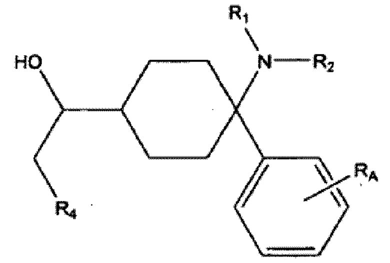 (5)

(6) 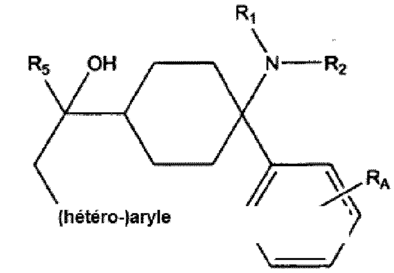 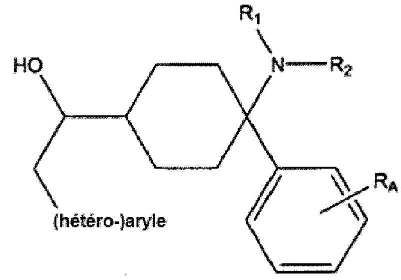 (7)

(8) 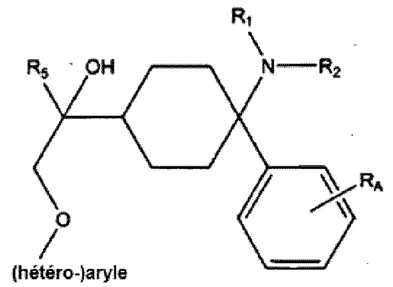 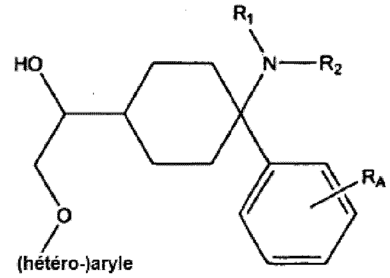 (9)

dans lesquelles, s'ils sont présents,
$R_A$ représente -H, -F, -Cl, -CN, $-NO_2$ ou $-OCH_3$ ; et
(hétéro-)aryle représente hétéroaryle ou aryle, à chaque fois non substitué ou substitué une ou plusieurs fois.

**6.** Composé selon la revendication 1, choisi dans le groupe constitué par :

- le 1-(4-(diméthylamino)-4-phénylcyclohexyl)-2-phényléthanol ;
- le 1-(4-(diméthylamino)-4-phénylcyclohexyl)-2-phénoxyéthanol ;
- le 1-(4-(diméthylamino)-4-phénylcyclohexyl)-2-(1H-indol-1-yl)éthanol ;
- le 1-(4-(diméthylamino)-4-phénylcyclohexyl)-2-(isoindolin-2-yl)éthanol,
- le 1-(4-(diméthylamino)-4-phénylcyclohexyl)-2-(4-fluorophényl)éthanol ;
- le 1-(4-(diméthylamino)-4-(3-fluorophényl)cyclohexyl)-2-phényléthanol ;
- le 1-(4-(diméthylamino)-4-(3-méthoxyphényl)cyclohexyl)-2-phényléthanol ;
- le 1-(4-(diméthylamino)-4-(thiophén-2-yl)cyclohexyl)-2-phényléthanol ;
- le 1-(4-butyl-4-(diméthylamino)cyclohexyl)-2-phényléthanol ;
- le 1-cyclopentyl-2-(4-(diméthylamino)-4-phénylcyclohexyl)-3-phénylpropan-2-ol ;
- le 1-(4-(diméthylamino)-4-phénylcyclohexyl)-1-phényl-2-(pyridin-4-yl)éthanol ;
- le 1-(4-(diméthylamino)-4-phénylcyclohexyl)-2-(phénylthio)éthanol ;
- le 1-(4-(diméthylamino)-4-phénylcyclohexyl)-2-(phénylsulfonyl)éthanol ;
- le 2-(cyclohexyloxy)-1-(4-(diméthylamino)-4-phénylcyclohexyl)éthanol ;
- le 2-(benzyloxy)-1-(4-(diméthylamino)-4-phénylcyclohexyl)éthanol ;
- le 1-(4-(diméthylamino)-4-phénylcyclohexyl)-2-phénéthoxyéthanol ;
- le 2-((1*H*-indol-3-yl)méthoxy)-1-(4-(diméthylamino)-4-phénylcyclohexyl)éthanol ;
- le 2-(2-(1*H*-indol-3-yl)éthoxy)-1-(4-(diméthylamino)-4-phénylcyclohexyl)éthanol ;
- le 1-(4-(diméthylamino)-4-phénylcyclohexyl)-2-((2-(triéthylsilyl)-1*H*-indol-3-yl)-méthoxy)éthanol ;
- le 2-(4,4a-dihydro-1*H*-pyrido[3,4-b]indol-2(3*H*,9*H*,9a*H*)-yl)-1-(4-(diméthylamino)-4-(3-fluorophényl)cyclohexyl)éthanol ;
- le 2-(4-(diméthylamino)-4-phénylcyclohexyl)-1-phénylpropan-2-ol ;
- le 2-(4-(diméthylamino)-4-phénylcyclohexyl)-1,3-diphénylpropan-2-ol ;
- le 2-(4-(diméthylamino)-4-phénylcyclohexyl)-1-phényl-3-(pyridin-2-yl)propan-2-ol ;
- le 2-(4-(diméthylamino)-4-phénylcyclohexyl)-1-phényl-3-(pyridin-3-yl)propan-2-ol ; et
- le 2-(4-(diméthylamino)-4-phénylcyclohexyl)-1-phényl-3-(pyridin-4-yl)propan-2-ol ;
et leurs sels physiologiquement compatibles.

**7.** Médicament contenant au moins un composé selon l'une quelconque des revendications 1 à 6 sous la forme d'un stéréoisomère individuel ou de leur mélange, des composés libres et/ou de leurs sels physiologiquement compatibles, ainsi qu'éventuellement des additifs et/ou adjuvants appropriés et/ou éventuellement des agents actifs supplémentaires.

**8.** Utilisation d'un composé selon l'une quelconque des revendications 1 à 6 sous la forme d'un stéréoisomère individuel ou de leur mélange, des composés libres et/ou de leurs sels physiologiquement compatibles, pour la fabrication d'un médicament pour le traitement de la douleur.

**9.** Utilisation d'un composé selon l'une quelconque des revendications 1 à 6 sous la forme d'un stéréoisomère individuel ou de leur mélange, des composés libres et/ou de leurs sels et/ou solvates physiologiquement compatibles, pour la fabrication d'un médicament pour le traitement des états d'anxiété, du stress et des syndromes associés avec le stress, des dépressions, de l'épilepsie, de la maladie d'Alzheimer, de la démence sénile, des dysfonctionnements cognitifs généraux, des troubles de l'apprentissage et de la mémoire (en tant que nootropique), des phénomènes de sevrage, de l'abus et/ou de la dépendance à l'alcool et/ou à des drogues et/ou à des médicaments, des dysfonctionnements sexuels, des maladies cardiovasculaires, de l'hypotension, de l'hypertension, des acouphènes, du prurit, de la migraine, des déficiences auditives, du manque de motilité intestinale, des troubles de l'alimentation, de l'anorexie, de l'obésité, des troubles locomoteurs, de la diarrhée, de la cachexie, de l'incontinence urinaire ou en tant que relaxant musculaire, anticonvulsif ou anesthétique ou pour la co-administration lors d'un traitement avec un analgésique opioïde ou avec un anesthétique, pour la diurèse ou l'antinatriurèse, l'anxiolyse, pour la modulation de l'activité motrice, pour la modulation de la distribution des neurotransmetteurs et le traitement de maladies neurodégénératives associées, pour le traitement de phénomènes de sevrage et/ou pour la réduction de l'effet de dépendance des opioïdes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2002090317 A
- WO 200290330 A
- WO 2003008370 A
- WO 2003008371 A
- WO 2003080557 A
- WO 2004043899 A
- WO 2004043900 A
- WO 2004043902 A
- WO 2004043909 A
- WO 2004043949 A
- WO 2004043967 A
- WO 2005063769 A
- WO 2005066183 A
- WO 2005110970 A
- WO 2005110971 A
- WO 2005110973 A
- WO 2005110974 A
- WO 2005110975 A
- WO 2005110976 A
- WO 2005110977 A
- WO 2006018184 A
- WO 2006108565 A
- WO 2007079927 A
- WO 2007079928 A
- WO 2007079930 A
- WO 2007079931 A
- WO 2007124903 A
- WO 2008009415 A
- WO 2008009416 A
- WO 04043900 A


### In der Beschreibung aufgeführte Nicht-Patentliteratur


- **VON ARDATI et al.** *Mol. Pharmacol.,* 1997, vol. 51, 816-824
- **D'AMOUR ; SMITH.** *J. Pharm. Exp. Ther.,* 1941, 72, 74-79